# EUROPEAN PATENT SPECIFICATION

(11) **EP 2 137 312 B1**
(45) Date of publication and mention of the grant of the patent: **23.01.2013**
(21) Application number: 08733383.7
(22) Date of filing: 21.04.2008
(51) Int. Cl.: C12N 15/82

(54) **MANIPULATION OF PLANT SENESCENCE USING MODIFIED PROMOTERS**
MANIPULATION VON PFLANZENSENESZENZ MIT MODIFIZIERTEN PROMOTERN
MANIPULATION DE SÉNESCENCE DANS LES PLANTES À L'AIDE DE PROMOTEURS MODIFIÉS

(30) Priority: 24.04.2007 US 789526
(43) Date of publication of application: 30.12.2009
(60) Divisional application: 12153159.4
(73) Proprietor: Agriculture Victoria Services PTY Limited, Attwood, VIC 3049 (AU); LA TROBE UNIVERSITY, Bundoora, VIC 3083 (AU)
(72) Inventor: SPANGENBERG, German, Bundoora, Victoria 3083 (AU); RAMAGE, Carl. McDonald, Craigieburn, Victoria 3064 (AU); PALVIAINEN, Melissa, Ann, Eden Park, Victoria 3757 (AU); PARISH, Roger, W., Warrandyte, Victoria 3113 (AU); HEAZLEWOOD, Joshua, Attwood, Victoria 3049 (AU)
(74) Representative: Grund, Martin
(86) International application number: PCT/AU2008/000556
(87) International publication number: WO 2008/128293

(56) References cited:
- WO-A1-02/20772
- WO-A1-96/29858
- WO-A2-00/70061
- YI HAN LIN ET AL: "Organ-specific, developmentally-regulated and abiotic stress-induced activities of four Arabidopsis thaliana promoters in transgenic white clover (Trifolium repens L.)" PLANT SCIENCE, ELSEVIER IRELAND LTD, IE LNKD- DOI:10.1016/J.PLANTSCI.2003.08.011, vol. 165, no. 6, 1 December 2003 (2003-12-01), pages 1437-1444, XP008094987 ISSN: 0168-9452
- KRANZ H D ET AL: "TOWARDS FUNCTIONAL CHARACTERISATION OF THE MEMBERS OF THE R2R3-MYB GENE FAMILY FROM ARABIDOPSIS THALIANA" PLANT JOURNAL, BLACKWELL SCIENTIFIC PUBLICATIONS, OXFORD, GB LNKD- DOI:10.1046/J.1365-313X.1998.00278.X, vol. 16, no. 2, 1 January 1998 (1998-01-01), pages 263-276, XP000929447 ISSN: 0960-7412
- SONG FENG LI ET AL: "A NOVEL MYB-RELATED GENE FROM ARABIDOPSIS THALIANA" FEBS LETTERS, ELSEVIER, AMSTERDAM, NL LNKD- DOI:10.1016/0014-5793(95)01461-6, vol. 379, 1 January 1996 (1996-01-01), pages 117-121, XP002938163 ISSN: 0014-5793
- SIDORENKO LYUDMILA V ET AL: "Complex structure of a maize Myb gene promoter: Functional analysis in transgenic plants" PLANT JOURNAL, vol. 22, no. 6, June 2000 (2000-06), pages 471-482, XP002580920 ISSN: 0960-7412
- ZHANG J. ET AL.: 'Development of flooding-tolerant Arabidopsis thaliana by autoregulated cytokinin production' MOLECULAR BREEDING vol. 6, no. 2, 2000, pages 135 - 144, XP001058078
- REYNOLDS P.H.S.: 'Inducible Gene Expression in Plants', 1999, CABI PUBLISHING, WALLINGFORD, U.K. article GAN S. AND AMASINO R.M.: 'Developmental targeting of gene expression by the use of a senescence specific promoter', pages 169 - 186, XP008137602
- GAN S. AND AMASINO R.M.: 'Inhibition of leaf senescence by autoregulated production of cytokinin' SCIENCE vol. 270, 1995, pages 1986 - 1988, XP002085505
- SMART C.M. ET AL.: 'Delayed leaf senescence in tobacco plants transformed with tmr, a gene for cytokinin production in agrobacterium' THE PLANT CELL vol. 3, no. 7, 1991, pages 647 - 656, XP002014396

## Description

The present invention relates to methods of manipulating senescence in plants. The invention also relates to vectors useful in such methods, transformed plants with modified senescence characteristics and plant cells, seeds and other parts of such plants.

Leaf senescence involves metabolic and structural changes in cells prior to cell death. It also involves the recycling of nutrients to actively growing regions.

The regulation of plant and plant organ senescence by cytokinins has important agricultural consequences. Elevated cytokinin levels in leaves tend to retard senescence. A number of promoters have been used to regulate the expression of the ipt gene, whose product (isopentenyltransferase) catalyses a key step in cytokinin synthesis. However, in general, transgenic plants over-expressing the *ipt* gene have been reported to have retarded root and shoot growth, no root formation, reduced apical dominance, and reduced leaf area.

It is an object of the present invention to overcome, or at least alleviate, one or more of the difficulties or deficiencies associated with the prior art.

In one aspect, the present invention provides a method of manipulating senescence in a plant, said method including introducing into said plant a genetic construct including a modified *myb* gene promoter, including a nucleotide sequence selected from the group consisting of SEQ ID NOs: 2, 3, and 4 or a functionally active fragment or variant thereof, having at least 95% identity to the part of SEQ ID NOs: 2, 3, or 4 to which the fragment or variant corresponds and having a size of at least 300 nucleotides, operatively linked to a gene encoding an enzyme involved in biosynthesis of a cytokinin, or a functionally active fragment or variant thereof.

The manipulation of senescence relates to the plant and/or specific plant organs. Senescence of different plant organs, such as leaves, roots, shoots, stems, tubers, flowers, stolons, and fruits may be manipulated. The manipulation of plant and plant organ senescence may have important agricultural consequences, such as increased shelf life of e.g. fruits, flowers, leaves and tubers in horticultural produce and cut flowers, reduced perishability of horticultural crops, increased carbon fixation in senescence-retarded leaves leading to enhanced yields, enhanced biomass production in forage plants, enhanced seed production, etc.

"Manipulating senescence" generally relates to delaying senescence in the transformed plant relative to an untransformed control plant. However, for some applications it may be desirable to promote or otherwise modify senescence in the plant. Senescence may be promoted or otherwise modified for example, by utilizing an antisense gene.

An effective amount of said genetic construct may be introduced into said plant, by any suitable technique, for example by transduction, transfection or transformation. By "an effective amount" is meant an amount sufficient to result in an identifiable phenotypic trait in said plant, or a plant, plant seed or other plant part derived therefrom. Such amounts can be readily determined by an appropriately skilled person, taking into account the type of plant, the route of administration and other relevant factors. Such a person will readily be able to determine a suitable amount and method of administration. See, for example, Maniatis et al, Molecular Cloning: A Laboratory Manual, Cold Spring Harbor Laboratory, Cold Spring Harbor, the entire disclosure of which is incorporated herein by reference.

By a "modified *myb* gene promoter" is meant a promoter normally associated with a *myb* gene, which promoter is modified to delete or inactivate one or more root specific motifs and/or pollen specific motifs in said promoter.

While applicant does not wish to be restricted by theory, it is postulated that deletion or inactivation of one or more root specific motifs in said *myb* gene promoter may alleviate or overcome the problem of leaky expression of the gene encoding a cytokinin biosynthetic enzyme in plant meristems, which may affect root development in some species of plants. It is also postulated that deletion or inactivation of one or more pollen specific motifs in said *myb* gene promoter may alleviate or overcome the problem of leaky expression of the gene encoding a cytokinin biosynthetic enzyme in pollen, which may affect pollen development in some species of plants.

The modified *myb* gene promoter is a modified *myb32* gene promoter. The modified m*yb* gene promoter is from *Arabidopsis thaliana.*

A suitable promoter which may be modified according to the present invention is described in Li et al., Cloning of three MYB-like genes from Arabidopsis (PGR 99-138) Plant Physiology 121:313 (1999).

By a "root specific motif" is meant a sequence of 3-7 nucleotides, preferably 4-6 nucleotides, more preferably 5 nucleotides, which directs expression of an associated gene in the roots of a plant.

Preferably the root specific motif includes a consensus sequence ATATT or AATAT.

Preferably, between one and ten, more preferably between three and eight, even more preferably between five and seven root specific motifs are deleted or inactivated, preferably deleted, in said *myb* gene promoter.

The root specific motifs may be deleted by removing individual motifs or by removing a fragment of the promoter containing one or more motifs. For example, all or part of the region between nucleotides 1 and 530, preferably between nucleotides 110 and 530 of the *Arabidopsis thaliana myb* gene promoter may be deleted.

The deletion may be effected by cutting the nucleic acid, for example with restriction endonucleases, and ligating the cut ends to generate a promoter with a fragment removed.

For example, a modified *Arabidopsis thaliana myb* gene promoter may be prepared by removing a fragment between the *Xcml* site at positions 162-176 and the *Sspl* site at positions 520-525. This generates a modified *myb* gene promoter with 6 of the 7 root specific motifs deleted. Alternatively, all 7 of the root specific motifs may be deleted, for example by deleting the region upstream of the *Sspl* site at positions 520-525, or by deleting the region between nucleotides 1 and 120 together with the region between the *Xcml* site at positions 162-176 and the *Sspl* site at positions 520-525.

A root specific motif may be inactivated by adding, deleting, substituting or derivatizing one or more nucleotides within the motif, so that it no longer has the preferred consensus sequence.

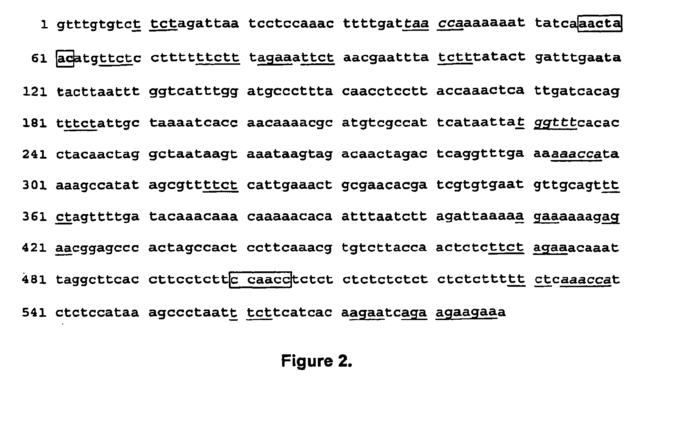

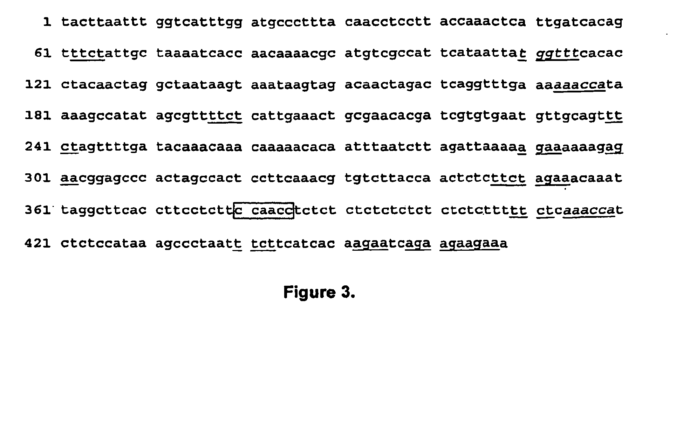

The modified *myb* gene promoter includes a nucleotide sequence selected from the group consisting of the sequences shown in Figures 2, 3 and 4 hereto (Sequence ID Nos: 2, 3 and 4, respectively) and functionally active fragments and variants thereof having at least 95% identity to the part of SEQ ID NOs: 2, 3, or 4 to which the fragment or variant corresponds and having a size of at least 300 nucleotides.

By a "pollen specific motif" is meant a sequence of 3-7 nucleotides, preferably 4-6 nucleotides, more preferably 4 or 5 nucleotides, which directs expression of an associated gene in the pollen of a plant.

Preferably the pollen specific motif includes a consensus sequence selected from the group consisting of TTCT and AGAA.

Preferably, between one and thirty, more preferably between three and fifteen, even more preferably between four and ten pollen specific motifs are deleted or inactivated, preferably deleted, in said *myb* gene promoter.

The pollen specific motifs may be deleted by removing individual motifs or by removing a fragment of the promoter containing one or more motifs. For example, all or part of the region between nucleotides 1 and 540, preferably between nucleotides 390 and 540 of the *Arabidopsis thaliana myb* gene promoter may be deleted.

The deletion may be effected by cutting the nucleic acid, for example with restriction endonucleases, and ligating the cut ends to generate a promoter with a fragment removed.

For example, a modified *Arabidopsis thaliana myb* gene promoter may be prepared by removing a fragment between the *Xcml* site at positions 162-176 and the *Sspl* site at positions 520-525. This generates a modified *myb* gene promoter with 4 of the 23 pollen specific motifs deleted. Alternatively, 10 of the pollen specific motifs may be deleted, for example by deleting the region upstream of the *Sspl* site at positions 520-525.

A pollen specific motif may be inactivated by adding, deleting, substituting or derivatizing one or more nucleotides within the motif, so that it no longer has the preferred consensus sequence.

The modified *myb* gene promoter includes a nucleotide sequence selected from the group consisting of the sequences shown in Figures 2, 3 and 4 hereto (Sequence ID Nos: 2. 3 and 4, respectively) and functionally active fragments and variants thereof having at least 95% identity to the part of SEQ ID NOs: 2, 3, or 4 to which the fragment or variant corresponds and having a size of at least 300 nucleotides]₂.

In a further aspect of the present invention there is provided a method of enhancing biomass in a plant, said method include introducing into said plant a genetic construct including a *myb* gene promoter including a nucleotide sequence selected from the group consisting of SEQ ID NOs: 2, 3, and 4; or a functionally active fragment or variant thereof having at least 95% identity to the part of SEQ ID NOs: 2, 3, or 4 to which the fragment or variant corresponds and having a size of at least 300 nucleotides; operatively linked to a gene encoding an enzyme involved in biosynthesis of a cytokinin, or a functionally active fragment or variant thereof.

The *myb* gene promoter or a functionally active fragment or variant thereof is a modified *myb* gene promoter.

The modified *myb* gene promoter is a modified *myb* gene promoter as hereinbefore described.

By "enhancing biomass" is meant enhancing or increasing in a transformed plant relative to an untransformed control plant a growth characteristic selected from the group consisting of total leaf area, cumulative leaf area, leaf growth dynamics (i.e. number of leaves over time), stolon length, percentage of flowering plants and seed yield per flower or per area sown. "Enhancing biomass" also includes reducing or decreasing percentage stolon death in a transformed plant relative to an untransformed control plant.

In particular, applicants have found that while the seed weight (i.e. weight of thousand seeds) of transgenic plants according to the present invention was indistinguishable from non-transgenic control plants, the total seed yield expressed on the basis of per flower or per area sown was significantly higher in the transgenic plants when compared with non-transgenic control plants of equivalent flowering intensity.

By "functionally active" in relation to a *myb* gene promoter or modified *myb* gene promoter is meant that the fragment or variant (such as an analogue, derivative or mutant) is capable of manipulating senescence in a plant by the method of the present invention. Such variants include naturally occurring allelic variants and non-naturally occurring variants. Additions, deletions, substitutions and derivatizations of one or more of the nucleotides are contemplated so long as the modifications do not result in loss of functional activity of the fragment or variant.

The functionally active fragment or variant has at least 95% identity to the relevant part of the above mentioned sequence to which the fragment or variant corresponds. The fragment has a size of at least 300 nucleotides.

By a "gene encoding an enzyme involved in biosynthesis of a cytokinin" is meant a gene encoding an enzyme involved in the synthesis of cytokines such as kinetin, zeatin and benzyl adenine, for example a gene encoding isopentyl transferase (*ipt*), or an *ipt*-like gene such as the *sho* gene (eg. from petunia). Preferably the gene is an isopentenyl transferase (*ipf*) gene or *sho* gene. In a preferred embodiment, the gene is from a species selected from the group consisting of *Agrobacterium,* more preferably *Agrobacterium tumefaciens; Lotus,* more preferably *Lotus japonicus*; and *Petunia,* more preferably *Petunia hybrida.*

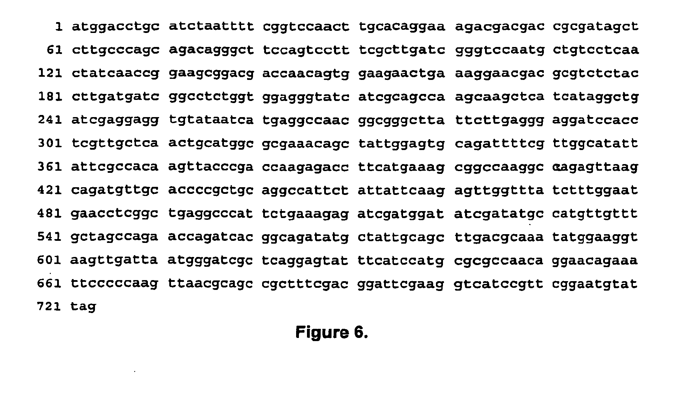

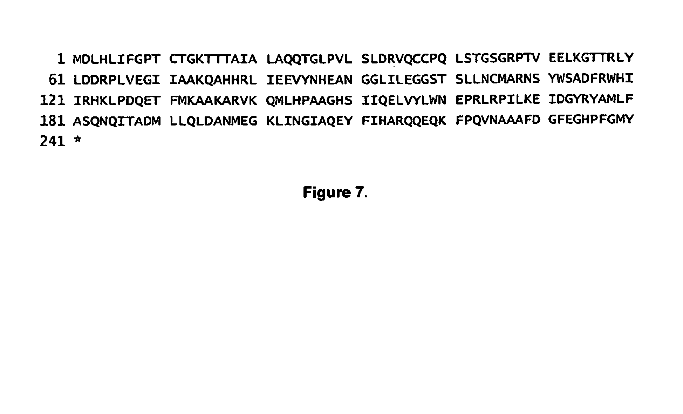

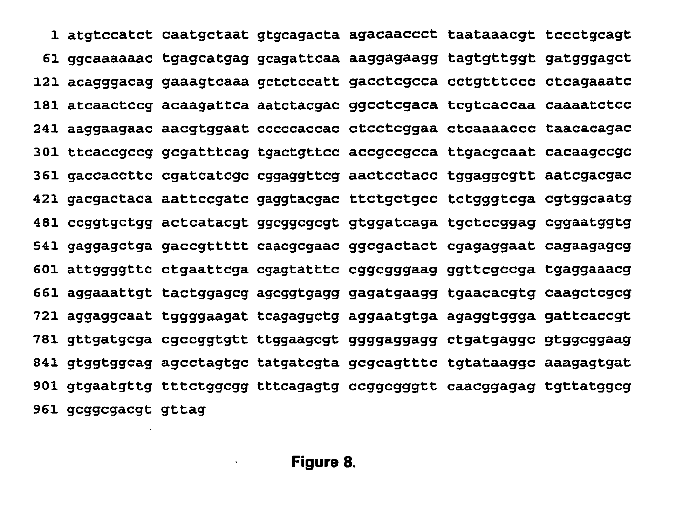

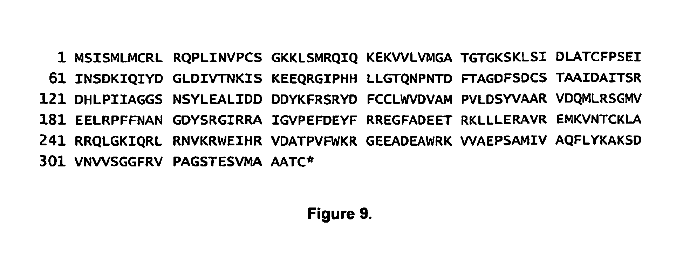

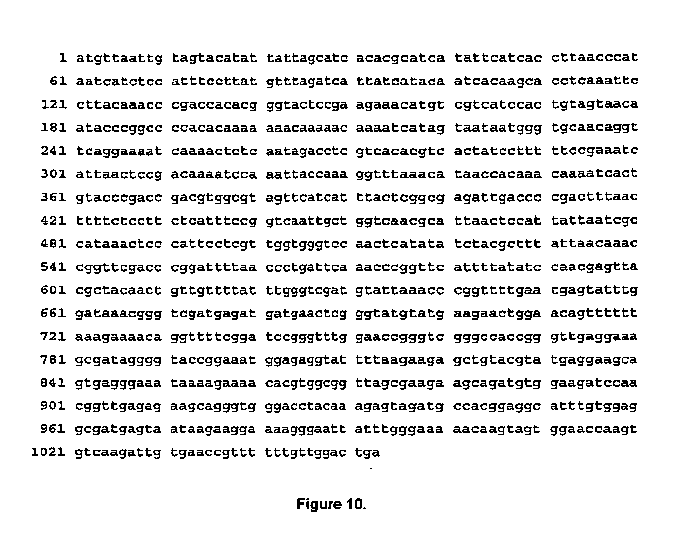

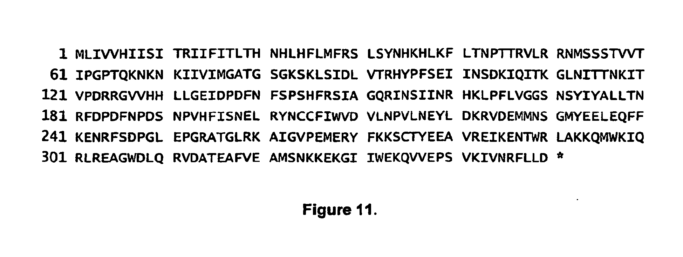

Most preferably the gene includes a nucleotide sequence selected from the group consisting of the sequences shown in Figures 6, 8 and 10 hereto (Sequence ID Nos: 5, 7 and 9) sequences encoding the polypeptides shown in Figures 7, 9 and 11 hereto (Sequence ID Nos: 6, 8 and 10), and functionally active fragments and variants thereof.

By "functionally active" in relation to a gene encoding a cytokinin biosynthetic enzyme is meant that the fragment or variant (such as an analogue, derivative or mutant) is capable of manipulating senescence in a plant by the method of the present invention. Such variants include naturally occurring allelic variants and non-naturally occurring variants. Additions, deletions, substitutions and derivatizations of one or more of the nucleotides are contemplated so long as the modifications do not result in loss of functional activity of the fragment or variant. Preferably the functionally active fragment or variant has at least approximately 80% identity to the relevant part of the above mentioned sequence, to which the fragment or variant corresponds more preferably at least approximately 90% identity, most preferably at least approximately 95% identity. Such functionally active variants and fragments include, for example, those having conservative nucleic acid changes or nucleic acid changes which result in conservative amino acid substitutions of one or more residues in the corresponding amino acid sequence. For example, the functionally active variant may include one or more conservative nucleic acid substitutions of a sequence shown in Figure 6, 8 or 10, the resulting functionally active variant encoding an amino acid sequence shown in Figure 7, 9 or 11, respectively. Preferably the fragment has a size of at least 20 nucleotides, more preferably at least 50 nucleotides, more preferably at least 100 nucleotides, more preferably at least 500 nucleotides.

The genetic construct may be introduced into the plant by any suitable technique. Techniques for incorporating the genetic constructs of the present invention into plant cells (for example by transduction, transfection or transformation) are well known to those skilled in the art. Such techniques include *Agrobacterium* mediated introduction, electroporation to tissues, cells and protoplasts, protoplast fusion, injection into reproductive organs, injection into immature embryos and high velocity projectile introduction to cells, tissues, calli, immature and mature embryos, biolistic transformation and combinations thereof. The choice of technique will depend largely on the type of plant to be transformed, and may be readily determined by an appropriately skilled person.

Cells incorporating the genetic construct of the present invention may be selected, as described below, and then cultured in an appropriate medium to regenerate transformed plants, using techniques well known in the art. The culture conditions, such as temperature, pH and the like, will be apparent to the person skilled in the art. The resulting plants may be reproduced, either sexually or asexually, using methods well known in the art, to produce successive generations of transformed plants.

The methods of the present invention may be applied to a variety of plants, including monocotyledons [such as grasses (e.g. forage, turf and bioenergy grasses including perennial ryegrass, tall fescue, Italian ryegrass, red fescue, reed canary grass, big bluestem, cordgrass, napiergrass, wildrye, wild sugarcane, Miscanthus), corn, oat, wheat and barley)], dicotyledons [such as *Arabidopsis,* tobacco, soybean, clovers (e.g. white clover, red clover, subterranean clover), alfalfa, canola, vegetable brassicas, lettuce, spinach] and gymnosperms.

In a further aspect of the present invention there is provided a vector capable of manipulating senescence in a plant, said vector including a modified *myb* gene promoter including a nucleotide sequence selected from the group consisting of SEQ ID NOs: 2, 3, and 4; or a functionally active fragment or variant thereof having at least 95% identity to the part of SEQ ID NOs: 2, 3, or 4 to which the fragment or variant corresponds and having a size of at least 300 nucleotides; operatively linked to a gene encoding an enzyme involved in the biosynthesis of a cytokinin, or a functionally active fragment or variant thereof.

In a still further aspect of the present invention there is provided a vector capable of enhancing biomass in a plant, said vector including a *myb* gene promoter, or a functionally active fragment or variant thereof, operatively linked to a gene encoding an enzyme involved in the biosynthesis of a cytokinin, or a functionally active fragment or variant thereof.

The *myb* gene promoter or a functionally active fragment or variant thereof is a modified *myb* gene promoter, as described herein.

In a preferred embodiment of this aspect of the invention, the vector may further include a terminator; said promoter, gene and terminator being operably linked.

By "operably linked" is meant that said promoter is capable of causing expression of said gene in a plant cell and said terminator is capable of terminating expression of said gene in a plant cell. Preferably, said promoter is upstream of said gene and said terminator is downstream of said gene.

The vector may be of any suitable type and may be viral or non-viral. The vector may be an expression vector. Such vectors include chromosomal, non-chromosomal and synthetic nucleic acid sequences, eg. derivatives of plant viruses; bacterial plasmids; derivatives of the Ti plasmid from *Agrobacterium tumefaciens;* derivatives of the Ri plasmid from *Agrobacterium rhizogenes;* phage DNA; yeast artificial chromosomes; bacterial artificial chromosomes; binary bacterial artificial chromosomes; vectors derived from combinations of plasmids and phage DNA. However, any other vector may be used as long as it is replicable or integrative or viable in the plant cell.

The promoter, gene and terminator may be of any suitable type and may be endogenous to the target plant cell or may be exogenous, provided that they are functional in the target plant cell.

A variety of terminators which may be employed in the vectors of the present invention are also well known to those skilled in the art. The terminator may be from the same gene as the promoter sequence or a different gene. Particularly suitable terminators are polyadenylation signals, such as the CaMV 35S polyA and other terminators from the nopaline synthase (nos) and the octopine synthase (ocs) genes.

The vector, in addition to the promoter, the gene and the terminator, may include further elements necessary for expression of the gene, in different combinations, for example vector backbone, origin of replication (ori), multiple cloning sites, spacer sequences, enhancers, introns (such as the maize Ubiquitin *Ubi* intron), antibiotic resistance genes and other selectable marker genes [such as the neomycin phosphotransferase (*nptII*) gene, the hygromycin phosphotransferase (*hph*) gene, the phosphinothricin acetyltransferase (*bar* or *pat*) gene], and reporter genes (such as beta-glucuronidase (GUS) gene (*gusA)*]. The vector may also contain a ribosome binding site for translation initiation. The vector may also include appropriate sequences for amplifying expression.

As an alternative to use of a selectable marker gene to provide a phenotypic trait for selection of transformed host cells, the presence of the vector in transformed cells may be determined by other techniques well known in the art, such as PCR (polymerase chain reaction), Southern blot hybridisation analysis, histochemical assays (e.g. GUS assays), thin layer chromatography (TLC), northern and western blot hybridisation analyses.

Those skilled in the art will appreciate that the various components of the vector are operably linked, so as to result in expression of said gene. Techniques for operably linking the components of the vector of the present invention are well known to those skilled in the art. Such techniques include the use of linkers, such as synthetic linkers, for example including one or more restriction enzyme sites.

In a further aspect of the present invention there is provided a transgenic plant cell, plant, plant seed or other plant part, with modified senescence characteristics or enhanced biomass. Preferably said plant cell, plant, plant seed or other plant part includes a vector according to the present invention. Preferably the transgenic plant cell, plant, plant seed or other plant part is produced by a method according to the present invention.

The present invention also provides a transgenic plant, plant seed or other plant part derived from a plant cell of the present invention.

The present invention also provides a transgenic plant, plant seed or other plant part derived from a plant of the present invention.

The present invention will now be more fully described with reference to the accompanying examples and drawings. It should be understood, however, that the description following is illustrative only and should not be taken in any way as a restriction on the generality of the invention described above.

### In the figures:

**Figure 1** shows the nucleotide sequence of the promoter from *myb32* gene (*atmyb32*) from *Arabidopsis thaliana* (Sequence ID No: 1)*, Atmyb32* promoter sequence with MYB type, pollen specific and Root specific motifs highlighted.
**Figure 2** shows an *Atmyb32* promoter sequence variant *(Atmyb32xs)* with the Xcml - Sspl plant sequence deleted. MYB type, pollen specific and Root specific motifs are highlighted. WAACCA (*underline*/*italics*) MYB1AT; GTTAGTT **(bold/box)** MYB1LEPR; CCWACC (box) MYBPZM; AGAAA (underline) POLLEN1 LELAT52; ATATT (bold) ROOTMOTIFTAPOX1 (Sequence ID No: 2).
**Figure 3** shows an *Atmyb32* promoter variant sequence with all root motifs deleted. MYB type, pollen specific and Root specific motifs are highlighted. WAACCA (*underline*/*italics*) MYB1AT; CCWACC (box) MYBPZM; AGAAA (underline) POLLEN1LELAT52 (Sequence ID No: 3)
**Figure 4** shows an *Atmyb32* promoter variant sequence with the *Sspl* site upstream sequence deleted. MYB type, pollen specific and Root specific motifs are highlighted. WAACCA (*underline*/*italics*) MYB1AT; CCWACC (box) MYBPZM; AGAAA (underline) POLLEN1 LELAT52 (Sequence ID No: 4).

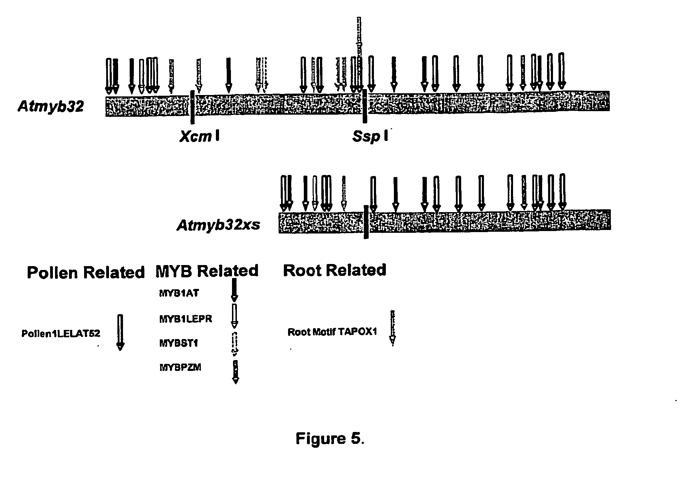

**Figure 5** shows Motifs in *Atmyb32* and *Atmyb32xs* promoter sequences.
**Figure 6** shows the nucleotide sequence of the isopentenyl transferase *(ipt)* gene from *Agrobacterium tumefaciens* (Sequence ID No: 5).
**Figure 7** shows the deduced amino acid sequence of the isopentyl transferase gene from *Agrobacterium tumefaciens* (Sequence ID No. 6)
**Figure 8** shows the nucleotide sequence of the isopentyl transferase gene from *Lotus japonicus* (Sequence ID No. 7).
**Figure 9** shows the deduced amino acid sequence of the isopentyl transferase gene from *Lotus japonicus* (Sequence ID No. 8).
**Figure 10** shows the Nucleotide sequence of the cytokinin biosynthesis *Sho* gene from *Petunia hybrida* (Sequence ID No. 9)
**Figure 11** shows the Deduced amino acid sequence of the cytokinin biosynthesis Sho gene from *Petunia hybrida* (Sequence ID No. 10).

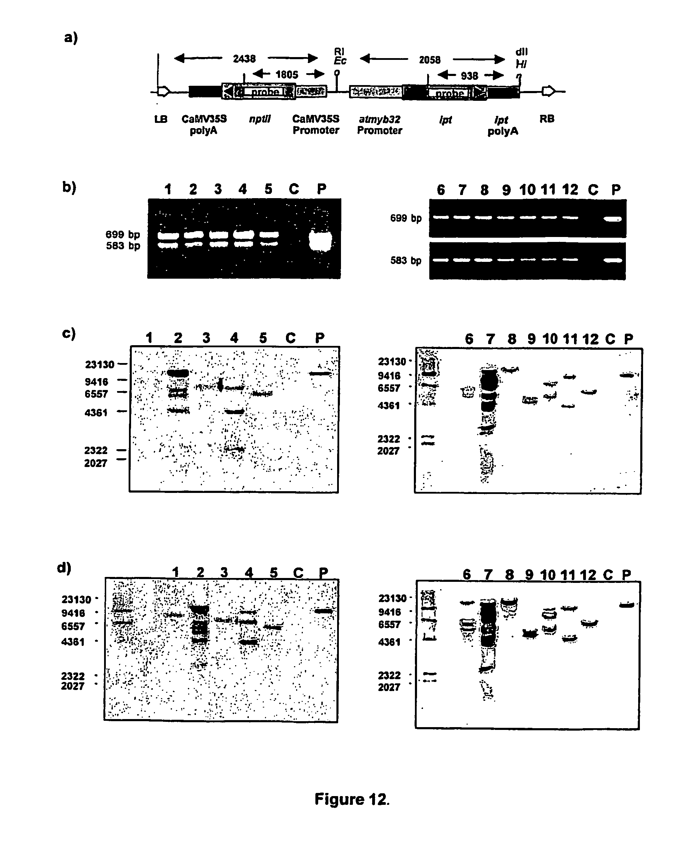

**Figure 12** shows PCR and Southern DNA analysis of *atmyb32::ipt* transgenic white clover (*Trifolium repens*) plants. a) The T-DNA region of patmyb32:ipt showing restriction enzyme sites and location of the probes used for Southern hybridization analysis. b) Ethidium bromide stained 1% agarose gel of the PCR amplified 599 bp *nptII* and 583 bp *ipt* products. c) Southern blot hybridization with *Hind*III digested total genomic DNA isolated from PCR positive white clover plants hybridized with the *ipt* probe. d) Southern blot hybridization with *Hind*III digested total genomic DNA isolated from PCR positive white clover plants hybridized with the *nptII* probe. Lanes 1-2: two independent kanamycin resistant cv. Haifa regenerants, code: Hmi01, Hmi08 respectively; Lanes 3-12: twelve independent kanamycin resistant cv. Irrigation regenerants, codes: Imi06, Imi07, Imi08, Imi09, Imi10, Imi11, Imi12, Imi14, Imi16, Imi18 respectively; Lane C: non-transformed white clover; Lane P: positive control plasmid patmyb32ipt.

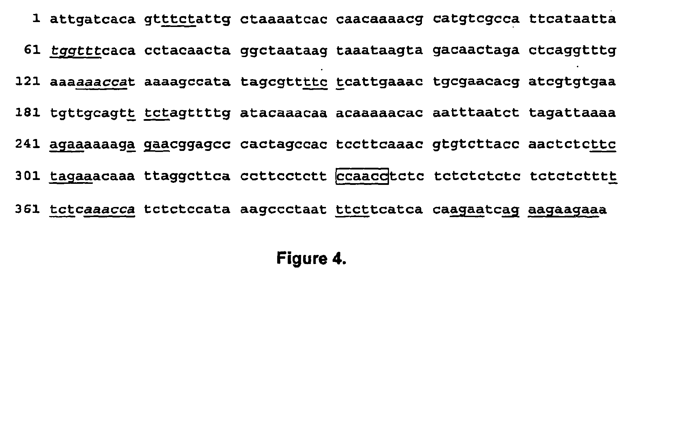

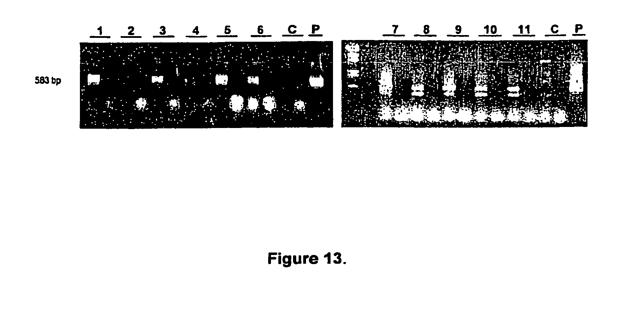

**Figure 13** shows RT-PCR analysis of *ipt* mRNA expression in *atmyb32::ipt* transgenic white clover (*T. repens*) plants. Lane 1-11 are samples from 11 independent transgenic lines with corresponding plant codes as in Figure 4.8; Lane C, Control non-transformed plant; Lane P, plasmid as positive control. Total RNA was isolated from leaf tissues. Total RNA (13 µg) was used for each reverse transcription reaction and 1/5 of RT product was amplified by PCR. DNA products on the gel on the right were amplified by 2X 30 cycles intensive PCR. No reverse transcriptase was added to the corresponding RT-PCR reaction loaded into alternate lanes.

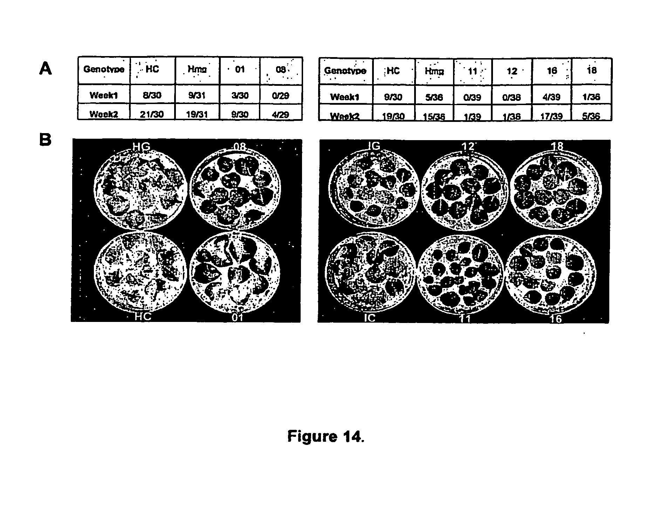

**Figure 14** shows a senescence bioassay of excised leaves from *atmyb32::ipt* transgenic white clover (*T. repens*) plants. At least 30 leaves were collected from each line from similar positions on stolons of plant lines. A. The number of yellowing leaves as a fraction of the total number of excised leaves. B. Typical appearance of leaves kept on water under light for two weeks. Key to plant lines: HC, IC and Hmg, Img, non-transformed and *atmyb32::gusA* transgenic plants (cv. Haifa and Irrigation) respectively; 01 and 08, *atmyb32::ipt* transgenic Haifa lines Hmi01 and Hmi08 respectively; 11, 12, 16 and 18 *atmyb32::ipt* transgenic Irrigation lines Imi11, Imi12, Imi16 and Imi18 respectively.

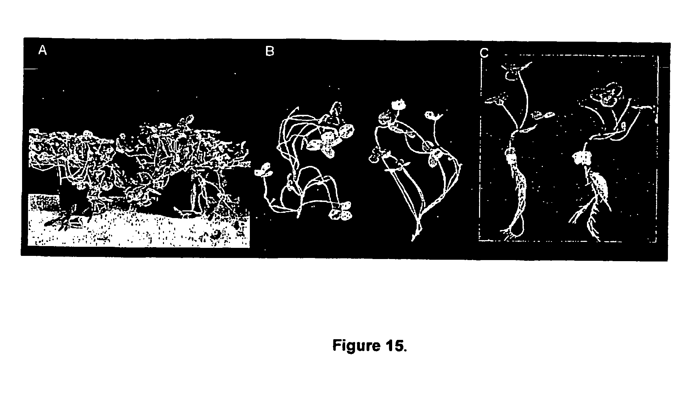

**Figure 15** shows A) General plant morphology, B) Normal shoot development, and C) Normal root development in *atmyb32::ipt* transgenic white clover (*T*. *repens)* (right) plants compared to control plants (left).
**Figure 16** shows vector details for *pBMVkAtMYB32-900::ipt* [Gene: Isopentyl transferase (*IPT*); Vector. pBMVkAtMYB32-900::ipt-nos (backbone pPZPRCS2); Selectable marker: spec; Plant selectable marker cassette: 35S::kan::35ST; Gene promoter: *AtMYB32-900;* Gene terminator: nos.]
**Figure 17** shows nucleotide sequence of vector *pBMVkAtMYB32-900::ipt-nos* (Sequence ID No. 11)
**Figure 18** shows vector details for *pBMVkAtMYB32xs::ipt-nos* [Gene: Isopentyl transferase (*IPT*); Vector: pBMVkAtMYB32XS::ipt-nos (backbone pPZPRCS2); Selectable marker: spec; Plant selectable marker cassette: 35S::kan::35ST; Gene promoter: AtMYB32-xs; Gene terminator: nos.]
**Figure 19** shows nucleotide sequence of vector *pBMVkAtMYB32xs::ipt-nos* (Sequence ID No. 12).
**Figure 20** shows vector details for *pBMVhAtMY832-900::ipt-nos* [Gene: Isopentyl transferase **(***IPT*); Vector: pBMVhAtMYB32-900::ipt-nos (backbone pPZPRCS2); Selectable marker: spec; Plant selectable marker cassette: 35S::hph::35ST; Gene promoter: AtMYB32-900; Gene terminator: nos.]
**Figure 21** shows nucleotide sequence of vector *pBMVhAtMYB32-900::ipt-nos* (Sequence ID No. 13)
**Figure 22** shows vector details for *pBMVhAtMYB32xs::ipt-nos* [Gene: Isopentyl transferase (*IPT*); Vector: pBMVhAtMYB32XS::ipt-nos (backbone pPZPRCS2); Selectable marker: spec; Plant selectable marker cassette: 35S::hph::35ST; Gene promoter: AtMYB32-xs; Gene terminator: nos.]

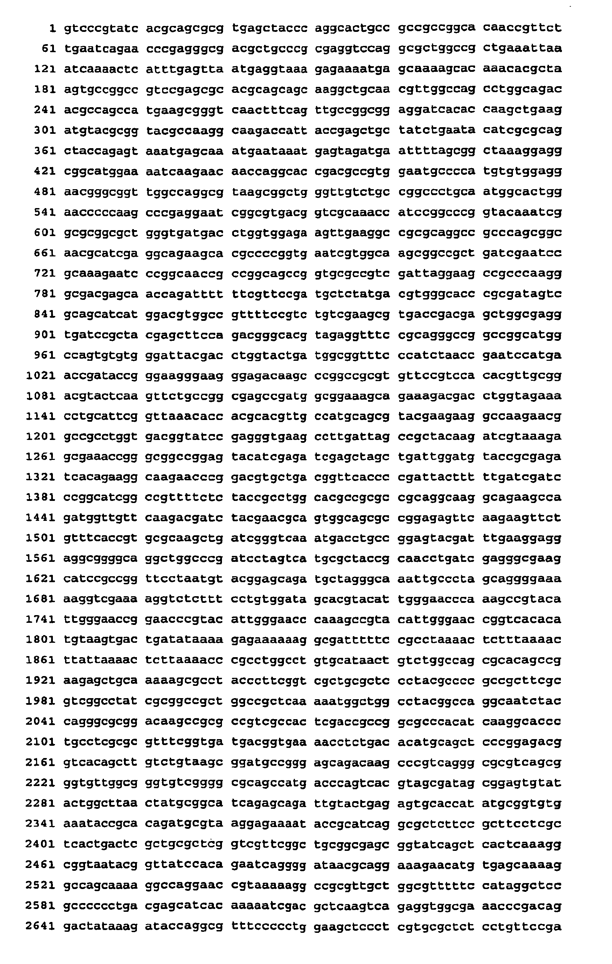

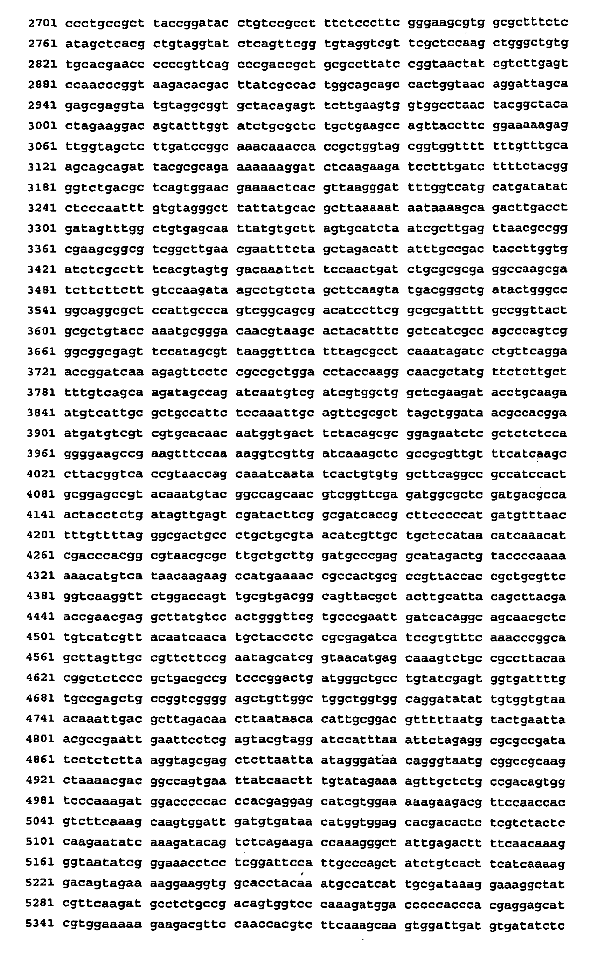

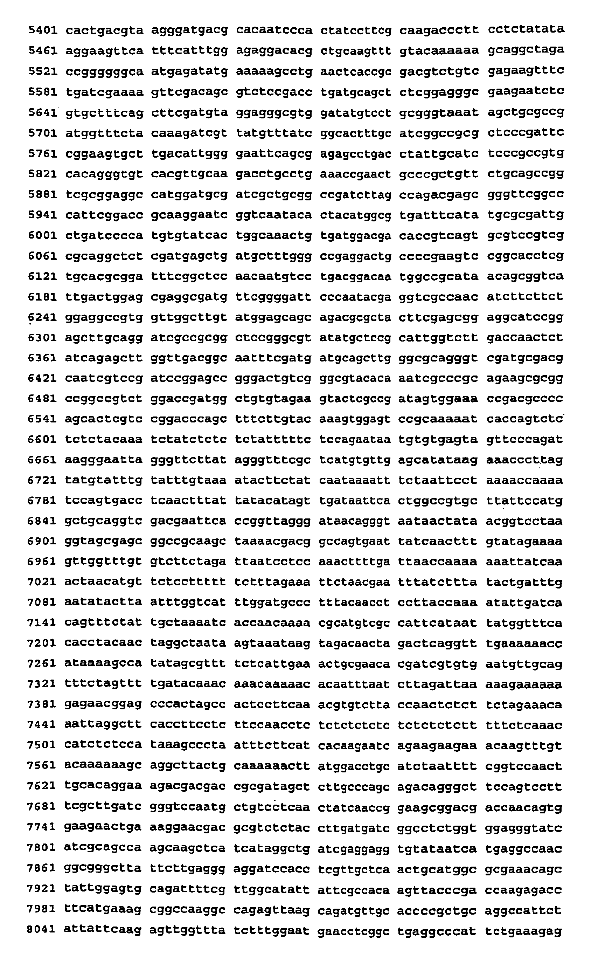

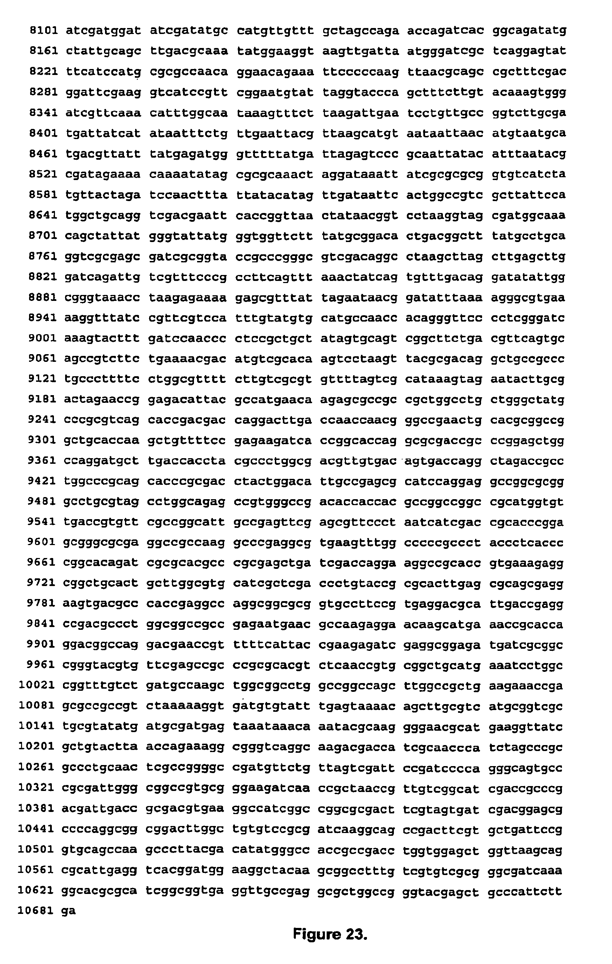

**Figure 23** shows nucleotide sequence of vector *pBMVhAtMYB32xs::ipt-nos* (Sequence ID No. 14)
**Figure 24** shows vector details for *pBSubn-AtMYB32-900::ipt-nos* [Gene: Isopentyl transferase *(IPT)*; Vector: pBSubn-AtMYB32-900::ipt-nos (backbone pPZPRCS2); Selectable marker. spec; Plant selectable marker cassette: Ubi::bar::nos; Gene promoter: AtMYB32-900; Gene terminator: nos.]
**Figure 25** shows nucleotide sequence of vector *pBSAtMYB32900::ipt-nos* (Sequence ID No. 15)
**Figure 26** shows generation of transgenic canola containing the *pBMVhATMYB3-900::ipt-nos* and *pBMVhATMYB32xs::ipt-nos.* **A**. Canola seeds are germinated *in vitro;* **B**. Hypocotyl sections are excised from 7-day-old seedlings and inoculated with an *Agrobacterium* suspension; **C&D**. Regeneration from inoculated hypocotyl sections under hygromycin selection; **E-J.** Transgenic To canola plants carrying the *pBMVhATMYB3-900::ipt-nos* and *pBMVhATMYB32xs::ipt-nos* vectors.
**Figure 27** shows a process for biolistic transformation of wheat.
**Figure 28** shows biolistic transformation of wheat (*Triticum aestivum* L. MPB Bobwhite 26). Donor plant production **(A & B);** zygotic embryo isolation **(C&D);** Regeneration under glufosinate selection **(E-G);** Root formation under selection **(H)**; To plants growing under containment glasshouse conditions for recovery of transgenic offspring **(I).**
**Figure 29** shows contained field trial of transgenic white clover plants expressing chimeric Atmyb32::ipt genes.
**Figure 30** shows comparative assessment of growth rates and growth dynamics of transgenic white clover plants expressing chimeric Atmyb32::ipt genes with non-transgenic control white clover plants. A) Growth Rates B) Growth Dynamics C)
   Growth Characteristics (after 45 days) □ Transgenic white clover ■ Non-transgenic control white clover (light) (dark)
**Figure 31** shows flowering intensity (i.e. number of ripe flower per m²) of transgenic white clover plants expressing chimeric Atmyb32::ipt genes (i.e. LXR 12, LXR 18 and LXR 11) and non-transgenic control white clover plants (i.e. WT) under contained field conditions.
**Figure 32** shows seed weight (i.e. weight of thousand seeds, in grams) of transgenic white clover plants expressing chimeric Atmyb32::ipt genes (i.e. LXR 12, LXR 18 and LXR 11) and non-transgenic control white clover plants (i.e. WT) under contained field conditions.
**Figure 33** shows seed yield per flower (in milligrams) of transgenic white clover plants expressing chimeric Atmyb32::ipt genes (i.e. LXR 12, LXR 18 and LXR 11) and non-transgenic control white clover plants (i.e. WT) under contained field conditions.
**Figure 34** shows seed yield per area (in kg/ha) of transgenic white clover plants expressing chimeric Atmyb32::ipt genes (i.e. LXR 12, LXR 18 and LXR 11) and non-transgenic control white clover plants (i.e. WT) under contained field conditions.
**Figure 35** shows generation of transgenic alfalfa plants containing the chimeric *pBMVkATMYB3-900::ipt-nos* and *pBMVkATMYB32xs::ipt-nos* genes **A.** Petiole explants from alfalfa clones C2-3, C2-4 and 19-17 are used for inoculation with an *Agrobacterium* suspension and lead to the production of transformed embryogenic calli following selection in presence of kanamycin; **B-D.** Regeneration of transgenic alfalfa plantlets carrying chimeric genes from *pBMVkATMY83-900::ipt-nos* and *pBMVkATMYB32xs::ipt-nos* vectors, from somatic embryos grown *in vitro.*
**Figure 36** shows PCR analysis of transgenic canola plants (T1 LXR canola plants Line 4). Genomic DNA was isolated from different transgenic canola plants of T₁ LXR04 lines and subjected to PCR using primers specific for **A**. the selectable marker (*hph*) or **B**. the candidate gene of interest (*IPT*).
**Figure 37** shows expression analysis of the *IPT* gene in T₁ transgenic canola plants (T1 LXR canola relative IPT leaf expression).
**Figure 38** shows transgenic canola displaying delay of detached cotyledon senescence as compared to wild-type control cotyledons 7 days following detachment.
**Figure 39** shows the senescence score of T₁ transgenic canola cotyledons at 7 days following detachment.
**Figure 40** shows transgenic canola displaying delay of detached juvenile leaf senescence as compared to wild-type control cotyledons 14 days following detachment.
**Figure 41** shows senescence score of T₁ transgenic canola juvenile first leaves at 14 days following detachment.
**Figure 42** shows Southern hybridisation analysis of transgenic wheat lines. Lanes include: MW - molecular weight; WT - wild-type; Transgenic wheat lines LXR3, LXR4, LXR13, LXR16 and positive plasmid controls containing 10 and 20 pg.
**Figure 43** shows expression analysis of independent T₁ transgenic wheat lines (IPT quantitative expression in wheat). Quantitative transcript values were determined in femtomoles (fmol) per microgram of RNA using a standard curve derived from plasmid DNA containing the target sequence. Samples represent high, medium and low expression classes for candidate gene *IPT*.
**Figure 44** shows phenotypic variation of glasshouse grown T₁ transgenic wheat plants. **A.** T₁ LXR 13 wheat plants displaying normal phenotype as compared to null control wheat plants. **B.** T₁ LXR 04 wheat plants displaying stunted phenotype and increased flag leaf number as compared to null control wheat plants.
**Figure 45** shows transgenic wheat displaying delay of leaf senescence as compared to null control leaves 7 days following detachment.

### Examples

Examples not pertaining to the invention claimed are for illustrative purposes only

### Example 1

### Atmyb32 promoter sequence and promoter sequence variants

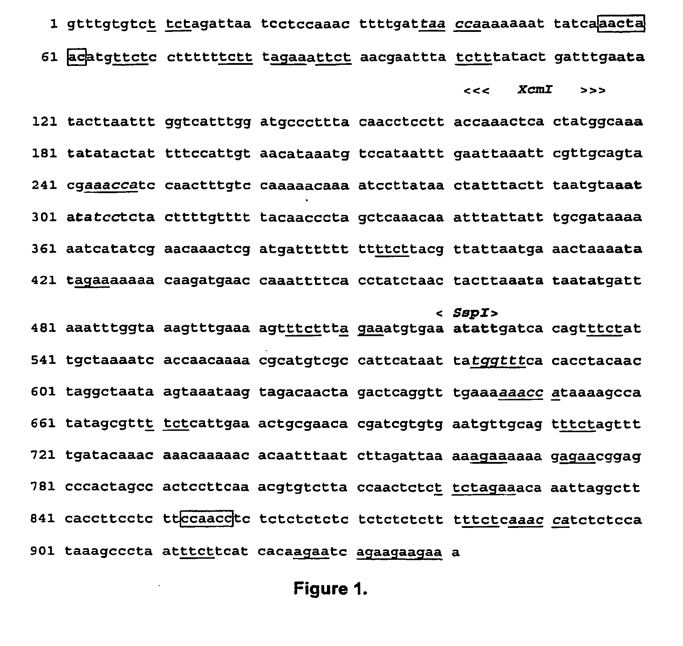

The Atmybb32 promoter sequence and variants thereof are shown In Figures 1-4.

### Example 2

### Cytokinin biosynthesis genes

Examples of cytokinin biosynthesis genes suitable for use in the present invention are shown in Figures 6, 8 and 10. Suitable genes also include those encoding the polypeptides shown in Figures 7, 9 and 11.

### Example 3

### Production of transgenic white clover plants

Transgenic white clover plants (*Trifolium repens* cv. Haifa and Irrigation) were produced by *Agrobacterium*-mediated transformation using a binary vector carrying the chimeric *atmyb32::ipt* gene (Figure 12a). The transgenic plants were screened by PCR using *Ipt* and *nptII* primers (Figure 12b). *Hind*III digested genomic DNA samples subjected to Southern DNA hybridization analysis showed that the DNA fragments greater than 4.4 kb were detected in all lanes by both *ipt* and *nptII* probes, demonstrating the presence and integration of full-length T-DNA into the white clover genome (Figure 12). Transgenic lines Hmi01, Imi06, Imi11, and Imi18 (Lane 1, 3, 5, 8 and 12 respectively) appeared to have a single copy of full-length T-DNA integrated in the genome. Other transgenic lines had multiple copies of the *atmyb32::ipt* transgene.

### Example 4

### IPT gene expression in transgenic white clover plants

The expression of the *atmyb32::ipt* transgene in transgenic white clover (*T*. *repens*) plants was assessed by RT-PCR. The *ipt* mRNA was detected in leaf tissues of all *atmyb32::ipt* transgenic white clover plants examined, with varying levels of PCR products detected (Figure 13).

### Example 5

### Delayed detached leaf senescence in transgenic white clover plants

Experiments were performed to assess detached leaf senescence of *atmyb32::ipt* transgenic plants. Rapid yellowing was observed in detached leaves from non-transformed and *atmyb32::gusA* transgenic white clover plants of both cultivars within one week. Transgenic lines Hmi01, Hmi08, Imi16 and Imi18 showed delayed senescence while Imi11 and Imi12 showed no sign of yellowing by the end of 7 days. After two weeks, the leaves of all *atmyb32::ipt* transgenic plants were much greener than those of non-transformed and *atmyb32::gusA* control transgenic plants (Figure 14). The degree of senescence in excised leaves was in the order HC, Hmg > Hmi01 > Hmi08 for cv. Haifa, and IC and Img > Imi16 > Imi18 > Imi11 and Imi12 for cv. Irrigation. HC is Haifa untransformed control, Hmg is Haifa *atmyb32::gusA* control, IC is Irrigation untransformed control, Img is Irrigation *atmyb32::gusA* control. Hmi01, Hmi08, Imi16, Imi18, Imi11 and Imi12 are independent *atmyb32::ipt* transgenic white clover plants from the cultivar Haifa (H) and Irrigation (I), respectively.

### Example 6

### Plant morphology and root development in transgenic white clover plants

Normal plant morphology as well as normal shoot and normal root development was observed in *atmyb32:ipt* transgenic white clover plants (Figure 6), thus indicating that the regulated expression of the ipt gene under control of the *atmyb32* promoter did not negatively affect neither rooting nor apical dominance of the transgenic white clover plants (Table 1).

**Table 1**

| **Transformant** | **Cultivar** | **Construct** | ***ipt* copy No** | **Phenotype** |
|---|---|---|---|---|
| Hmi01 | Haifa | *Atmyb32::ipt* | 1 | Normal |
| Hmi08 | Haifa | *Atmyb32::ipt* | >3 | Normal |
| Imi06 | Irrigation | *Atmyb32::ipt* | 1 | Normal |
| Imi07 | Irrigation | *Atmyb32::ipt* | 3 | Normal |
| Imi09 | Irrigation | *Atmyb32::ipt* | >3 | Normal |
| Imi10 | Irrigation | *Atmyb32::ipt* | >3 | Normal |
| Imi11 | Irrigation | *Atmyb32::ipt* | 1 | Normal |
| Imi12 | Irrigation | *Atmyb32::ipt* | 2 | Normal |
| Imi16 | Irrigation | *Atmyb32::ipt* | 2 | Normal |
| Imi18 | Irrigation | *Atmyb32::ipt* | 1 | Normal |

Normal plant morphology and normal rooting was observed in ten independent *atmyb32::ipt* transgenic white clover lines analysed. Estimated *ipt* gene copy numbers in the ten independent *atmyb32::ipt* transgenic white clover lines are shown.

### Example 7

### Generation of vectors for plant transformation

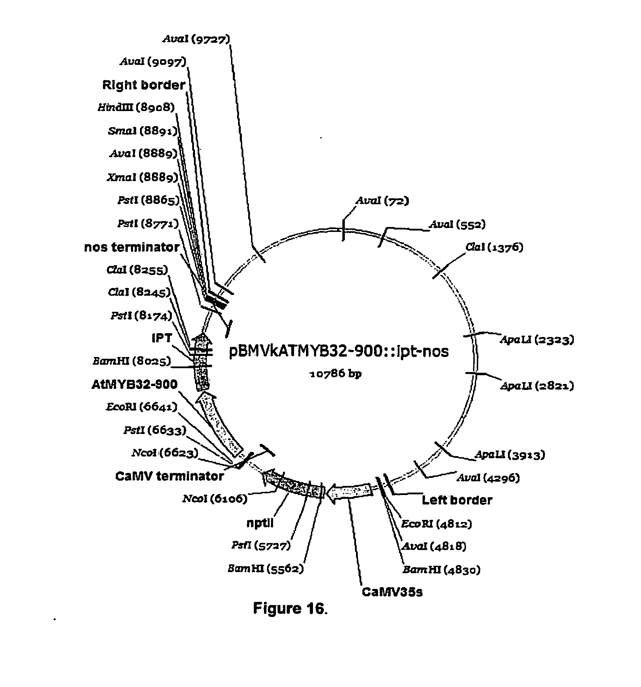

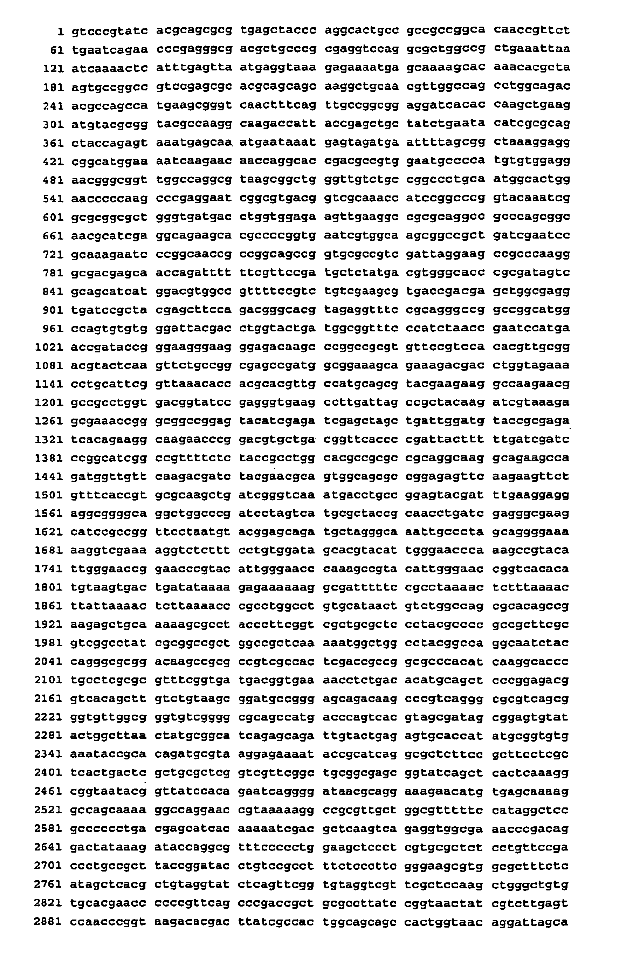

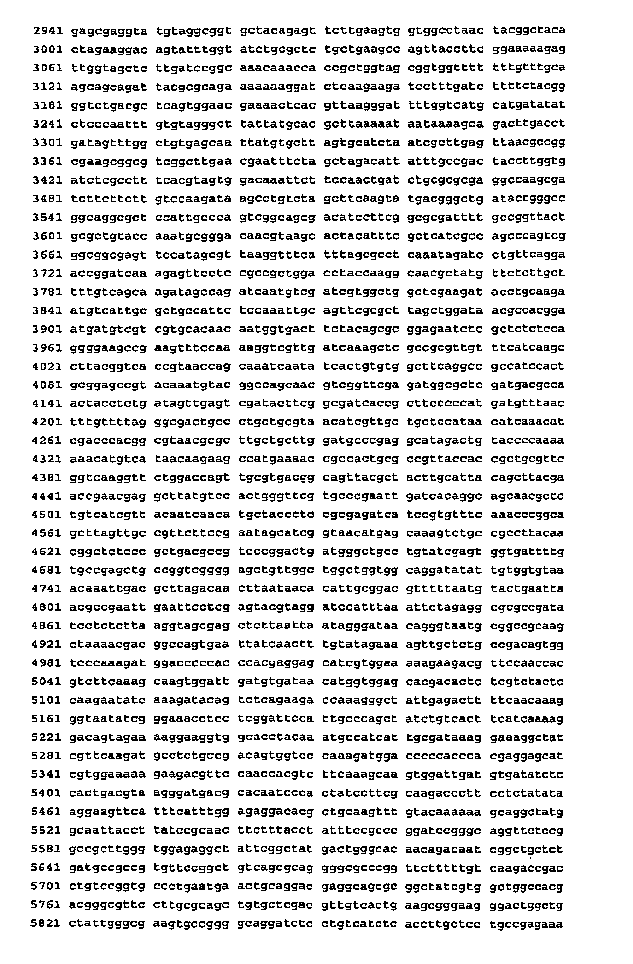

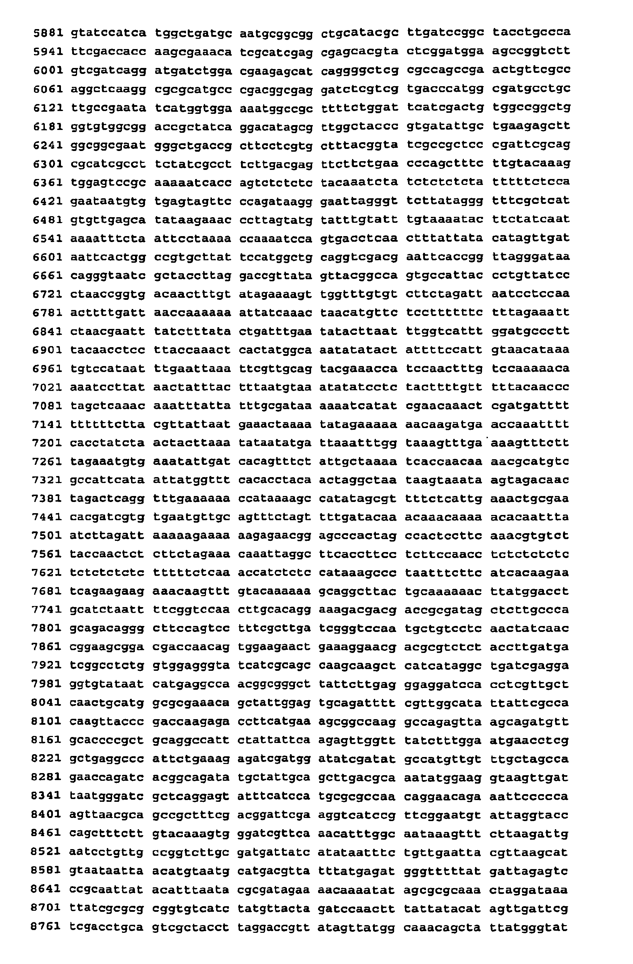

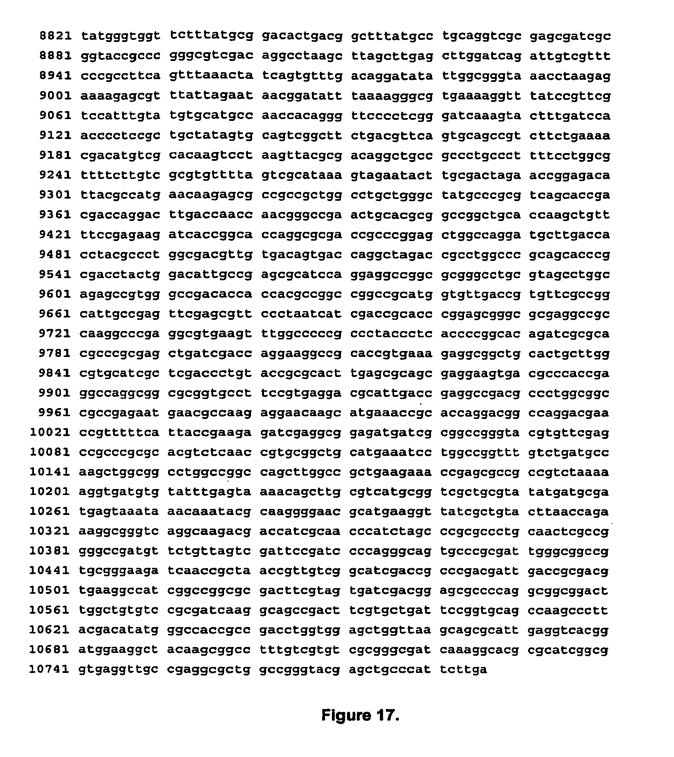

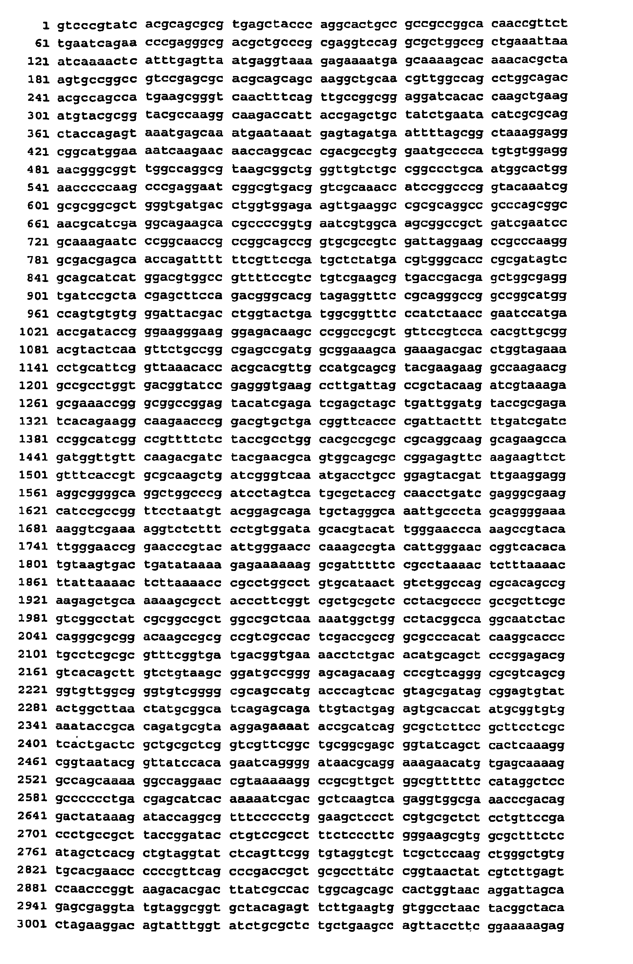

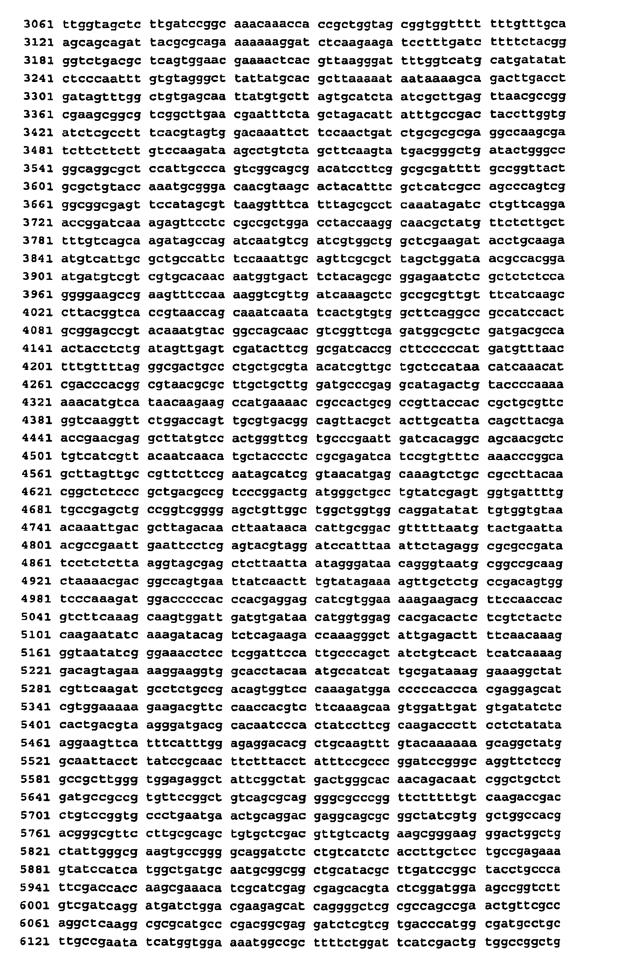

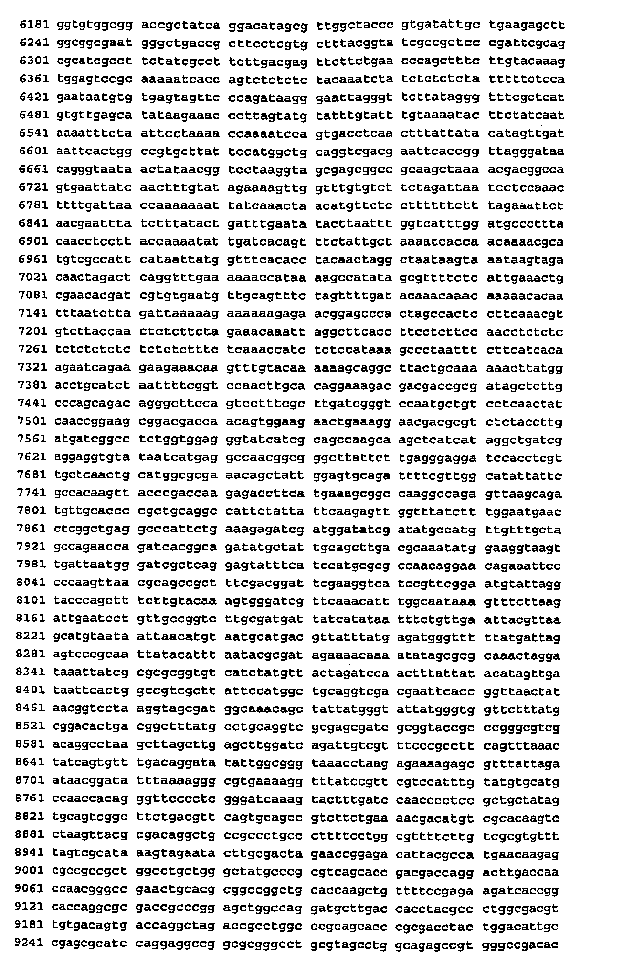

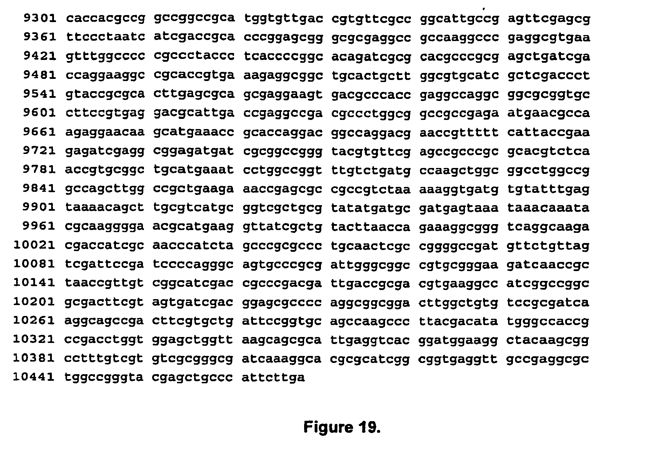

Four binary vectors have been generated for *Agrobacterium-mediated* transformation of plants (Figures 16-19). Each vector has a pPZP200 vector backbone (Hajdukiewicz *et al.,* 1994) and contains either chimeric *Atmyb32-900::ipt-nos* or *Atmyb32-xs::ipt-nos* with or without a chimeric *35S::nptll-35st* or *35S::hph-35st* selectable marker cassettes.

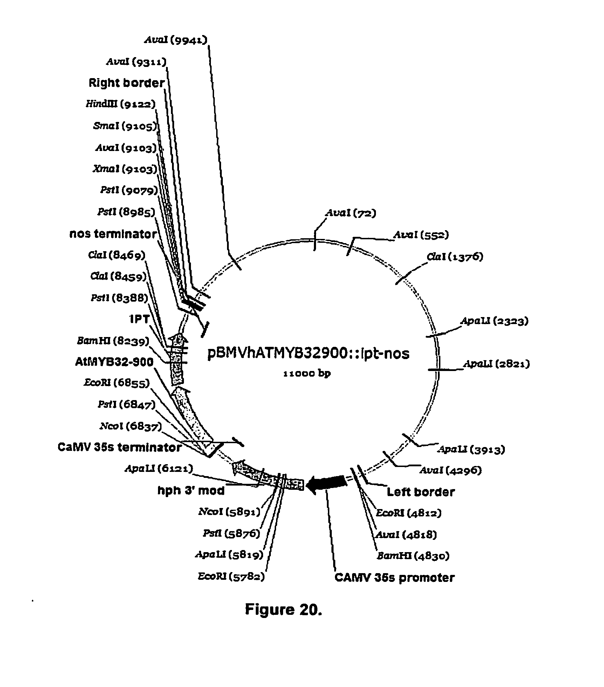

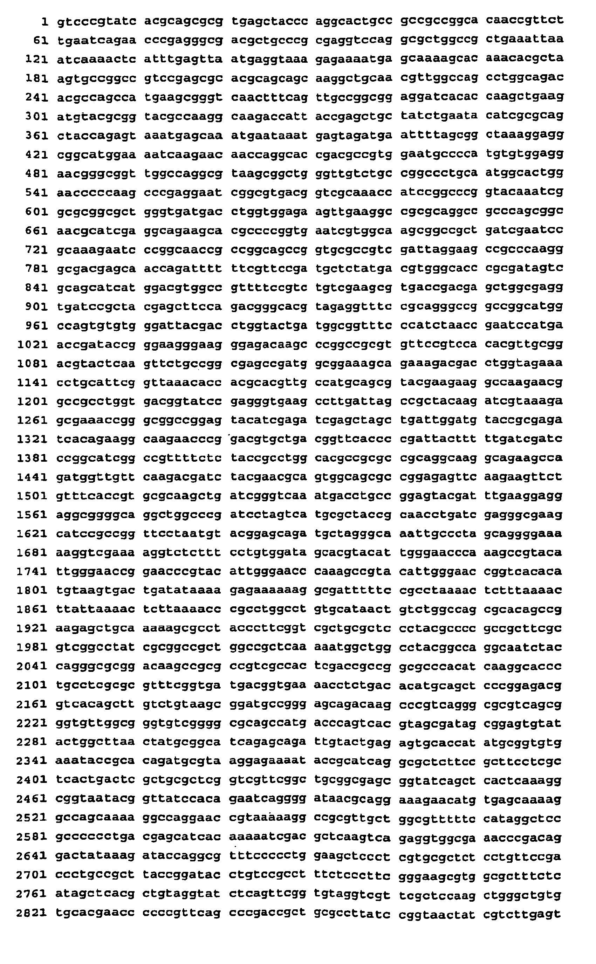

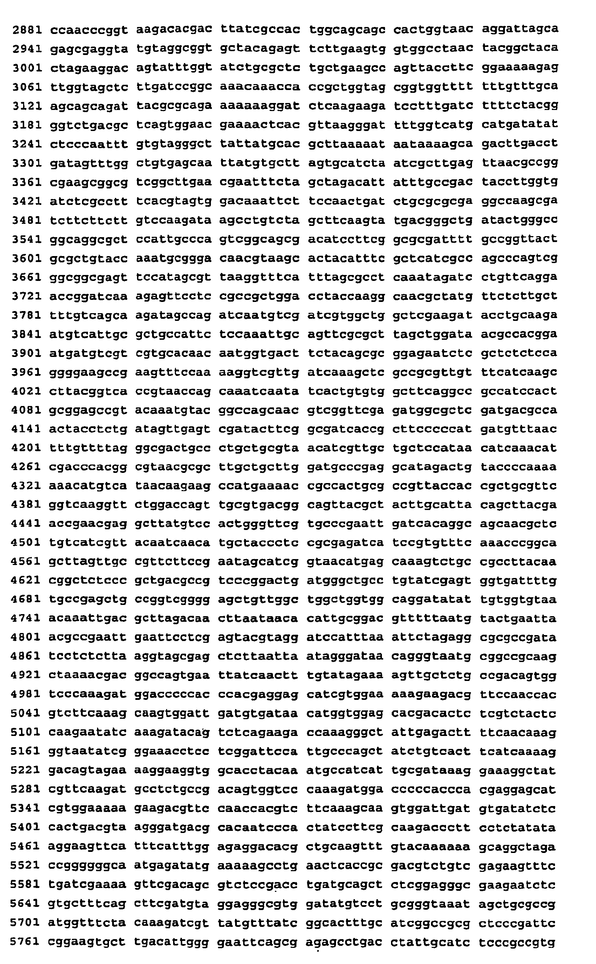

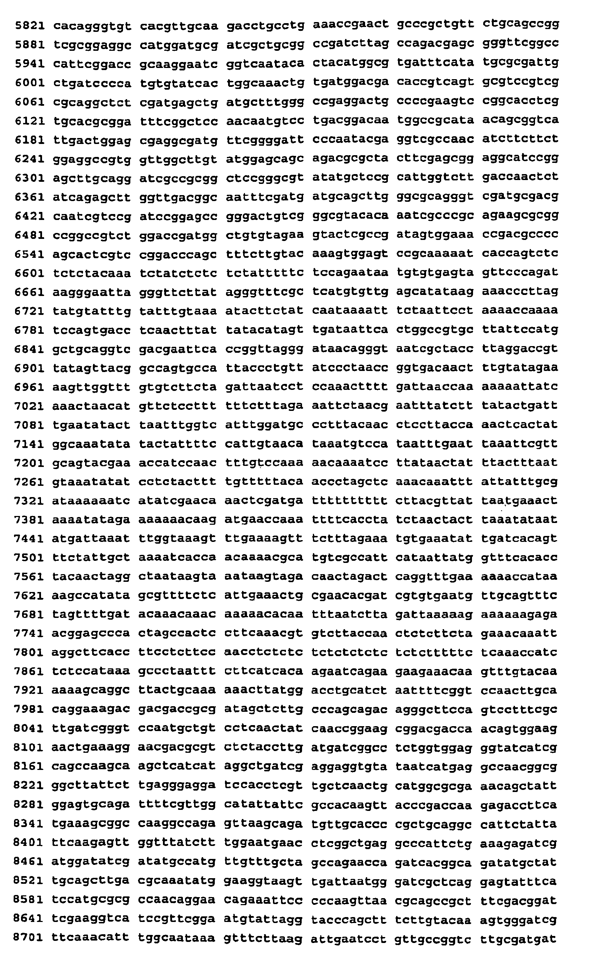

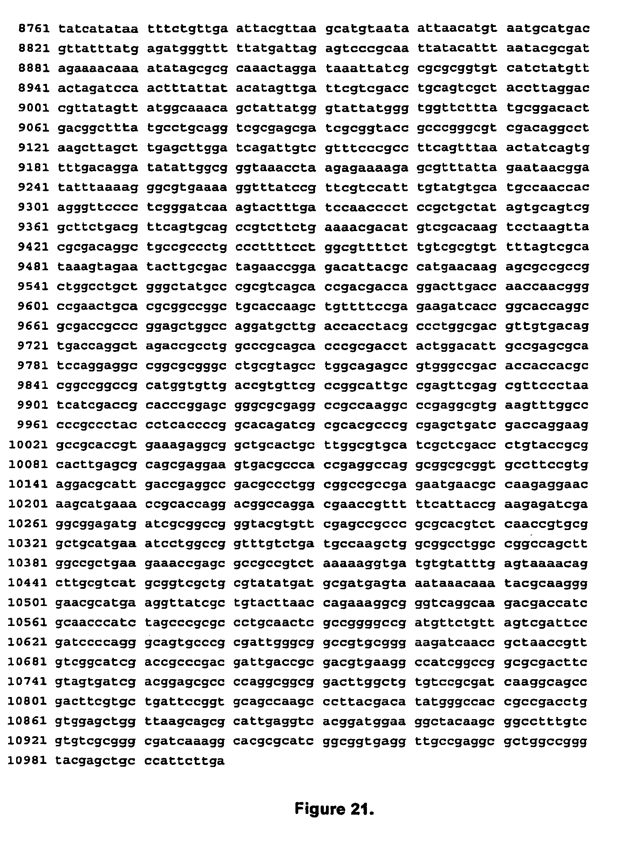

One transformation vector has been constructed for biolistic transformation (Figures 20 and 21). The transformation vector contains chimeric *Atmyb32-900::ipf-35st* with a chimeric *Ubi:bar-nos* selectable marker cassette.

The *Atmyb32* promoter, promoter variant *Atmyb32xs,* the isopentyl transferase gene and terminators *35st* and *nos* were amplified by PCR using Gateway™ (Invitrogen) adapted primers and cloned into a pDONR221 entry vectors. These were subsequently cloned using recombination into destination vectors containing the conventionally cloned selectable marker cassettes. All vectors were fully sequenced following strict quality assurance protocols.

### Example 8

### Agrobacterium-mediated transformation of canola (Brassica napus)

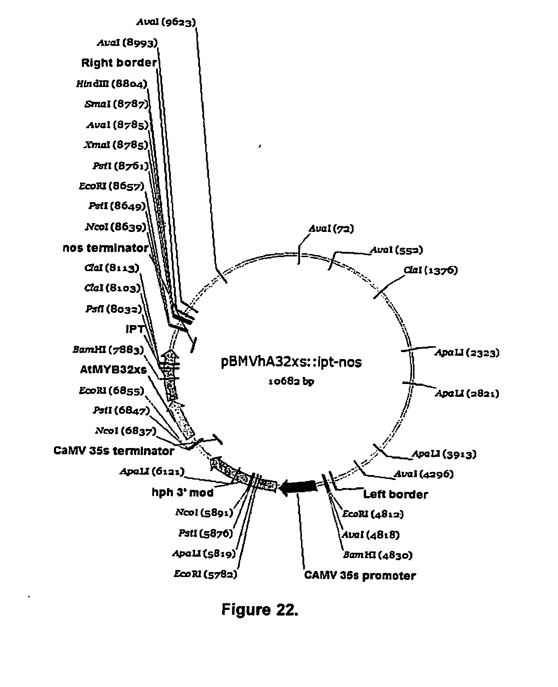

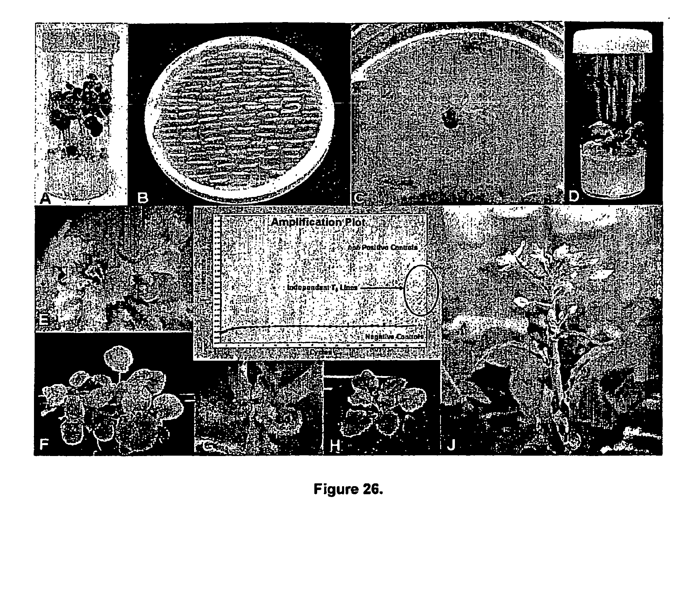

Binary vectors *pBMVhATMYB3-900::ipt-nos* (Figure 20) and *pBMVhATMY832xs::ipt-nos* (Figure 22) containing chimeric *ipt* genes under control of *Atmyb32* promoter (Figure 1) and Atmyb32xs variant promoter sequence with deleted root-specific motifs (Figure 2) were used for *Agrobacterium-mediated* transformation of *Brassica napus* hypocotyl segments (Figure 26).

*Brassica napus* seeds are surface sterilised in 70% ethanol for 2 minutes, washed 3 times in sterile water then further surface sterilised in a solution containing 1% (w/v) Calcium hypochlorite and 0.1 % (v/v) Tween 20 for 30 minutes. The seeds are washed at least 3 times in sterile water and planted in 120 ml culture vessels containing a solidified germination medium containing 1x Murashige and Skoog (Murashige and Skoog Physiol. Plant, 15: 473-497, 1962) macronutrients, 1x micronutrients and B5 organic vitamins, supplemented with 500 mg/L MES, 2% (w/v) sucrose at a pH of 5.8 with the addition of 4 g/L Gelrite. The vessels are incubated at 25 °C under 16h light/8h dark conditions for 7 days to encourage germination.

After 7 days, seedlings of *Brassica napus* (whole seedlings) are transferred to a liquid medium consisting of 1x Murashige and Skoog macronutrients, 1x micronutrients and B5 organic vitamins, supplemented with 500 mg/L MES, 3% (w/v) sucrose at a pH of 5.8. Seedlings are grouped together and the roots and cotyledons removed prior to cutting the hypocotyls into 7-10mm sections and plating on 9 X 1.5 cm petri dishes containing a preconditioning medium consisting of 1x Murashige and Skoog macronutrients, 1x micronutrients and B5 organic vitamins, supplemented with 500 mg/L MES, 3% (w/v) sucrose at a pH of 5.8 solidified with 6.4 g/l Bacto-Agar.

Hypocotyl sections are cultured for 24 hours prior to inoculation with an *Agrobacterium* suspension OD₆₀₀ = 0.2 for 30 minutes consisting of 1 x Murashige and Skoog macronutrients, 1 x micronutrients and B5 organic vitamins, supplemented with 500 mg/L MES, 100 µM Acetosyringone, 3% (w/v) sucrose at a pH of 5.8.

Following inoculation, hypocotyl sections are blotted on sterile paper towels and transferred to 9 x 1.5 cm petri dishes containing 1x Murashige and Skoog macronutrients, 1x micronutrients and B5 organic vitamins, supplemented with 500 mg/L MES, 100 µM Acetosyringone, 1 mg/L 2,4-D, 3% (w/v) sucrose at a pH of 5.8 solidified with 8 g/l Bacto-Agar. Explants are incubated at 25°C under 16h light/8h dark conditions for 72 hours for co-cultivation.

Following co-cultivation, 20-30 hypocotyl explants are transferred to 9 X 1.5 cm petri dishes containing a solidified selection medium consisting of 1x Murashige and Skoog macronutrients, 1x micronutrients and B5 organic vitamins, supplemented with 500 mg/L MES, 1 mg/L 2,4-D, 3% (w/v) sucrose at a pH of 5.8 solidified with 8 g/l Bacto-Agar, supplemented with 250 mg/l timentin and 10 mg/l hygromycin to select for hygromycin-resistant shoots. Plates are incubated at 25°C under 16h light/8h dark conditions.

After 7 days hypocotyl explants are transferred to 9 X 2.0cm petri dishes containing a solidified regeneration media consisting of 1x Murashige and Skoog macronutrients, 1x micronutrients and B5 organic vitamins, supplemented with 500 mg/L MES, 1 mg/L 2,4-D, 3% (w/v) sucrose at a pH of 5.8 solidified with 8 g/l Bacto-Agar, supplemented with 4 mg/l BAP, 2 mg/l Zeatin, 5 mg/l Silver Nitrate, 250 mg/l timentin and 10 mg/l hygromycin. Plates are incubated under direct light at 25°C under fluorescent light conditions (16 hr light/8 hr dark photoperiod; 55 µmol m⁻² sec⁻¹) for 4 weeks to encourage shoot development.

Regeneration is monitored weekly and hypocotyl explants transferred to fresh 9 X 2.0cm petri dishes containing solidified regeneration media, RM supplemented with 4 mg/l benzyladenine, 2 mg/l zeatin, 5 mg/l silver nitrate, 250 mg/l timentin and 10 mg/l hygromycin for 6-8 weeks to encourage shoot development.

Hygromycin-resistant (Hyg^{r}) shoots are transferred to 120 ml vessels containing solidified root induction medium, RIM1, consisting of 1x Murashige and Skoog macronutrients, 1x micronutrients and B5 organic vitamins, supplemented with 500 mg/L MES, 1 mg/L 2,4-D, 1% (w/v) sucrose at a pH of 5.8 solidified with 8 g/l Bacto-Agar supplemented with 250 mg/l timentin. Shoots are incubated under direct fluorescent light at 25°C (16 hr light/8 hr dark photoperiod; 55 µmol m⁻² sec⁻¹) to encourage shoot elongation and root development over 4-5 weeks. All Hyg^{r} shoots with developed shoot and root systems are transferred to soil and grown under glasshouse conditions.

### Example 9

### Biolistic transformation of wheat (Triticum aestivum L.)

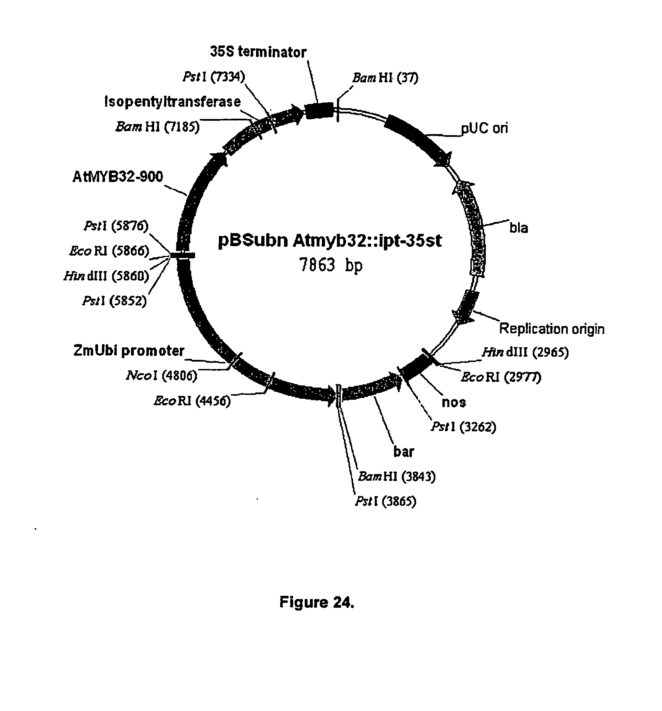

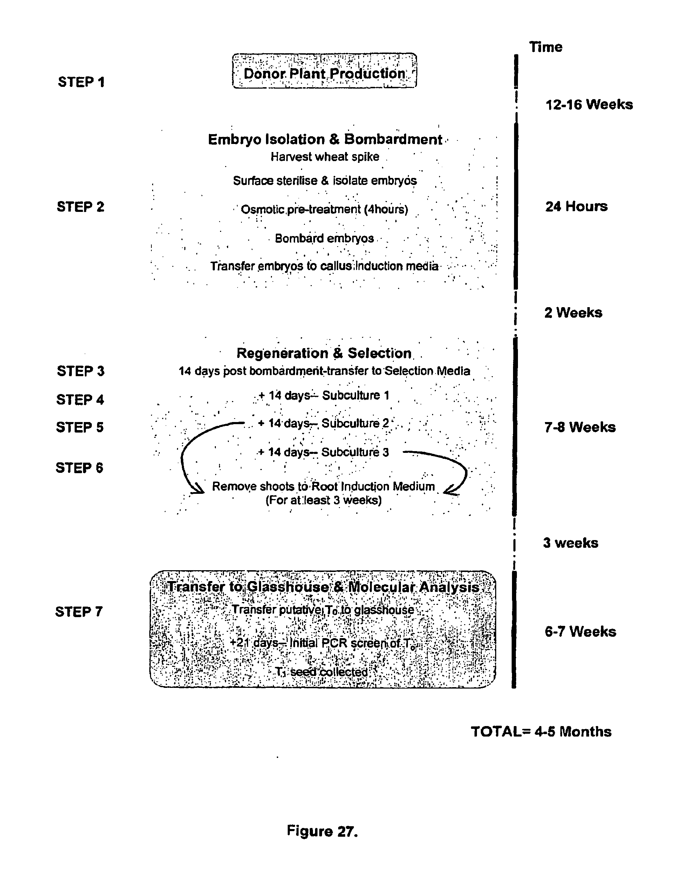

Transformation vectors containing chimeric *ipt* genes under control of *Atmyb32* promoter (Figure 1) and Atmyb32xs variant promoter sequence with deleted root-specific motifs (Figure 2) were used for biolistic transformation of wheat (*Triticum aestivum* L. MPB Bobwhite 26). A representative vector is shown in Figure 24. A schematic of the procedure for biolistic transformation of wheat is outlined in Figure 27. The transformation procedure includes the following steps:

### Step 1 (Donor Plant Production):

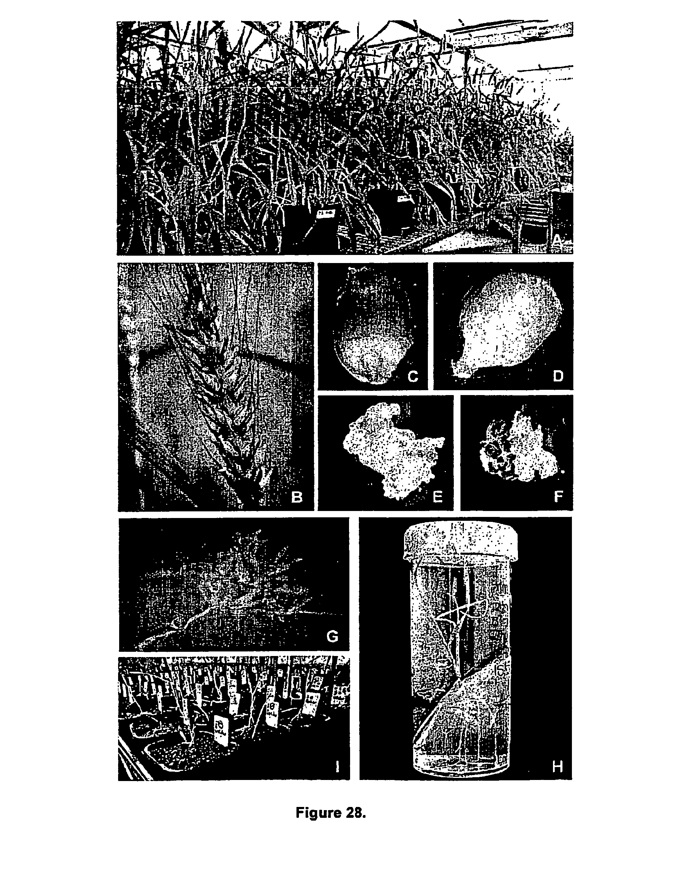

*Triticum aestivum* (Bobwhite 26) seed is used for the production of donor plant material. Wheat plants are grown in a nursery mix consisting of composted pine bark, perlite and vermiculite, with five plants per pot to a maximum pot size of 20 cm. Plants are kept under glasshouse conditions at approximately 22-24° C for 12-16 weeks (Figure 28A). Once the first spike emerges from the flag leaf, plants are tagged and embryos collected from the tallest heads 12-15 days post anthesis.

### Step 2 (Day 1)

Spikes at the desired stage of development are harvested (Figure 28B). Caryopsis are removed from the spikes and surface sterilised for 20 minutes in a 0.8% (v/v) NaOCl solution and rinsed at least four times in sterile distilled water.

Embryos up to 10 mm in length are aseptically excised from each caryopsis (removing the axis) using a dissecting microscope and cultured axial side down on an osmotic medium (E3maltose) consisting of 2x Murashige and Skoog (1962) macronutrients, 1x micronutrients and organic vitamins, 40 mg/L thiamine, 150 mg/L L-asparagine, supplemented with 15% (w/v) maltose, 0.8% (w/v) Sigma-agar and 2.5 mg/L 2,4-D (Figure 28C&D). Embryos are cultured on 60 mm x 15 mm clear polypropylene petrie dishes with 15 mL of media. Culture plates are incubated at 24 °C in the dark for 4 hours prior to bombardment. Embryos are bombarded using a BioRad PDS1000 gene gun at 900 psi and at 6 cm with 1 µg of vector plasmid DNA precipitated onto 0.6 µm gold particles. Following bombardment, embryos are incubated overnight in the dark on the osmotic media.

### Step 3 (Day 2):

Embryos are transferred to a callus induction medium (E3calli) consisting of 2x Murashige and Skoog (1962) macronutrients and 1x micronutrients and organic vitamins, 40 mg/L thiamine, 150 mg/L L-asparagine, supplemented with 6% (w/v) sucrose, 0.8% (w/v) Sigma-agar and 2.5 mg/L 2,4-D. Embryos are cultured for two weeks at 24 °C in the dark.

### Step 4 (Day16):

After 2 weeks of culture on E3calli, embryos have produced embryogenic callus and are subcultured onto a selection medium (E3Select) consisting of 2x Murashige and Skoog (1962) macronutrients and 1x micronutrients and organic vitamins, 40 mg/L thiamine, 150 mg/L L-asparagine, supplemented with 2% (w/v) sucrose, 0.8% (w/v) Sigma-agar, 5 mg/L of D,L phosphinothricin (PPT) and no plant growth regulators (Figure 28E-G). Cultures are incubated for further 14 days on E3Select at 24 °C in the light and a 12-hour photoperiod.

### Step 5 (Day 30):

After 14 days culture on E3Select, embryogenic callus is sub-cultured onto fresh E3Select for a further 14 days (Figure 28E-G).

### Step 6 (Day 44):

After about 4 weeks on E3Select, developing plantlets are excised from the embryonic callus mass and grown for a further three weeks in 65 mm X 80 mm or 65 mm x 150 mm polycarbonate tissue culture vessels containing root induction medium (RM). Root induction medium consists of 1x Murashige and Skoog (1962) macronutrients, micronutrients and organic vitamins, 40 mg/L thiamine, 150 mg/L L-asparagine, supplemented with 2% (w/v) sucrose, 0.8% (w/v) Sigma-agar, and 5 mg/L of PPT (Figure 28H). Remaining embryogenic callus is sub-cultured onto E3Select for another 14 days.

### Step 7 (Day 65+):

Regenerated plantlets surviving greater than 3 weeks on RM with healthy root formation are potted into a nursery mix consisting of peat and sand (1:1) and kept at 22-24 °C with elevated humidity under a nursery humidity chamber system (Figure 28). After two weeks, plants are removed from the humidity chamber and hand watered and liquid fed Aquasol™ weekly until maturity. The T₀ plants are sampled for genomic DNA and molecular analysis. T₁ seed is collected and planted for high-throughput Q-PCR analysis (Figure 28J).

### Example 10

### Agronomic performance of transgenic white clover plants

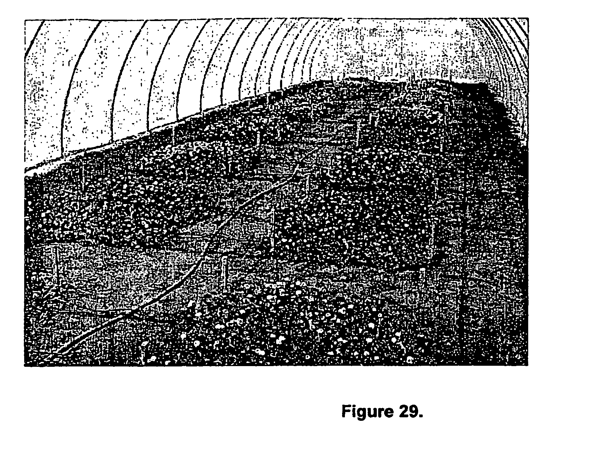

The agronomic performance of *atmyb32::ipt* transgenic white clover (*Trifolium repens*) plants, relative to that of non-transgenic control white clover plants, was evaluated under environmentally controlled growth chamber conditions and in contained field trials (Figure 29).

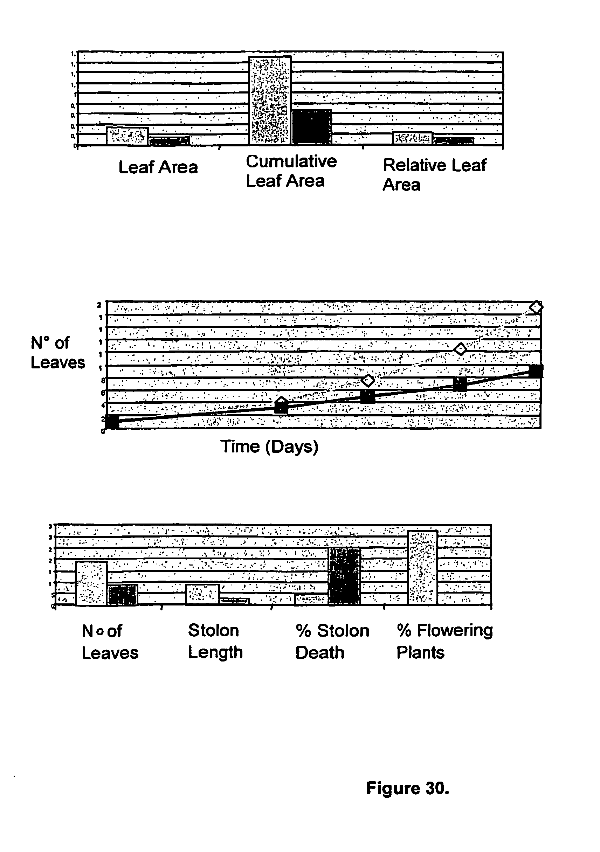

Transgenic white clover plants expressing chimeric Atmyb32::ipt genes assessed under controlled growth chamber conditions revealed a significantly enhanced biomass accumulation and reductions in manifestations of senescence, when compared with non-transgenic control white clover plants (Figure 30). The transgenic white clover plants expressing chimeric Atmyb32::ipt genes showed enhanced total leaf area, increased cumulative leaf area, higher leaf growth dynamics (i.e. number of leaves over time), higher stolon length and increased % flowering plants as well as reduced stolon senescence and death compared with non-transgenic control white clover plants (Figure 30A-C).

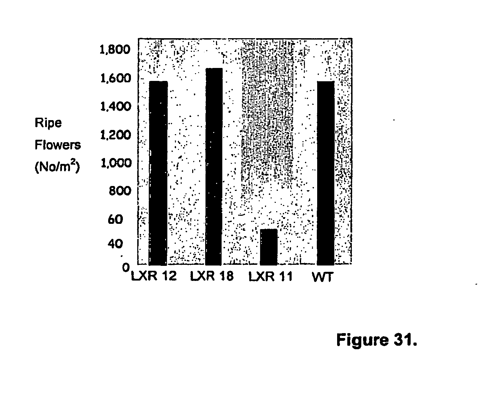

The seed yield performance of 3 independent *atmyb32::ipt* expressing transgenic white clover plants (i.e. LXR 12, LXR 18 and LXR 11) was also comparatively assessed with non-transgenic control plants (i.e. wild type, WT) under contained field conditions. Two independent *atmyb32::ipt* expressing transgenic white clover plants (i.e. LXR 12 and LXR 18) with indistinguishable flowering intensity (i.e. number of ripe flowers per m²) to the non-transgenic control plant (i.e. WT) were selected for field evaluation (Figure 31).

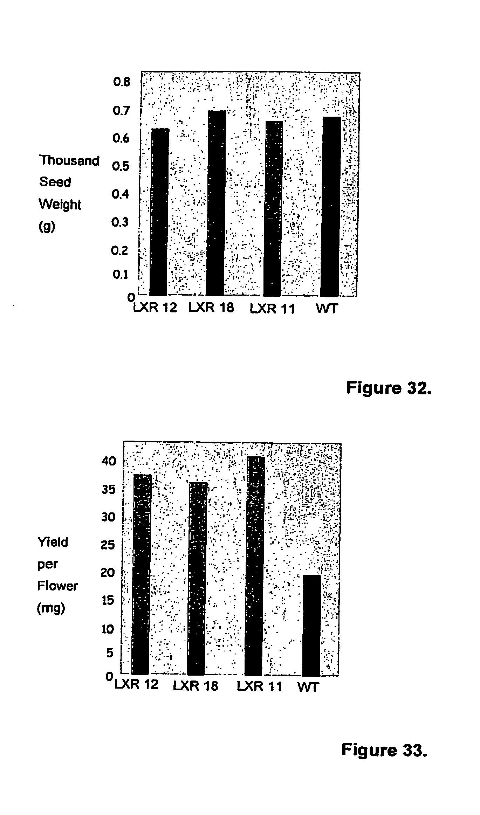

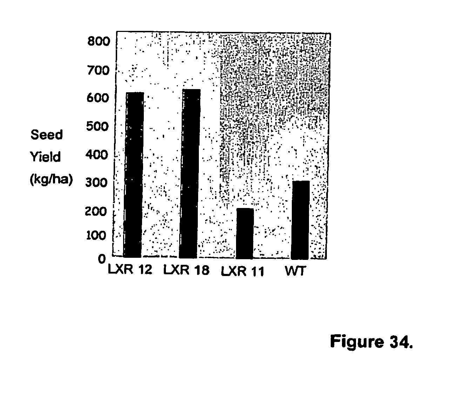

While the seed weight (i.e. weight of thousand seeds) of transgenic white clover plants expressing chimeric Atmyb32::ipt genes (i.e. LXR 12, LXR 18 and LXR 11) was indistinguishable from non-transgenic control white clover plants (i.e. WT) (Figure 32), the total seed yield expressed on the basis of per flower (Figure 33), and per area sown (Figure 34) was doubled in transgenic white clover plants expressing chimeric Atmyb32::ipt genes (i.e. LXR 12 and LXR 18) when compared with non-transgenic control white clover plants of equivalent flowering intensity (i.e. WT).

### Example 11

### Agrobacterium-mediated transformation of alfalfa (Medicago sativa)

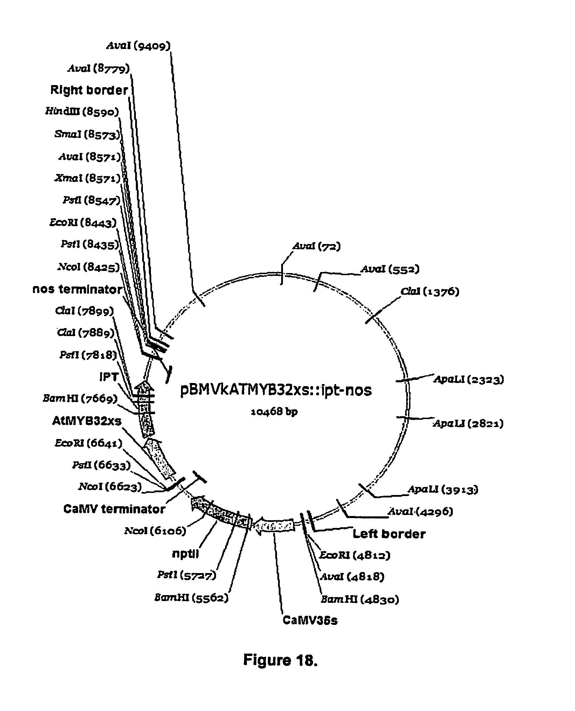

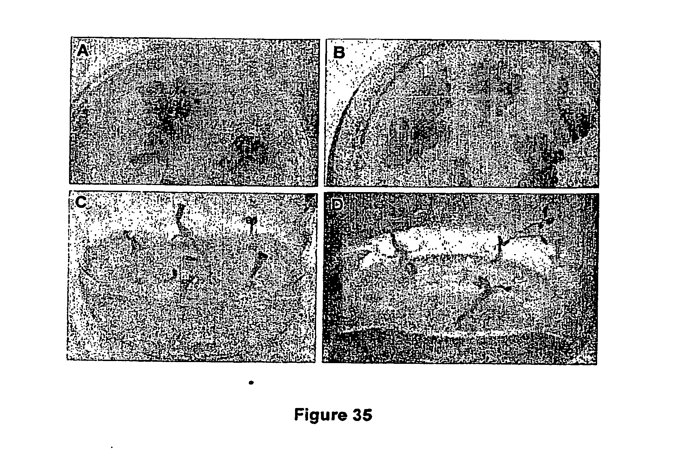

The binary vector *pBMVkATMYB32xs::ipt-nos* (Figure 18) containing chimeric *ipt* genes under control of Atmyb32xs variant promoter sequence with deleted root-specific motifs (Figure 2) was used for *Agrobacterium-mediated* transformation of *Medicago sativa* petiole explants from highly-regenerable alfalfa (*M. sativa*) clones C2-3, C2-4 and 19-17 (Figure 35).

Following co-cultivation with *Agrobacterium tumefaciens* strain LBA 4404 harbouring the binary vector *pBMVkATMYB32xs::ipt-nos,* the alfalfa explants were washed with medium containing cefotaxime and used for induction of embryogenic callus under selective medium containing 25 mg/l kanamycin. Transgenic embryogenic alfalfa calli were recovered and allowed to regenerate transgenic alfalfa shoots, which were transferred on rooting medium leading to the recovery of transgenic alfalfa plants expressing chimeric *ipt* genes under control of Atmyb32xs variant promoter (Figure 35).

It will be understood that the invention disclosed and defined in this specification extends to all alternative combinations of two or more of the individual features mentioned or evident from the text or drawings. All of these different combinations constitute various alternative aspects of the invention.

### Example 12

### Production of transgenic canola plants

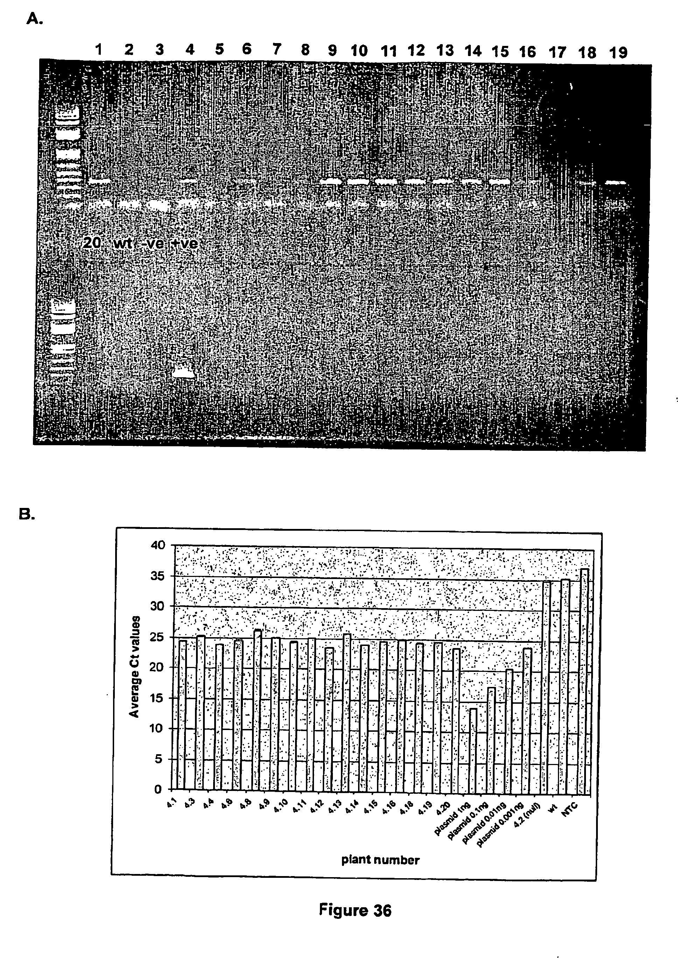

Transgenic canola plants (*Brassica napus*) were produced by *Agrobacterium-*mediated transformation using binary vectors (Figures 20 and 22) carrying the chimeric *atmyb32::ipt* gene. The genetic modification has been characterised for the presence of the candidate gene (*IPT*) or the selectable marker (*hph*) using PCR at the T₁ generation (Figures 36).

Figure 36 illustrates PCR analysis of transgenic canola plants. Genomic DNA was isolated from different transgenic canola plants of T₁ LXR04 lines and subjected to PCR using primers specific for the selectable marker (*hph* gene) or the candidate gene of interest (*IPT*). In Figure 36A *hph* specific primers were used to amplify a product from genomic DNA and were visualised on an agarose gel. Figure 36B demonstrates the use of *IPT* specific primers to amplify genomic DNA using a fluorescent PCR method. The primers are specific for the target sequence which results in detectable fluorescence that is inversely proportional to the amount of accumulated PCR product.

### Example 13

### IPT gene expression in transgenic canola plants

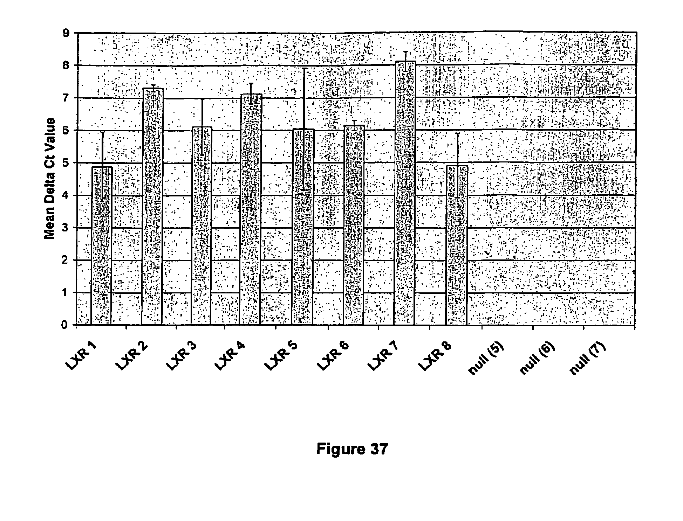

The expression of the *atmyb32::ipt* transgene in transgenic canola was assessed using a fluorescent RT-PCR method specific for the target sequence (Figure 37). The *IPT* mRNA was detected in tissues and relative expression levels were compared among lines and with null controls. Null controls are progeny lines that have undergone the transformation process but do not contain target sequences after crossing.

### Example 14

### Delayed detached leaf senescence in transgenic canola plants

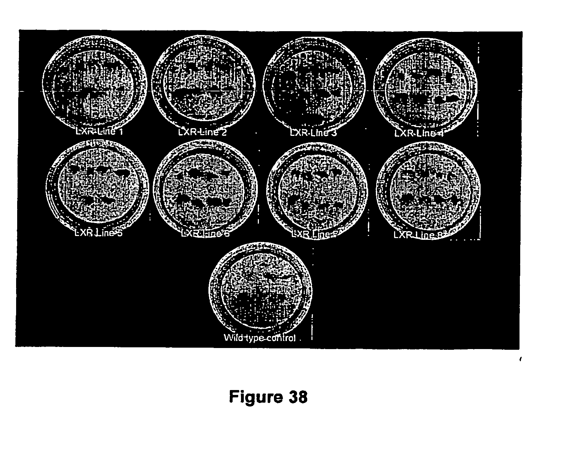

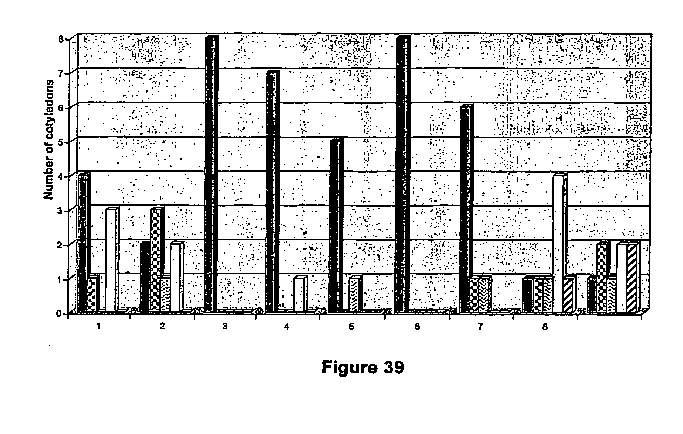

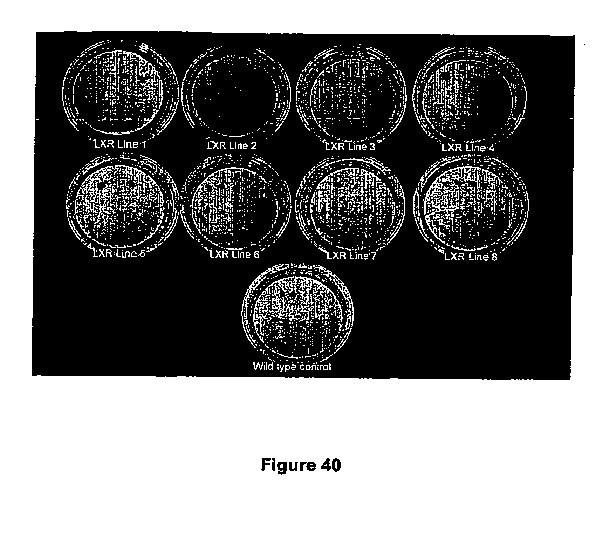

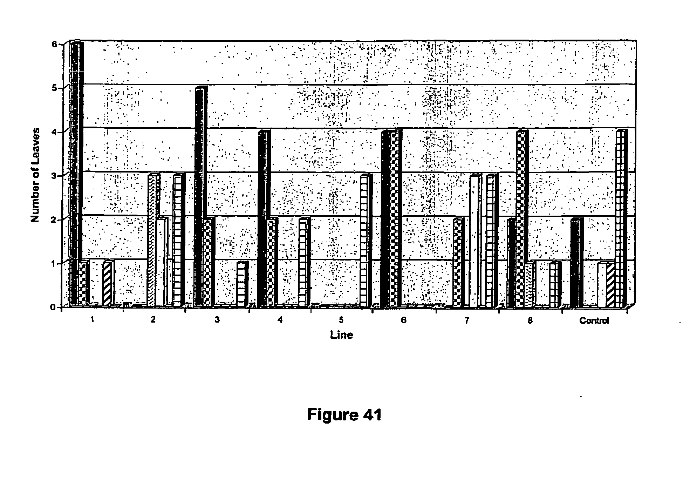

Experiments were performed to assess detached leaf senescence of *atmyb32::ipf* transgenic plants. Figures 38 to 41 indicate detached senescence assay data associated with expression of the candidate gene in canola. Assays for detached cotyledons and leaves were conducted to induce aging and asses the senescence phenotype of transformed canola as compared to wild-type controls. At day 7 and 14 of the detached senescence assays the progress of senescence was qualitatively scored for each tissue sample as either 0 - no possible signs of senescence; 1 - first visible signs of senescence with a paling of the green colour: 2 - further progression of senescence with yellowing in colour becoming noticeable; 3 - the tissue was mostly yellow in colour, however a pale green colour remained evident; 4- progression to completely yellow in colour; 5 - yellow in colour with some bleaching and patches of necrosis (Figures 39 and 41).

### Example 15

### Production of transgenic wheat plants

Genetic transformation of wheat was based on biolistic transformation of zygotic embryos from *Triticum aestivum* L Bobwhite 26 wheat line as outlined in Figures 27 and 28.

The chimeric *atmyb32::ipt* gene was inserted into the wheat genome by particle bombardment using whole plasmids so vector backbone sequences may also be incorporated into the genome (Figure 24).

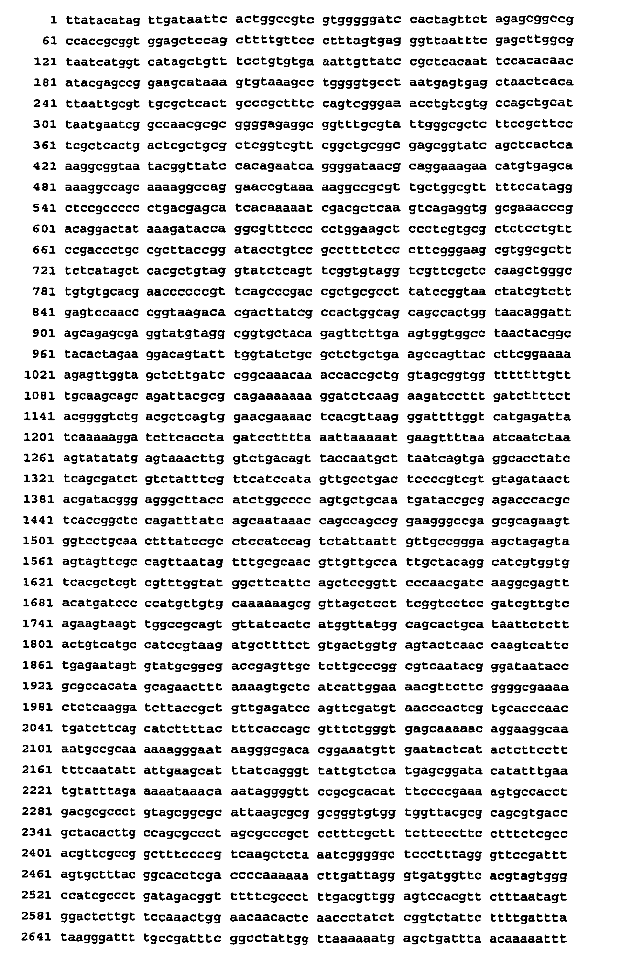

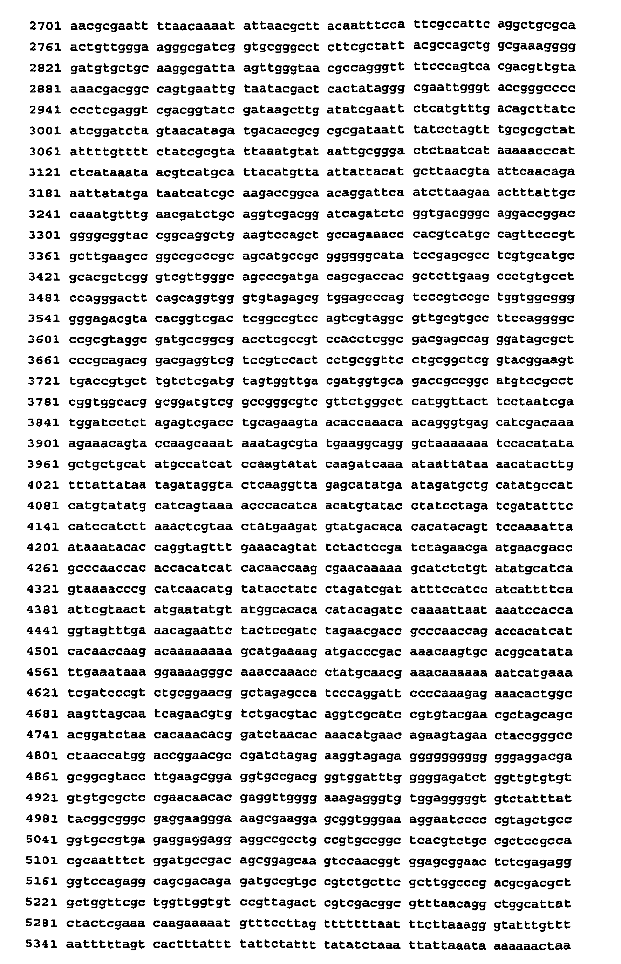

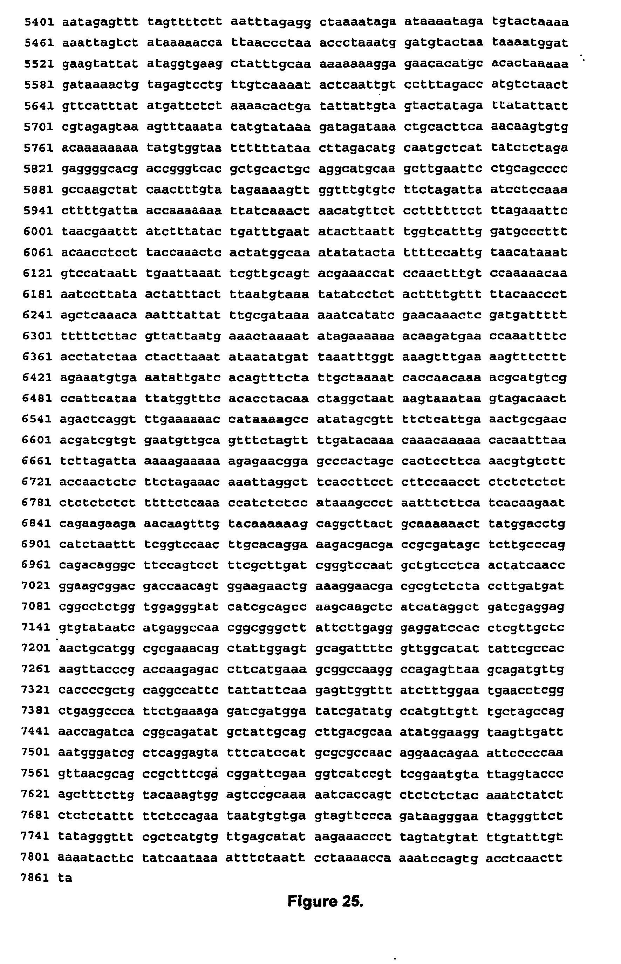

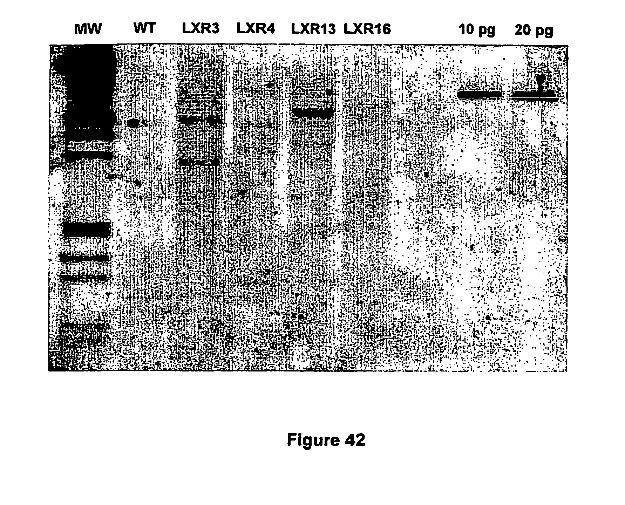

The transformation vector has been fully sequenced (Figure 25). The genetic modification has been characterised for the presence of the candidate gene by Southern analysis at the T₁ generation (Figure 42).

Figure 42 illustrates Southern hybridisation analysis of transgenic wheat plants. Genomic DNA was isolated from different T₁ lines of transgenic wheat plants and digested with a restriction enzyme to determine candidate gene copy number. The control is non-transformed wild-type *Triticum aestivum* 'Bobwhite 26'. Digests were electrophoresed, transferred to nylon membrane and probed with a full-length DIG labelled *IPT* gene, as a probe. A range of copy numbers was observed.

### Example 16

### IPT gene expression in transgenic wheat plants

RNA was extracted from young leaf tissue of glasshouse grown transgenic T₁ wheat plants containing the *IPT* gene driven by the AtMYB32 promoter and first strand cDNA prepared.

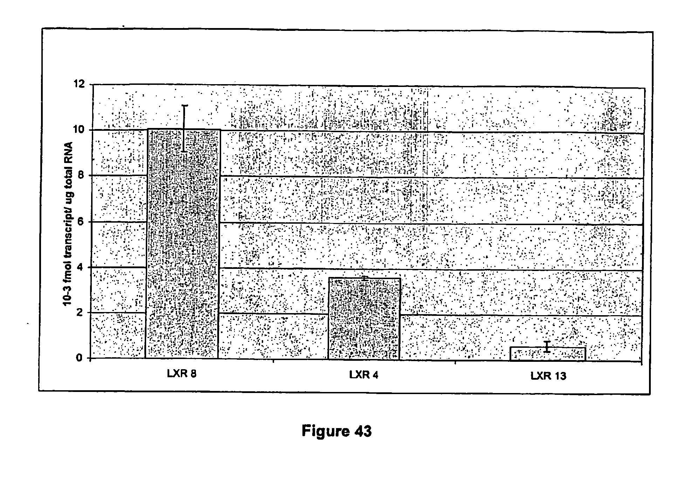

Quantitative expression of the transgene was determined using a probe based qRT-PCR method for the target sequence. Representative examples of high, medium and low expressing lines for each of the constructs are presented in Figure 43. A primer /probe set designed to the endogenous sucrose synthase gene was also used as a control. All amplification plots of the control gene began with in one cycle of each other indicating differences in the level of detection of the GMOs is due to variation in expression.

Both the PCR primers and probe are specific for the target sequence which results in detectable fluorescence that is proportional to the amount of accumulated PCR product. Serially diluted plasmid DNA containing the target sequence being detected was employed to create a standard curve for quantification.

### Example 17

### Plant morphology in transgenic wheat plants

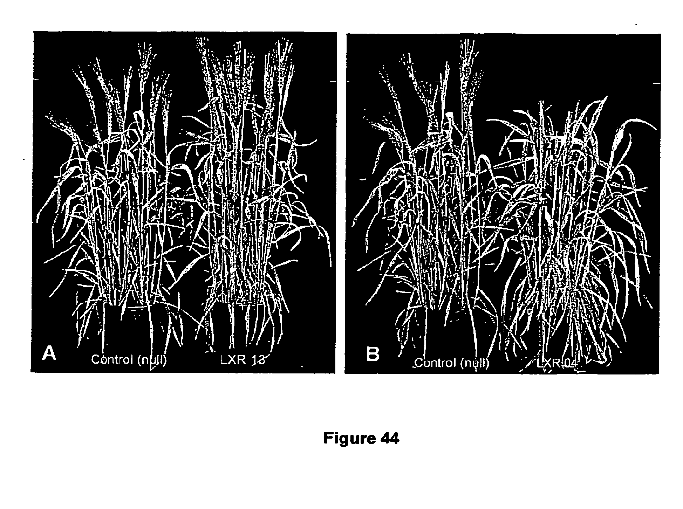

Differences in growth characteristics were observed in the glasshouse within and among transgenic wheat lines. The phenotypes predominantly observed among T₁ wheat plants included stunted plant height, tillering intensity, leaf number, as well as vegetative biomass (Figure 44).

### Example 18

### Delayed detached leaf senescence in transgenic wheat plants

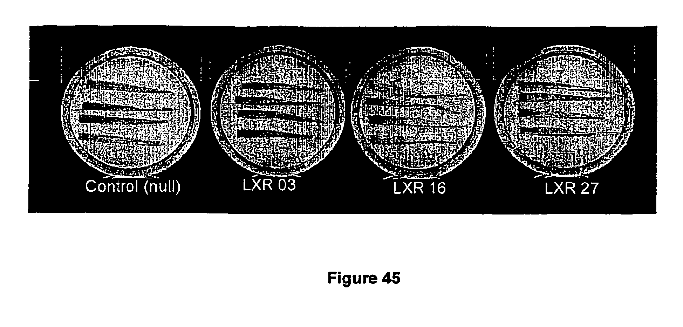

A detached leaf assay was used to asses induced aging and the senescence phenotype of transformed wheat leaves as compared to null controls (Figure 45). Null controls are progeny lines that have undergone the transformation process but do not contain target sequences after crossing.

It will also be understood that the term "comprises" (or its grammatical variants) as used in this specification is equivalent to the term "includes" and should not be taken as excluding the presence of other elements or features.

Documents cited in this specification are for reference purposes only and their inclusion is not an acknowledgement that they form part of the common general knowledge in the relevant art.

## Claims

1. A method of manipulating senescence in a plant, said method including introducing into said plant a genetic construct including a modified *myb* gene promoter including a nucleotide sequence selected from the group consisting of Sequence ID Nos: 2, 3 and 4, or a functionally active fragment or variant thereof having at least 95% identity to the part of SEQ ID No 2, 3 or 4 to which the fragment or variant corresponds and having a size of at least 300 nucleotides, operatively linked to a gene encoding an enzyme involved in biosynthesis of a cytokinin, or a functionally active fragment or variant thereof, wherein one or more root specific motifs and/or pollen specific motifs are deleted or inactivated in said modified *myb* gene promoter.

2. A method according to claim 1 wherein one or more root specific motifs are deleted or inactivated and wherein each of said root specific motifs includes a consensus sequence ATATT or AATAT; or wherein one or more pollen specific motifs are deleted or inactivated and wherein each of said pollen specific motifs includes a consensus sequence TTCT or AGAA.

3. A method according to any one of claims 1 or 2 wherein said gene encoding an enzyme involved in biosynthesis of a cytokinin is an isopentenyl transferase (*ipt*) or *sbo* gene, or a functionally active fragment or variant thereof.

4. A method according to any one of claims 1 or 2 wherein said gene encoding an enzyme involved in biosynthesis of a cytokinin includes a nucleotide sequence selected from the group consisting of Sequence ID Nos: 5, 7 and 9, sequences encoding the polypeptides having a sequence of Sequence ID Nos: 6, 8 and 10, and functionally active fragments and variants thereof.

5. A method according to any one of claims 1-4 wherein said genetic construct is introduced into said plant by *Agrobacterium-*mediated or biolistic transformation of plant cells and wherein plant cells incorporating the genetic construct are selected and then cultured to regenerate transformed plants.

6. A method according to claim 1, having between one and ten root specific motifs deleted or inactivated, said root specific motifs having a consensus sequence ATATT or AATAT, or having between one and thirty pollen specific motifs deleted or inactivated, said pollen specific motifs having a consensus sequence TTCT or AGAA; said modified *myb* gene promoter being operatively linked to an *ipt* or *sho* gene.

7. A vector capable of manipulating senescence in a plant, said vector including a modified *myb* gene promoter including a nucleotide sequence selected from the group consisting of Sequence ID Nos: 2, 3 and 4, or a functionally active fragment or variant thereof having at least 95% identity to the part of SEQ ID No 2, 3 or 4 to which the fragment or variant corresponds and having a size of at least 300 nucleotides, operatively linked to a gene encoding an enzyme involved in the biosynthesis of a cytokinin, or a functionally active fragment or variant thereof, wherein one or more root specific motifs and/or pollen specific motifs are deleted or inactivated in said modified *myb* gene promoter.

8. A vector according to claim 7, further including a terminator; said promoter, gene and terminator being operatively linked.

9. A vector according to claim 7 or 8 wherein said one or more root specific motifs are deleted or inactivated in said modified *myb* gene promoter and wherein each of said root specific motifs includes a consensus sequence ATATT or AATAT; or wherein one or more pollen specific motifs are deleted or inactivated in said modified *myb* promoter and wherein each of said pollen specific motifs includes a consensus sequence TTCT or AGAA.

10. A vector according to any one of claims 7-9 wherein said gene encoding an enzyme involved in biosynthesis of a cytokinin is an isopentenyl transferase (*ipt*) or *sho* gene or a functionally active fragment or variant thereof.

11. A vector according to any one of claims 7-9 wherein said gene encoding an enzyme involved in biosynthesis of a cytokinin includes a nucleotide sequence selected from the group consisting of Sequence ID Nos: 5, 7 and 9, and sequences encoding polypeptides having a sequence of Sequence 10 Nos: 6, 8 and 10.

12. A transgenic plant cell, plant, plant seed or other plant part, with modified senescence characteristics, said plant cell, plant, plant seed or other plant part including a genetic construct including a modified *myb* gene promoter including a nucleotide sequence selected from the group consisting of Sequence 10 Nos: 2, 3 and 4, or a functionally active fragment or variant thereof having at least 95% identity to the part of SEQ ID No 2, 3 or 4 to which the fragment or variant corresponds and having a size of at least 300 nucleotides, operatively linked to a gene encoding an enzyme involved in biosynthesis of a cytokinin, or a functionally active fragment or variant thereof, wherein one or more root specific motifs and/or pollen specific motifs are deleted or inactivated in said modified *myb* gene promoter.

13. A transgenic plant cell, plant, plant seed or other plant part, according to claim 12 produced by a method including introducing into said plant a genetic construct including a modified *myb* gene promoter including a nucleotide sequence selected from the group consisting of Sequence ID Nos: 2, 3 and 4, or a functionally active fragment or variant thereof having at least 95% identity to the part of SEQ ID No 2, 3 or 4 to which the fragment or variant corresponds and having a size of at least 300 nucleotides, operatively linked to a gene encoding an enzyme involved in biosynthesis of a cytokinin, or a functionally active fragment or variant thereof, wherein one or more root specific motifs and/or pollen specific motifs are deleted or inactivated in said modified *myb* gene promoter.

14. A transgenic plant, plant seed or other plant part derived from a plant cell or plant according to claim 12.

15. A method of enhancing biomass in a plant, said method including introducing into said plant a genetic construct including a modified *myb* gene promoter including a nucleotide sequence selected from the group consisting of Sequence ID Nos: 2, 3 and 4, or a functionally active fragment or variant thereof having at least 95% identity to the part of SEQ ID No 2, 3 or 4 to which the fragment or variant corresponds and having a size of at least 300 nucleotides, operatively linked to a gene encoding an enzyme involved in biosynthesis of a cytokinin, or a functionally active fragment or variant thereof, wherein one or more root specific motifs and/or pollen specific motifs are deleted or inactivated in said modified *myb* gene promoter.

16. A vector capable of enhancing biomass in a plant, said vector including a modified *myb* gene promoter including a nucleotide sequence selected from the group consisting of Sequence ID Nos: 2, 3 and 4, or a functionally active fragment or variant thereof having at least 95% identity to the part of SEQ ID No 2, 3 or 4 to which the fragment or variant corresponds and having a size of at least 300 nucleotides, operatively linked to a gene encoding an enzyme involved in the biosynthesis of a cytokinin, or a functionally active fragment or variant thereof, wherein one or more root specific motifs and/or pollen specific motifs are deleted or inactivated in said modified *myb* gene promoter.

## Patentansprüche

1. Ein Verfahren zum Manipulieren von Seneszenz in einer Pflanze, jenes Verfahren umfassend Einführen, in jene Pflanze, eines genetischen Konstruktes umfassend einen modifizierten *myb*-Gen-Promotor umfassend eine Nukleotidsequenz ausgewählt aus der Gruppe bestehend aus Sequenz-ID-Nos: 2, 3 und 4, oder ein funktionell aktives Fragment oder Variante davon mit mindestens 95% Identität zu dem Teil von SEQ ID No 2, 3 oder 4, dem das Fragment oder die Variante entspricht, und mit einer Größe von mindestens 300 Nukleotiden, operativ verknüpft mit einem Gen, das für ein Enzym kodiert, das an der Biosynthese eines Cytokinins beteiligt ist, oder einem funktionell aktiven Fragments oder Variante davon, wobei ein oder mehr wurzelspezifische Motive und/oder pollenspezifische Motive in jenem modifizierten *myb*-Gen-Promotor deletiert oder inaktiviert sind.

2. Ein Verfahren gemäß Anspruch 1, wobei ein oder mehr wurzelspezifische Motive deletiert oder inaktiviert sind und wobei jedes jener wurzelspezifischen Motive eine Konsensussequenz ATATT oder AATAT umfasst; oder wobei ein oder mehr pollenspezifische Motive deletiert oder inaktiviert sind und wobei jedes jener pollenspezifischen Motive eine Konsensussequenz TTCT oder AGAA umfasst.

3. Ein Verfahren gemäß irgendeinem der Ansprüche 1 oder 2, wobei jenes Gen, das für ein Enzym kodiert, das an der Biosynthese eines Cytokinins beteiligt ist, ein Isopentenyltransferase- (*ipt*-) oder *sho*-Gen oder ein funktionell aktives Fragment oder Variante davon ist.

4. Ein Verfahren gemäß irgendeinem der Ansprüche 1 oder 2, wobei jenes Gen, das für ein Enzym kodiert, das an der Biosynthese eines Cytokinins beteiligt ist, eine Nukleotidsequenz ausgewählt aus der Gruppe bestehend aus Sequenz-ID-Nos: 5, 7 und 9, Sequenzen, die Polypeptide mit einer Sequenz aus Sequenz-ID-Nos: 6, 8 und 10 kodieren, und funktionell aktiven Fragmenten und Varianten davon umfasst.

5. Ein Verfahren gemäß irgendeinem der Ansprüche 1-4, wobei jenes genetische Konstrukt in jene Pflanze mittels *Agrobacterium-*vermittelter oder biolistischer Transformierung von Pflanzenzellen eingeführt wird und wobei Pflanzenzellen, die das genetische Konstrukt inkorporieren, selektiert und dann kultiviert werden, um transformierte Pflanzen zu regenerieren.

6. Ein Verfahren gemäß Anspruch 1 mit zwischen einem und zehn deletierten oder inaktivierten wurzelspezifischen Motiven, wobei jene wurzelspezifischen Motive eine Konsensussequenz ATATT oder AATAT haben, oder mit zwischen einem und dreißig deletierten oder inaktivierten pollenspezifischen Motiven, wobei jene pollenspezifischen Motive eine Konsensussequenz TTCT oder AGAA haben; wobei jener modifizierte *myb-*Gen-Promotor operativ mit einem *ipt*- oder *sho*-Gen verknüpft ist.

7. Ein Vektor, der in der Lage ist, Seneszenz in einer Pflanze zu manipulieren, jener Vektor umfassend einen modifizierten *myb*-Gen-Promotor umfassend eine Nukleotidsequenz ausgewählt aus der Gruppe bestehend aus Sequenz-ID-Nos: 2, 3 und 4, oder ein funktionell aktives Fragment oder Variante davon mit mindestens 95% Identität zu dem Teil von SEQ ID No 2, 3 oder 4, dem das Fragment oder die Variante entspricht, und mit einer Größe von mindestens 300 Nukleotiden, operativ verknüpft mit einem Gen, das für ein Enzym kodiert, das an der Biosynthese eines Cytokinins beteiligt ist, oder einem funktionell aktiven Fragments oder Variante davon, wobei ein oder mehr wurzelspezifische Motive und/oder pollenspezifische Motive in jenem modifizierten *myb*-Gen-Promotor deletiert oder inaktiviert sind.

8. Ein Vektor gemäß Anspruch 7 weiter umfassend einen Terminator; wobei jener Promotor, jenes Gen und jener Terminator operativ verknüpft sind.

9. Ein Vektor gemäß Anspruch 7 oder 8, wobei jene ein oder mehr wurzelspezifischen Motive in jenem *myb*-Gen Promotor deletiert oder inaktiviert sind und wobei jedes jener wurzelspezifischen Motive eine Konsensussequenz ATATT oder AATAT umfasst; oder wobei ein oder mehr pollenspezifische Motive in jenem *myb*-Gen Promotor deletiert oder inaktiviert sind und wobei jedes jener pollenspezifischen Motive eine Konsensussequenz TTCT oder AGAA umfasst.

10. Ein Vektor gemäß irgendeinem der Ansprüche 7-9, wobei jenes Gen, das für ein Enzym kodiert, das an der Biosynthese eines Cytokinins beteiligt ist, ein Isopentenyltransferase-(*ipt*-) oder *sho*-Gen oder ein funktionell aktives Fragment oder Variante davon ist.

11. Ein Vektor gemäß irgendeinem der Ansprüche 7-9, wobei jenes Gen, das für ein Enzym kodiert, das an der Biosynthese eines Cytokinins beteiligt ist, eine Nukleotidsequenz ausgewählt aus der Gruppe bestehend aus Sequenz-ID-Nos: 5, 7 und 9, und Sequenzen, die Polypeptide mit einer Sequenz aus Sequenz-ID-Nos: 6, 8 und 10 kodieren, umfasst.

12. Eine transgene Pflanzenzelle, Pflanze, Pflanzensamen oder anderes Pflanzenteil mit modifizierten Seneszenzcharakteristika, jene Pflanzenzelle, jene Pflanze, jener Pflanzensamen oder jenes andere Pflanzenteil umfassend ein genetisches Konstrukt umfassend einen modifizierten *myb*-Gen-Promotor umfassend eine Nukleotidsequenz ausgewählt aus der Gruppe bestehend aus Sequenz-ID-Nos: 2, 3 und 4, oder ein funktionell aktives Fragment oder Variante davon mit mindestens 95% Identität zu dem Teil von SEQ ID No 2, 3 oder 4, dem das Fragment oder die Variante entspricht, und mit einer Größe von mindestens 300 Nukleotiden, operativ verknüpft mit einem Gen, das für ein Enzym kodiert, das an der Biosynthese eines Cytokinins beteiligt ist, oder einem funktionell aktiven Fragments oder Variante davon, wobei ein oder mehr wurzelspezifische Motive und/oder pollenspezifische Motive in jenem modifizierten myb-Gen-Promotor deletiert oder inaktiviert sind.

13. Eine transgene Pflanzenzelle, Pflanze, Pflanzensamen oder anderes Pflanzenteil gemäß Anspruch 12, hergestellt mittels eines Verfahrens umfassend Einführen, in jene Pflanze, eines genetischen Konstruktes umfassend einen modifizierten *myb*-Gen-Promotor umfassend eine Nukleotidsequenz ausgewählt aus der Gruppe bestehend aus Sequenz-ID-Nos: 2, 3 und 4, oder ein funktionell aktives Fragment oder Variante davon mit mindestens 95% Identität zu dem Teil von SEQ ID No 2, 3 oder 4, dem das Fragment oder die Variante entspricht, und mit einer Größe von mindestens 300 Nukleotiden, operativ verknüpft mit einem Gen, das für ein Enzym kodiert, das an der Biosynthese eines Cytokinins beteiligt ist, oder einem funktionell aktiven Fragments oder Variante davon, wobei ein oder mehr wurzelspezifische Motive und/oder pollenspezifische Motive in jenem modifizierten *myb*-Gen-Promotor deletiert oder inaktiviert sind.

14. Eine transgene Pflanze, Pflanzensamen oder anderes Pflanzenteil abgeleitet von einer Pflanzenzelle oder Pflanze gemäß Anspruch 12.

15. Ein Verfahren zum Steigern von Biomasse in einer Pflanze, jenes Verfahren umfassend Einführen, in jene Pflanze, eines genetischen Konstruktes umfassend einen modifizierten *myb*-Gen-Promotor umfassend eine Nukleotidsequenz ausgewählt aus der Gruppe bestehend aus Sequenz-ID-Nos: 2, 3 und 4, oder ein funktionell aktives Fragment oder Variante davon mit mindestens 95% Identität zu dem Teil von SEQ ID No 2, 3 oder 4, dem das Fragment oder die Variante entspricht, und mit einer Größe von mindestens 300 Nukleotiden, operativ verknüpft mit einem Gen, das für ein Enzym kodiert, das an der Biosynthese eines Cytokinins beteiligt ist, oder einem funktionell aktiven Fragments oder Variante davon, wobei ein oder mehr wurzelspezifische Motive und/oder pollenspezifische Motive in jenem modifizierten *myb*-Gen-Promotor deletiert oder inaktiviert sind.

16. Ein Vektor, der in der Lage ist, die Biomasse in einer Pflanze zu steigern, jener Vektor umfassend einen modifizierten *myb*-Gen-Promotor umfassend eine Nukleotidsequenz ausgewählt aus der Gruppe bestehend aus Sequenz-ID-Nos: 2, 3 und 4, oder ein funktionell aktives Fragment oder Variante davon mit mindestens 95% Identität zu dem Teil von SEQ ID No 2, 3 oder 4, dem das Fragment oder die Variante entspricht, und mit einer Größe von mindestens 300 Nukleotiden, operativ verknüpft mit einem Gen, das für ein Enzym kodiert, das an der Biosynthese eines Cytokinins beteiligt ist, oder einem funktionell aktiven Fragments oder Variante davon, wobei ein oder mehr wurzelspezifische Motive und/oder pollenspezifische Motive in jenem modifizierten *myb*-Gen-Promotor deletiert oder inaktiviert sind.

## Revendications

1. Procédé de manipulation de la sénescence dans une plante, ledit procédé comprenant l'introduction dans ladite plante d'un produit d'assemblage génétique comprenant un promoteur du gène *myb* modifié comprenant une séquence nucléotidique choisie dans le groupe constitué de Séquence ID No : 2, 3 et 4, ou d'un fragment ou d'un variant fonctionnellement actif de celles-ci ayant au moins 95 % d'identité avec la partie de SEQ ID No : 2, 3 ou 4 à laquelle le fragment ou le variant correspond et ayant une taille d'au moins 300 nucléotides, lié fonctionnellement à un gène codant pour une enzyme impliquée dans la biosynthèse d'une cytokinine, ou à un fragment ou un variant fonctionnellement actif de celui-ci, dans lequel un ou plusieurs motifs spécifiques de la racine et/ou motifs spécifiques du pollen sont délétés ou inactivés dans ledit promoteur du gène *myb* modifié.

2. Procédé selon la revendication 1, dans lequel un ou plusieurs motifs spécifiques de la racine sont délétés ou inactivés et dans lequel chacun desdits motifs spécifiques de la racine comprend une séquence consensus ATATT ou AATAT ; ou dans lequel un ou plusieurs motifs spécifiques du pollen sont délétés ou inactivés et dans lequel chacun desdits motifs spécifiques du pollen comprend une séquence consensus TTCT ou AGAA.

3. Procédé selon l'une quelconque des revendications 1 ou 2, dans lequel ledit gène codant pour une enzyme impliquée dans la biosynthèse d'une cytokinine est un gène de l'isopentényl-transférase (*ipt*) ou *sho,* ou un fragment ou un variant fonctionnellement actif de celui-ci.

4. Procédé selon l'une quelconque des revendications 1 ou 2, dans lequel ledit gène codant pour une enzyme impliquée dans la biosynthèse d'une cytokinine comprend une séquence nucléotidique choisie dans le groupe constitué de Séquence ID No : 5, 7 et 9, des séquences codant pour les polypeptides ayant une séquence de Séquence ID No : 6, 8 et 10, et des fragments et des variants fonctionnellement actifs de ceux-ci.

5. Procédé selon l'une quelconque des revendications 1 à 4, dans lequel ledit produit d'assemblage génétique est introduit dans ladite plante par une transformation médiée par *Agrobacterium* ou biolistique de cellules végétales et dans lequel les cellules végétales incorporant le produit d'assemblage génétique sont choisies et ensuite cultivées pour régénérer des plantes transformées.

6. Procédé selon la revendication 1, ayant entre un et dix motifs spécifiques de la racine délétés ou inactivés, lesdits motifs spécifiques de la racine ayant une séquence consensus ATATT ou AATAT, ou ayant entre un et trente motifs spécifiques du pollen délétés ou inactivés, lesdits motifs spécifiques du pollen ayant une séquence consensus TTCT ou AGAA ; ledit promoteur du gène *myb* modifié étant lié fonctionnellement à un gène *ipt* ou *sho.*

7. Vecteur capable de manipuler la sénescence dans une plante, ledit vecteur comprenant un promoteur du gène *myb* modifié comprenant une séquence nucléotidique choisie dans le groupe constitué de Séquence ID No : 2, 3 et 4, ou d'un fragment ou d'un variant fonctionnellement actif de celles-ci ayant au moins 95 % d'identité avec la partie de SEQ ID no : 2, 3 ou 4 à laquelle le fragment ou le variant correspond et ayant une taille d'au moins 300 nucléotides, lié fonctionnellement à un gène codant pour une enzyme impliquée dans la biosynthèse d'une cytokinine, ou à un fragment ou un variant fonctionnellement actif de celui-ci, dans lequel un ou plusieurs motifs spécifiques de la racine et/ou motifs spécifiques du pollen sont délétés ou inactivés dans ledit promoteur du gène *myb* modifié.

8. Vecteur selon la revendication 7, comprenant en outre un terminateur ; lesdits promoteur, gène et terminateur étant liés fonctionnellement.

9. Vecteur selon la revendication 7 ou 8, dans lequel lesdits un ou plusieurs motifs spécifiques de la racine sont délétés ou inactivés dans ledit promoteur du gène *myb* modifié et dans lequel chacun desdits motifs spécifiques de la racine comprend une séquence consensus ATATT ou AATAT ; ou dans lequel un ou plusieurs motifs spécifiques du pollen sont délétés ou inactivés dans ledit promoteur *myb* modifié et dans lequel chacun desdits motifs spécifiques du pollen comprend une séquence consensus TTCT ou AGAA.

10. Vecteur selon l'une quelconque des revendications 7 à 9, dans lequel ledit gène codant pour une enzyme impliquée dans la biosynthèse d'une cytokinine est un gène de l'isopentényl-transférase (*ipt*) ou *sho* ou un fragment ou un variant fonctionnellement actif de celui-ci.

11. Vecteur selon l'une quelconque des revendications 7 à 9, dans lequel ledit gène codant pour une enzyme impliquée dans la biosynthèse d'une cytokinine comprend une séquence nucléotidique choisie dans le groupe constitué de Séquence ID No : 5, 7 et 9, et des séquences codant pour des polypeptides ayant une séquence de Séquence ID No : 6, 8 et 10.

12. Cellule végétale, plante, graine végétale ou autre partie de plante transgénique, avec des caractéristiques de sénescence modifiées, ladite cellule végétale, plante, graine végétale ou autre partie de plante comprenant un produit d'assemblage génétique comprenant un promoteur du gène *myb* modifié comprenant une séquence nucléotidique choisie dans le groupe constitué de Séquence ID No : 2, 3 et 4, ou d'un fragment ou d'un variant fonctionnellement actif de celles-ci ayant au moins 95 % d'identité avec la partie de SEQ ID No : 2, 3 ou 4 à laquelle le fragment ou le variant correspond et ayant une taille d'au moins 300 nucléotides, lié fonctionnellement à un gène codant pour une enzyme impliquée dans la biosynthèse d'une cytokinine ou à un fragment ou un variant fonctionnellement actif de celui-ci, dans laquelle un ou plusieurs motifs spécifiques de la racine et/ou motifs spécifiques du pollen sont délétés ou inactivés dans ledit promoteur du gène *myb* modifié.

13. Cellule végétale, plante, graine végétale ou autre partie de plante transgénique, selon la revendication 12, produite par un procédé comprenant l'introduction dans ladite plante d'un produit d'assemblage génétique comprenant un promoteur du gène *myb* modifié comprenant une séquence nucléotidique choisie dans le groupe constitué de Séquence ID No : 2, 3 et 4, ou d'un fragment ou d'un variant fonctionnellement actif de celles-ci ayant au moins 95 % d'identité avec la partie de SEQ ID No : 2, 3 ou 4 à laquelle le fragment ou le variant correspond et ayant une taille d'au moins 300 nucléotides, lié fonctionnellement à un gène codant pour une enzyme impliquée dans la biosynthèse d'une cytokinine, ou à un fragment ou un variant fonctionnellement actif de celui-ci, dans laquelle un ou plusieurs motifs spécifiques de la racine et/ou motifs spécifiques du pollen sont délétés ou inactivés dans ledit promoteur du gène *myb* modifié.

14. Plante, graine végétale ou autre partie de plante transgénique dérivée d'une cellule végétale ou d'une plante selon la revendication 12.

15. Procédé d'amplification de la biomasse dans une plante, ledit procédé comprenant l'introduction dans ladite plante d'un produit d'assemblage génétique comprenant un promoteur du gène *myb* modifié comprenant une séquence nucléotidique choisie dans le groupe constitué de Séquence ID No : 2, 3 et 4, ou d'un fragment ou d'un variant fonctionnement actif de celles-ci ayant au moins 95 % d'identité avec la partie de SEQ ID No : 2, 3 ou 4 à laquelle le fragment ou le variant correspond et ayant une taille d'au moins 300 nucléotides, lié fonctionnellement à un gène codant pour une enzyme impliquée dans la biosynthèse d'une cytokinine, ou à un fragment ou un variant fonctionnellement actif de celui-ci, dans lequel un ou plusieurs motifs spécifiques de la racine et/ou motifs spécifiques du pollen sont délétés ou inactivés dans ledit promoteur du gène *myb* modifié.

16. Vecteur capable d'amplifier la biomasse dans une plante, ledit vecteur comprenant un promoteur du gène *myb* modifié comprenant une séquence nucléotidique choisie dans le groupe constitué de Séquence ID No : 2, 3 et 4, ou d'un fragment ou d'un variant fonctionnellement actif de celles-ci ayant au moins 95 % d'identité avec la partie de SEQ ID No : 2, 3 ou 4 à laquelle le fragment ou le variant correspond et ayant une taille d'au moins 300 nucléotides, lié fonctionnellement à un gène codant pour une enzyme impliquée dans la biosynthèse d'une cytokinine, ou à un fragment ou un variant fonctionnellement actif de celui-ci, dans lequel un ou plusieurs motifs spécifiques de la racine et/ou motifs spécifiques du pollen sont délétés ou inactivés dans ledit promoteur du gène *myb* modifié.


### SEQUENCE LISTING

<110> Agriculture Victoria Services Pty Ltd La Trobe University
<120> Manipulation of plant senescence using modified promotors
<130> 118-006
<140> 08 733 383.7
   <141> 2008-04-21
<150> US 10/363723
   <151> 2001-08-30
<150> PCT/AU2008/000556
   <151> 2008-04-21
<150> US 11/798526
   <151> 2007-04-24
<150> PCT/AU01/01092
   <151> 2001-08-30
<160> 15
<170> Patent In version 3.5
<210> 1
   <211> 941
   <212> DNA
   <213> Arabidopsis thaliana
<400> 1
   gtttgtgtct tctagattaa tcctccaaac ttttgattaa ccaaaaaaat tatcaaacta 60
   acatgttctc ctttttcttt tagaaattct aacgaattta tctttatact gatttgaata 120
   tacttaattt ggtcatttgg atgcccttta caacctcctt accaaactca ctatggcaaa 180
   tatatactat tttccattgt aacataaatg tccataattt gaattaaatt cgttgcagta 240
   cgaaaccatc caactttgtc caaaaacaaa atccttataa ctatttactt taatgtaaat 300
   atatcctcta cttttgtttt tacaacccta gctcaaacaa atttattatt tgcgataaaa 360
   aatcatatcg aacaaactcg atgatttttt ttttcttacg ttattaatga aactaaaata 420
   tagaaaaaaa caagatgaac caaattttca cctatctaac tacttaaata taatatgatt 480
   aaatttggta aagtttgaaa agtttcttta gaaatgtgaa atattgatca cagtttctat 540
   tgctaaaatc accaacaaaa cgcatgtcgc cattcataat tatggtttca cacctacaac 600
   taggctaata agtaaataag tagacaacta gactcaggtt tgaaaaaacc ataaaagcca 660
   tatagcgttt tctcattgaa actgcgaaca cgatcgtgtg aatgttgcag tttctagttt 720
   tgatacaaac aaacaaaaac acaatttaat cttagattaa aaagaaaaaa gagaacggag 780
   cccactagcc actccttcaa acgtgtctta ccaactctct tctagaaaca aattaggctt 840
   caccttcctc ttccaacctc tctctctctc tctctctctt tttctcaaac catctctcca 900
   taaagcccta atttcttcat cacaagaatc agaagaagaa a 941
<210> 2
   <211> 588
   <212> DNA
   <213> Arabidopsis thaliana
<400> 2
   gtttgtgtct tctagattaa tcctccaaac ttttgattaa ccaaaaaaat tatcaaacta 60
   acatgttctc cttttttctt tagaaattct aacgaattta tctttatact gatttgaata 120
   tacttaattt ggtcatttgg atgcccttta caacctcctt accaaactca ttgatcacag 180
   tttctattgc taaaatcacc aacaaaacgc atgtcgccat tcataattat ggtttcacac 240
   ctacaactag gctaataagt aaataagtag acaactagac tcaggtttga aaaaaccata 300
   aaagccatat agcgttttct cattgaaact gcgaacacga tcgtgtgaat gttgcagttt 360
   ctagttttga tacaaacaaa caaaaacaca atttaatctt agattaaaaa gaaaaaagag 420
   aacggagccc actagccact ccttcaaacg tgtcttacca actctcttct agaaacaaat 480
   taggcttcac cttcctcttc caacctctct ctctctctct ctctcttttt ctcaaaccat 540
   ctctccataa agccctaatt tcttcatcac aagaatcaga agaagaaa 588
<210> 3
   <211> 468
   <212> DNA
   <213> Arabidopsis thaliana
<400> 3
   tacttaattt ggtcatttgg atgcccttta caacctcctt accaaactca ttgatcacag 60
   tttctattgc taaaatcacc aacaaaacgc atgtcgccat tcataattat ggtttcacac 120
   ctacaactag gctaataagt aaataagtag acaactagac tcaggtttga aaaaaccata 180
   aaagccatat agcgttttct cattgaaact gcgaacacga tcgtgtgaat gttgcagttt 240
   ctagttttga tacaaacaaa caaaaacaca atttaatctt agattaaaaa gaaaaaagag 300
   aacggagccc actagccact ccttcaaacg tgtcttacca actctcttct agaaacaaat 360
   taggcttcac cttcctcttc caacctctct ctctctctct ctctcttttt ctcaaaccat 420
   ctctccataa agccctaatt tcttcatcac aagaatcaga agaagaaa 468
<210> 4
   <211> 419
   <212> DNA
   <213> Arabidopsis thaliana
<400> 4
   attgatcaca gtttctattg ctaaaatcac caacaaaacg catgtcgcca ttcataatta     60
   tggtttcaca cctacaacta ggctaataag taaataagta gacaactaga ctcaggtttg    120
   aaaaaaccat aaaagccata tagcgttttc tcattgaaac tgcgaacacg atcgtgtgaa    180
   tgttgcagtt tctagttttg atacaaacaa acaaaaacac aatttaatct tagattaaaa    240
   agaaaaaaga gaacggagcc cactagccac tccttcaaac gtgtcttacc aactctcttc    300
   tagaaacaaa ttaggcttca ccttcctctt ccaacctctc tctctctctc tctctctttt    360
   tctcaaacca tctctccata aagccctaat ttcttcatca caagaatcag aagaagaaa     419
<210> 5
   <211> 723
   <212> DNA
   <213> Agrobacterium tumefaciens
<400> 5
   atggacctgc atctaatttt cggtccaact tgcacaggaa agacgacgac cgcgatagct     60
   cttgcccagc agacagggct tccagtcctt tcgcttgatc gggtccaatg ctgtcctcaa    120
   ctatcaaccg gaagcggacg accaacagtg gaagaactga aaggaacgac gcgtctctac    180
   cttgatgatc ggcctctggt ggagggtatc atcgcagcca agcaagctca tcataggctg    240
   atcgaggagg tgtataatca tgaggccaac ggcgggctta ttcttgaggg aggatccacc    300
   tcgttgctca actgcatggc gcgaaacagc tattggagtg cagattttcg ttggcatatt    360
   attcgccaca agttacccga ccaagagacc ttcatgaaag cggccaaggc cagagttaag    420
   cagatgttgc accccgctgc aggccattct attattcaag agttggttta tctttggaat    480
   gaacctcggc tgaggcccat tctgaaagag atcgatggat atcgatatgc catgttgttt    540
   gctagccaga accagatcac ggcagatatg ctattgcagc ttgacgcaaa tatggaaggt    600
   aagttgatta atgggatcgc tcaggagtat ttcatccatg cgcgccaaca ggaacagaaa    660
   ttcccccaag ttaacgcagc cgctttcgac ggattcgaag gtcatccgtt cggaatgtat    720
   tag                                                                  723
<210> 6
   <211> 240
   <212> PRT
   <213> Agrobacterium tumefaciens
<400> 6
   Met Asp Leu His Leu Ile Phe Gly Pro Thr Cys Thr Gly Lys Thr Thr
   1               5                   10                  15
   Thr Ala Ile Ala Leu Ala Gln Gln Thr Gly Leu Pro Val Leu Ser Leu
               20                  25                  30
   Asp Arg Val Gln Cys Cys Pro Gln Leu Ser Thr Gly Ser Gly Arg Pro
           35                  40                  45
   Thr Val Glu Glu Leu Lys Gly Thr Thr Arg Leu Tyr Leu Asp Asp Arg
       50                  55                  60
   Pro Leu Val Glu Gly Ile Ile Ala Ala Lys Gln Ala His His Arg Leu
   65                  70                  75                  80
   Ile Glu Glu Val Tyr Asn His Glu Ala Asn Gly Gly Leu Ile Leu Glu
                   85                  90                  95
   Gly Gly Ser Thr Ser Leu Leu Asn Cys Met Ala Arg Asn Ser Tyr Trp
               100                 105                 110
   Ser Ala Asp Phe Arg Trp His Ile Ile Arg His Lys Leu Pro Asp Gln
           115                 120                 125
   Glu Thr Phe Met Lys Ala Ala Lys Ala Arg Val Lys Gln Met Leu His
       130                 135                 140
   Pro Ala Ala Gly His Ser Ile Ile Gln Glu Leu Val Tyr Leu Trp Asn
   145                 150                 155                 160
   Glu Pro Arg Leu Arg Pro Ile Leu Lys Glu Ile Asp Gly Tyr Arg Tyr
                   165                 170                 175
   Ala Met Leu Phe Ala Ser Gln Asn Gln Ile Thr Ala Asp Met Leu Leu
               180                 185                 190
   Gln Leu Asp Ala Asn Met Glu Gly Lys Leu Ile Asn Gly Ile Ala Gln
           195                 200                 205
   Glu Tyr Phe Ile His Ala Arg Gln Gln Glu Gln Lys Phe Pro Gln Val
       210                 215                 220
   Asn Ala Ala Ala Phe Asp Gly Phe Glu Gly His Pro Phe Gly Met Tyr
   225                 230                 235                 240
<210> 7
   <211> 975
   <212> DNA
   <213> Lotus japonicus
<400> 7
   atgtccatct caatgctaat gtgcagacta agacaaccct taataaacgt tccctgcagt     60
   ggcaaaaaac tgagcatgag gcagattcaa aaggagaagg tagtgttggt gatgggagct    120
   acagggacag gaaagtcaaa gctctccatt gacctcgcca cctgtttccc ctcagaaatc    180
   atcaactccg acaagattca aatctacgac ggcctcgaca tcgtcaccaa caaaatctcc    240
   aaggaagaac aacgtggaat cccccaccac ctcctcggaa ctcaaaaccc taacacagac    300
   ttcaccgccg gcgatttcag tgactgttcc accgccgcca ttgacgcaat cacaagccgc    360
   gaccaccttc cgatcatcgc cggaggttcg aactcctacc tggaggcgtt aatcgacgac    420
   gacgactaca aattccgatc gaggtacgac ttctgctgcc tctgggtcga cgtggcaatg    480
   ccggtgctgg actcatacgt ggcggcgcgt gtggatcaga tgctccggag cggaatggtg    540
   gaggagctga gaccgttttt caacgcgaac ggcgactact cgagaggaat cagaagagcg    600
   attggggttc ctgaattcga cgagtatttc cggcgggaag ggttcgccga tgaggaaacg    660
   aggaaattgt tactggagcg agcggtgagg gagatgaagg tgaacacgtg caagctcgcg    720
   aggaggcaat tggggaagat tcagaggctg aggaatgtga agaggtggga gattcaccgt    780
   gttgatgcga cgccggtgtt ttggaagcgt ggggaggagg ctgatgaggc gtggcggaag    840
   gtggtggcag agcctagtgc tatgatcgta gcgcagtttc tgtataaggc aaagagtgat    900
   gtgaatgttg tttctggcgg tttcagagtg ccggcgggtt caacggagag tgttatggcg    960
   gcggcgacgt gttag                                                      975
<210> 8
   <211> 324
   <212> PRT
   <213> Lotus japonicus
<400> 8
   Met Ser Ile Ser Met Leu Met Cys Arg Leu Arg Gln Pro Leu Ile Asn
   1               5                   10                  15
   Val Pro Cys Ser Gly Lys Lys Leu Ser Met Arg Gln Ile Gln Lys Glu
               20                  25                  30
   Lys Val Val Leu Val Met Gly Ala Thr Gly Thr Gly Lys Ser Lys Leu
           35                  40                  45
   Ser Ile Asp Leu Ala Thr Cys Phe Pro Ser Glu Ile Ile Asn Ser Asp
       50                  55                  60
   Lys Ile Gln Ile Tyr Asp Gly Leu Asp Ile Val Thr Asn Lys Ile Ser
   65                  70                  75                  80
   Lys Glu Glu Gln Arg Gly Ile Pro His His Leu Leu Gly Thr Gln Asn
                   85                  90                  95
   Pro Asn Thr Asp Phe Thr Ala Gly Asp Phe Ser Asp Cys Ser Thr Ala
               100                 105                 110
   Ala Ile Asp Ala Ile Thr Ser Arg Asp His Leu Pro Ile Ile Ala Gly
   115 120 125
   Gly Ser Asn Ser Tyr Leu Glu Ala Leu Ile Asp Asp Asp Asp Tyr Lys
   130 135 140
   Phe Arg Ser Arg Tyr Asp Phe Cys Cys Leu Trp Val Asp Val Ala Met
   145 150 155 160
   Pro Val Leu Asp Ser Tyr Val Ala Ala Arg Val Asp Gln Met Leu Arg
   165 170 175
   Ser Gly Met Val Glu Glu Leu Arg Pro Phe Phe Asn Ala Asn Gly Asp
   180 185 190
   Tyr Ser Arg Gly Ile Arg Arg Ala Ile Gly Val Pro Glu Phe Asp Glu
   195 200 205
   Tyr Phe Arg Arg Glu Gly Phe Ala Asp Glu Glu Thr Arg Lys Leu Leu
   210 215 220
   Leu Glu Arg Ala Val Arg Glu Met Lys Val Asn Thr Cys Lys Leu Ala
   225 230 235 240
   Arg Arg Gln Leu Gly Lys Ile Gln Arg Leu Arg Asn Val Lys Arg Trp
   245 250 255
   Glu Ile His Arg Val Asp Ala Thr Pro Val Phe Trp Lys Arg Gly Glu
   260 265 270
   Glu Ala Asp Glu Ala Trp Arg Lys Val Val Ala Glu Pro Ser Ala Met
   275 280 285
   Ile Val Ala Gln Phe Leu Tyr Lys Ala Lys Ser Asp Val Asn Val Val
   290 295 300
   Ser Gly Gly Phe Arg Val Pro Ala Gly Ser Thr Glu Ser Val Met Ala
   305 310 315 320
   Ala Ala Thr Cys
<210> 9
   <211> 1053
   <212> DNA
   <213> Petunia x hybrida
<400> 9
   atgttaattg tagtacatat tattagcatc acacgcatca tattcatcac cttaacccat      60
   aatcatctcc atttccttat gtttagatca ttatcataca atcacaagca cctcaaattc     120
   cttacaaacc cgaccacacg ggtactccga agaaacatgt cgtcatccac tgtagtaaca     180
   atacccggcc ccacacaaaa aaacaaaaac aaaatcatag taataatggg tgcaacaggt     240
   tcaggaaaat caaaactctc aatagacctc gtcacacgtc actatccttt ttccgaaatc     300
   attaactccg acaaaatcca aattaccaaa ggtttaaaca taaccacaaa caaaatcact     360
   gtacccgacc gacgtggcgt agttcatcat ttactcggcg agattgaccc cgactttaac     420
   ttttctcctt ctcatttccg gtcaattgct ggtcaacgca ttaactccat tattaatcgc     480
   cataaactcc cattcctcgt tggtgggtcc aactcatata tctacgcttt attaacaaac     540
   cggttcgacc cggattttaa ccctgattca aacccggttc attttatatc caacgagtta     600
   cgctacaact gttgttttat ttgggtcgat gtattaaacc cggttttgaa tgagtatttg     660
   gataaacggg tcgatgagat gatgaactcg ggtatgtatg aagaactgga acagtttttt     720
   aaagaaaaca ggttttcgga tccgggtttg gaaccgggtc gggccaccgg gttgaggaaa     780
   gcgatagggg taccggaaat ggagaggtat tttaagaaga gctgtacgta tgaggaagca     840
   gtgagggaaa taaaagaaaa cacgtggcgg ttagcgaaga agcagatgtg gaagatccaa     900
   cggttgagag aagcagggtg ggacctacaa agagtagatg ccacggaggc atttgtggag     960
   gcgatgagta ataagaagga aaagggaatt atttgggaaa aacaagtagt ggaaccaagt    1020
   gtcaagattg tgaaccgttt tttgttggac tga                                 1053
<210> 10
   <211> 350
   <212> PRT
   <213> Petunia x hybrida
<400> 10
   Met Leu Ile Val Val His Ile Ile Ser Ile Thr Arg Ile Ile Phe Ile
   1               5                   10                  15
   Thr Leu Thr His Asn His Leu His Phe Leu Met Phe Arg Ser Leu Ser
               20                  25                  30
   Tyr Asn His Lys His Leu Lys Phe Leu Thr Asn Pro Thr Thr Arg Val
           35                  40                  45
   Leu Arg Arg Asn Met Ser Ser Ser Thr Val Val Thr Ile Pro Gly Pro
       50                  55                  60
   Thr Gln Lys Asn Lys Asn Lys Ile Ile Val Ile Met Gly Ala Thr Gly
   65                  70                  75                  80
   Ser Gly Lys Ser Lys Leu Ser Ile Asp Leu Val Thr Arg His Tyr Pro
               85                  90                  95
   Phe Ser Glu Ile Ile Asn Ser Asp Lys Ile Gln Ile Thr Lys Gly Leu
               100                 105                 110
   Asn Ile Thr Thr Asn Lys Ile Thr Val Pro Asp Arg Arg Gly Val Val
           115                 120                 125
   His His Leu Leu Gly Glu Ile Asp Pro Asp Phe Asn Phe Ser Pro Ser
       130                 135                 140
   His Phe Arg Ser Ile Ala Gly Gln Arg Ile Asn Ser Ile Ile Asn Arg
   145                 150                 155                 160
   His Lys Leu Pro Phe Leu Val Gly Gly Ser Asn Ser Tyr Ile Tyr Ala
                   165                 170                 175
   Leu Leu Thr Asn Arg Phe Asp Pro Asp Phe Asn Pro Asp Ser Asn Pro
               180                 185                 190
   Val His Phe Ile Ser Asn Glu Leu Arg Tyr Asn Cys Cys Phe Ile Trp
           195                 200                 205
   Val Asp Val Leu Asn Pro Val Leu Asn Glu Tyr Leu Asp Lys Arg Val
       210                 215                 220
   Asp Glu Met Met Asn Ser Gly Met Tyr Glu Glu Leu Glu Gln Phe Phe
   225                 230                 235                 240
   Lys Glu Asn Arg Phe Ser Asp Pro Gly Leu Glu Pro Gly Arg Ala Thr
                   245                 250                 255
   Gly Leu Arg Lys Ala Ile Gly Val Pro Glu Met Glu Arg Tyr Phe Lys
               260                 265                 270
   Lys Ser Cys Thr Tyr Glu Glu Ala Val Arg Glu Ile Lys Glu Asn Thr
           275                 280                 285
   Trp Arg Leu Ala Lys Lys Gln Met Trp Lys Ile Gln Arg Leu Arg Glu
       290                 295                 300
   Ala Gly Trp Asp Leu Gln Arg Val Asp Ala Thr Glu Ala Phe Val Glu
   305                 310                 315                 320
   Ala Met Ser Asn Lys Lys Glu Lys Gly Ile Ile Trp Glu Lys Gln Val
                   325                 330                 335
   Val Glu Pro Ser Val Lys Ile Val Asn Arg Phe Leu Leu Asp
   340 345 350
<210> 11
   <211> 10786
   <212> DNA
   <213> Artificial
<220>
   <223> Synthetic construct
<400> 11
   gtcccgtatc acgcagcgcg tgagctaccc aggcactgcc gccgccggca caaccgttct 60
   tgaatcagaa cccgagggcg acgctgcccg cgaggtccag gcgctggccg ctgaaattaa 120
   atcaaaactc atttgagtta atgaggtaaa gagaaaatga gcaaaagcac aaacacgcta 180
   agtgccggcc gtccgagcgc acgcagcagc aaggctgcaa cgttggccag cctggcagac 240
   acgccagcca tgaagcgggt caactttcag ttgccggcgg aggatcacac caagctgaag 300
   atgtacgcgg tacgccaagg caagaccatt accgagctgc tatctgaata catcgcgcag 360
   ctaccagagt aaatgagcaa atgaataaat gagtagatga attttagcgg ctaaaggagg 420
   cggcatggaa aatcaagaac aaccaggcac cgacgccgtg gaatgcccca tgtgtggagg 480
   aacgggcggt tggccaggcg taagcggctg ggttgtctgc cggccctgca atggcactgg 540
   aacccccaag cccgaggaat cggcgtgacg gtcgcaaacc atccggcccg gtacaaatcg 600
   gcgcggcgct gggtgatgac ctggtggaga agttgaaggc cgcgcaggcc gcccagcggc 660
   aacgcatcga ggcagaagca cgccccggtg aatcgtggca agcggccgct gatcgaatcc 720
   gcaaagaatc ccggcaaccg ccggcagccg gtgcgccgtc gattaggaag ccgcccaagg 780
   gcgacgagca accagatttt ttcgttccga tgctctatga cgtgggcacc cgcgatagtc 840
   gcagcatcat ggacgtggcc gttttccgtc tgtcgaagcg tgaccgacga gctggcgagg 900
   tgatccgcta cgagcttcca gacgggcacg tagaggtttc cgcagggccg gccggcatgg 960
   ccagtgtgtg ggattacgac ctggtactga tggcggtttc ccatctaacc gaatccatga 1020
   accgataccg ggaagggaag ggagacaagc ccggccgcgt gttccgtcca cacgttgcgg 1080
   acgtactcaa gttctgccgg cgagccgatg gcggaaagca gaaagacgac ctggtagaaa 1140
   cctgcattcg gttaaacacc acgcacgttg ccatgcagcg tacgaagaag gccaagaacg 1200
   gccgcctggt gacggtatcc gagggtgaag ccttgattag ccgctacaag atcgtaaaga 1260
   gcgaaaccgg gcggccggag tacatcgaga tcgagctagc tgattggatg taccgcgaga 1320
   tcacagaagg caagaacccg gacgtgctga cggttcaccc cgattacttt ttgatcgatc 1380
   ccggcatcgg ccgttttctc taccgcctgg cacgccgcgc cgcaggcaag gcagaagcca 1440
   gatggttgtt caagacgatc tacgaacgca gtggcagcgc cggagagttc aagaagttct 1500
   gtttcaccgt gcgcaagctg atcgggtcaa atgacctgcc ggagtacgat ttgaaggagg 1560
   aggcggggca ggctggcccg atcctagtca tgcgctaccg caacctgatc gagggcgaag 1620
   catccgccgg ttcctaatgt acggagcaga tgctagggca aattgcccta gcaggggaaa 1680
   aaggtcgaaa aggtctcttt cctgtggata gcacgtacat tgggaaccca aagccgtaca 1740
   ttgggaaccg gaacccgtac attgggaacc caaagccgta cattgggaac cggtcacaca 1800
   tgtaagtgac tgatataaaa gagaaaaaag gcgatttttc cgcctaaaac tctttaaaac 1860
   ttattaaaac tcttaaaacc cgcctggcct gtgcataact gtctggccag cgcacagccg 1920
   aagagctgca aaaagcgcct acccttcggt cgctgcgctc cctacgcccc gccgcttcgc 1980
   gtcggcctat cgcggccgct ggccgctcaa aaatggctgg cctacggcca ggcaatctac 2040
   cagggcgcgg acaagccgcg ccgtcgccac tcgaccgccg gcgcccacat caaggcaccc 2100
   tgcctcgcgc gtttcggtga tgacggtgaa aacctctgac acatgcagct cccggagacg 2160
   gtcacagctt gtctgtaagc ggatgccggg agcagacaag cccgtcaggg cgcgtcagcg 2220
   ggtgttggcg ggtgtcgggg cgcagccatg acccagtcac gtagcgatag cggagtgtat 2280
   actggcttaa ctatgcggca tcagagcaga ttgtactgag agtgcaccat atgcggtgtg 2340
   aaataccgca cagatgcgta aggagaaaat accgcatcag gcgctcttcc gcttcctcgc 2400
   tcactgactc gctgcgctcg gtcgttcggc tgcggcgagc ggtatcagct cactcaaagg 2460
   cggtaatacg gttatccaca gaatcagggg ataacgcagg aaagaacatg tgagcaaaag 2520
   gccagcaaaa ggccaggaac cgtaaaaagg ccgcgttgct ggcgtttttc cataggctcc 2580
   gcccccctga cgagcatcac aaaaatcgac gctcaagtca gaggtggcga aacccgacag 2640
   gactataaag ataccaggcg tttccccctg gaagctccct cgtgcgctct cctgttccga 2700
   ccctgccgct taccggatac ctgtccgcct ttctcccttc gggaagcgtg gcgctttctc 2760
   atagctcacg ctgtaggtat ctcagttcgg tgtaggtcgt tcgctccaag ctgggctgtg 2820
   tgcacgaacc ccccgttcag cccgaccgct gcgccttatc cggtaactat cgtcttgagt 2880
   ccaacccggt aagacacgac ttatcgccac tggcagcagc cactggtaac aggattagca 2940
   gagcgaggta tgtaggcggt gctacagagt tcttgaagtg gtggcctaac tacggctaca 3000
   ctagaaggac agtatttggt atctgcgctc tgctgaagcc agttaccttc ggaaaaagag 3060
   ttggtagctc ttgatccggc aaacaaacca ccgctggtag cggtggtttt tttgtttgca 3120
   agcagcagat tacgcgcaga aaaaaaggat ctcaagaaga tcctttgatc ttttctacgg 3180
   ggtctgacgc tcagtggaac gaaaactcac gttaagggat tttggtcatg catgatatat 3240
   ctcccaattt gtgtagggct tattatgcac gcttaaaaat aataaaagca gacttgacct 3300
   gatagtttgg ctgtgagcaa ttatgtgctt agtgcatcta atcgcttgag ttaacgccgg 3360
   cgaagcggcg tcggcttgaa cgaatttcta gctagacatt atttgccgac taccttggtg 3420
   atctcgcctt tcacgtagtg gacaaattct tccaactgat ctgcgcgcga ggccaagcga 3480
   tcttcttctt gtccaagata agcctgtcta gcttcaagta tgacgggctg atactgggcc 3540
   ggcaggcgct ccattgccca gtcggcagcg acatccttcg gcgcgatttt gccggttact 3600
   gcgctgtacc aaatgcggga caacgtaagc actacatttc gctcatcgcc agcccagtcg 3660
   ggcggcgagt tccatagcgt taaggtttca tttagcgcct caaatagatc ctgttcagga 3720
   accggatcaa agagttcctc cgccgctgga cctaccaagg caacgctatg ttctcttgct 3780
   tttgtcagca agatagccag atcaatgtcg atcgtggctg gctcgaagat acctgcaaga 3840
   atgtcattgc gctgccattc tccaaattgc agttcgcgct tagctggata acgccacgga 3900
   atgatgtcgt cgtgcacaac aatggtgact tctacagcgc ggagaatctc gctctctcca 3960
   ggggaagccg aagtttccaa aaggtcgttg atcaaagctc gccgcgttgt ttcatcaagc 4020
   cttacggtca ccgtaaccag caaatcaata tcactgtgtg gcttcaggcc gccatccact 4080
   gcggagccgt acaaatgtac ggccagcaac gtcggttcga gatggcgctc gatgacgcca 4140
   actacctctg atagttgagt cgatacttcg gcgatcaccg cttcccccat gatgtttaac 4200
   tttgttttag ggcgactgcc ctgctgcgta acatcgttgc tgctccataa catcaaacat 4260
   cgacccacgg cgtaacgcgc ttgctgcttg gatgcccgag gcatagactg taccccaaaa 4320
   aaacatgtca taacaagaag ccatgaaaac cgccactgcg ccgttaccac cgctgcgttc 4380
   ggtcaaggtt ctggaccagt tgcgtgacgg cagttacgct acttgcatta cagcttacga 4440
   accgaacgag gcttatgtcc actgggttcg tgcccgaatt gatcacaggc agcaacgctc 4500
   tgtcatcgtt acaatcaaca tgctaccctc cgcgagatca tccgtgtttc aaacccggca 4560
   gcttagttgc cgttcttccg aatagcatcg gtaacatgag caaagtctgc cgccttacaa 4620
   cggctctccc gctgacgccg tcccggactg atgggctgcc tgtatcgagt ggtgattttg 4680
   tgccgagctg ccggtcgggg agctgttggc tggctggtgg caggatatat tgtggtgtaa 4740
   acaaattgac gcttagacaa cttaataaca cattgcggac gtttttaatg tactgaatta 4800
   acgccgaatt gaattcctcg agtacgtagg atccatttaa attctagagg cgcgccgata 4860
   tcctctctta aggtagcgag ctcttaatta atagggataa cagggtaatg cggccgcaag 4920
   ctaaaacgac ggccagtgaa ttatcaactt tgtatagaaa agttgctctg ccgacagtgg 4980
   tcccaaagat ggacccccac ccacgaggag catcgtggaa aaagaagacg ttccaaccac 5040
   gtcttcaaag caagtggatt gatgtgataa catggtggag cacgacactc tcgtctactc 5100
   caagaatatc aaagatacag tctcagaaga ccaaagggct attgagactt ttcaacaaag 5160
   ggtaatatcg ggaaacctcc tcggattcca ttgcccagct atctgtcact tcatcaaaag 5220
   gacagtagaa aaggaaggtg gcacctacaa atgccatcat tgcgataaag gaaaggctat 5280
   cgttcaagat gcctctgccg acagtggtcc caaagatgga cccccaccca cgaggagcat 5340
   cgtggaaaaa gaagacgttc caaccacgtc ttcaaagcaa gtggattgat gtgatatctc 5400
   cactgacgta agggatgacg cacaatccca ctatccttcg caagaccctt cctctatata 5460
   aggaagttca tttcatttgg agaggacacg ctgcaagttt gtacaaaaaa gcaggctatg 5520
   gcaattacct tatccgcaac ttctttacct atttccgccc ggatccgggc aggttctccg 5580
   gccgcttggg tggagaggct attcggctat gactgggcac aacagacaat cggctgctct 5640
   gatgccgccg tgttccggct gtcagcgcag gggcgcccgg ttctttttgt caagaccgac 5700
   ctgtccggtg ccctgaatga actgcaggac gaggcagcgc ggctatcgtg gctggccacg 5760
   acgggcgttc cttgcgcagc tgtgctcgac gttgtcactg aagcgggaag ggactggctg 5820
   ctattgggcg aagtgccggg gcaggatctc ctgtcatctc accttgctcc tgccgagaaa 5880
   gtatccatca tggctgatgc aatgcggcgg ctgcatacgc ttgatccggc tacctgccca 5940
   ttcgaccacc aagcgaaaca tcgcatcgag cgagcacgta ctcggatgga agccggtctt 6000
   gtcgatcagg atgatctgga cgaagagcat caggggctcg cgccagccga actgttcgcc 6060
   aggctcaagg cgcgcatgcc cgacggcgag gatctcgtcg tgacccatgg cgatgcctgc 6120
   ttgccgaata tcatggtgga aaatggccgc ttttctggat tcatcgactg tggccggctg 6180
   ggtgtggcgg accgctatca ggacatagcg ttggctaccc gtgatattgc tgaagagctt 6240
   ggcggcgaat gggctgaccg cttcctcgtg ctttacggta tcgccgctcc cgattcgcag 6300
   cgcatcgcct tctatcgcct tcttgacgag ttcttctgaa cccagctttc ttgtacaaag 6360
   tggagtccgc aaaaatcacc agtctctctc tacaaatcta tctctctcta tttttctcca 6420
   gaataatgtg tgagtagttc ccagataagg gaattagggt tcttataggg tttcgctcat 6480
   gtgttgagca tataagaaac ccttagtatg tatttgtatt tgtaaaatac ttctatcaat 6540
   aaaatttcta attcctaaaa ccaaaatcca gtgacctcaa ctttattata catagttgat 6600
   aattcactgg ccgtgcttat tccatggctg caggtcgacg aattcaccgg ttagggataa 6660
   cagggtaatc gctaccttag gaccgttata gttacggcca gtgccattac cctgttatcc 6720
   ctaaccggtg acaactttgt atagaaaagt tggtttgtgt cttctagatt aatcctccaa 6780
   acttttgatt aaccaaaaaa attatcaaac taacatgttc tccttttttc tttagaaatt 6840
   ctaacgaatt tatctttata ctgatttgaa tatacttaat ttggtcattt ggatgccctt 6900
   tacaacctcc ttaccaaact cactatggca aatatatact attttccatt gtaacataaa 6960
   tgtccataat ttgaattaaa ttcgttgcag tacgaaacca tccaactttg tccaaaaaca 7020
   aaatccttat aactatttac tttaatgtaa atatatcctc tacttttgtt tttacaaccc 7080
   tagctcaaac aaatttatta tttgcgataa aaaatcatat cgaacaaact cgatgatttt 7140
   ttttttctta cgttattaat gaaactaaaa tatagaaaaa aacaagatga accaaatttt 7200
   cacctatcta actacttaaa tataatatga ttaaatttgg taaagtttga aaagtttctt 7260
   tagaaatgtg aaatattgat cacagtttct attgctaaaa tcaccaacaa aacgcatgtc 7320
   gccattcata attatggttt cacacctaca actaggctaa taagtaaata agtagacaac 7380
   tagactcagg tttgaaaaaa ccataaaagc catatagcgt tttctcattg aaactgcgaa 7440
   cacgatcgtg tgaatgttgc agtttctagt tttgatacaa acaaacaaaa acacaattta 7500
   atcttagatt aaaaagaaaa aagagaacgg agcccactag ccactccttc aaacgtgtct 7560
   taccaactct cttctagaaa caaattaggc ttcaccttcc tcttccaacc tctctctctc 7620
   tctctctctc tttttctcaa accatctctc cataaagccc taatttcttc atcacaagaa 7680
   tcagaagaag aaacaagttt gtacaaaaaa gcaggcttac tgcaaaaaac ttatggacct 7740
   gcatctaatt ttcggtccaa cttgcacagg aaagacgacg accgcgatag ctcttgccca 7800
   gcagacaggg cttccagtcc tttcgcttga tcgggtccaa tgctgtcctc aactatcaac 7860
   cggaagcgga cgaccaacag tggaagaact gaaaggaacg acgcgtctct accttgatga 7920
   tcggcctctg gtggagggta tcatcgcagc caagcaagct catcataggc tgatcgagga 7980
   ggtgtataat catgaggcca acggcgggct tattcttgag ggaggatcca cctcgttgct 8040
   caactgcatg gcgcgaaaca gctattggag tgcagatttt cgttggcata ttattcgcca 8100
   caagttaccc gaccaagaga ccttcatgaa agcggccaag gccagagtta agcagatgtt 8160
   gcaccccgct gcaggccatt ctattattca agagttggtt tatctttgga atgaacctcg 8220
   gctgaggccc attctgaaag agatcgatgg atatcgatat gccatgttgt ttgctagcca 8280
   gaaccagatc acggcagata tgctattgca gcttgacgca aatatggaag gtaagttgat 8340
   taatgggatc gctcaggagt atttcatcca tgcgcgccaa caggaacaga aattccccca 8400
   agttaacgca gccgctttcg acggattcga aggtcatccg ttcggaatgt attaggtacc 8460
   cagctttctt gtacaaagtg ggatcgttca aacatttggc aataaagttt cttaagattg 8520
   aatcctgttg ccggtcttgc gatgattatc atataatttc tgttgaatta cgttaagcat 8580
   gtaataatta acatgtaatg catgacgtta tttatgagat gggtttttat gattagagtc 8640
   ccgcaattat acatttaata cgcgatagaa aacaaaatat agcgcgcaaa ctaggataaa 8700
   ttatcgcgcg cggtgtcatc tatgttacta gatccaactt tattatacat agttgattcg 8760
   tcgacctgca gtcgctacct taggaccgtt atagttatgg caaacagcta ttatgggtat 8820
   tatgggtggt tctttatgcg gacactgacg gctttatgcc tgcaggtcgc gagcgatcgc 8880
   ggtaccgccc gggcgtcgac aggcctaagc ttagcttgag cttggatcag attgtcgttt 8940
   cccgccttca gtttaaacta tcagtgtttg acaggatata ttggcgggta aacctaagag 9000
   aaaagagcgt ttattagaat aacggatatt taaaagggcg tgaaaaggtt tatccgttcg 9060
   tccatttgta tgtgcatgcc aaccacaggg ttcccctcgg gatcaaagta ctttgatcca 9120
   acccctccgc tgctatagtg cagtcggctt ctgacgttca gtgcagccgt cttctgaaaa 9180
   cgacatgtcg cacaagtcct aagttacgcg acaggctgcc gccctgccct tttcctggcg 9240
   ttttcttgtc gcgtgtttta gtcgcataaa gtagaatact tgcgactaga accggagaca 9300
   ttacgccatg aacaagagcg ccgccgctgg cctgctgggc tatgcccgcg tcagcaccga 9360
   cgaccaggac ttgaccaacc aacgggccga actgcacgcg gccggctgca ccaagctgtt 9420
   ttccgagaag atcaccggca ccaggcgcga ccgcccggag ctggccagga tgcttgacca 9480
   cctacgccct ggcgacgttg tgacagtgac caggctagac cgcctggccc gcagcacccg 9540
   cgacctactg gacattgccg agcgcatcca ggaggccggc gcgggcctgc gtagcctggc 9600
   agagccgtgg gccgacacca ccacgccggc cggccgcatg gtgttgaccg tgttcgccgg 9660
   cattgccgag ttcgagcgtt ccctaatcat cgaccgcacc cggagcgggc gcgaggccgc 9720
   caaggcccga ggcgtgaagt ttggcccccg ccctaccctc accccggcac agatcgcgca 9780
   cgcccgcgag ctgatcgacc aggaaggccg caccgtgaaa gaggcggctg cactgcttgg 9840
   cgtgcatcgc tcgaccctgt accgcgcact tgagcgcagc gaggaagtga cgcccaccga 9900
   ggccaggcgg cgcggtgcct tccgtgagga cgcattgacc gaggccgacg ccctggcggc 9960
   cgccgagaat gaacgccaag aggaacaagc atgaaaccgc accaggacgg ccaggacgaa 10020
   ccgtttttca ttaccgaaga gatcgaggcg gagatgatcg cggccgggta cgtgttcgag 10080
   ccgcccgcgc acgtctcaac cgtgcggctg catgaaatcc tggccggttt gtctgatgcc 10140
   aagctggcgg cctggccggc cagcttggcc gctgaagaaa ccgagcgccg ccgtctaaaa 10200
   aggtgatgtg tatttgagta aaacagcttg cgtcatgcgg tcgctgcgta tatgatgcga 10260
   tgagtaaata aacaaatacg caaggggaac gcatgaaggt tatcgctgta cttaaccaga 10320
   aaggcgggtc aggcaagacg accatcgcaa cccatctagc ccgcgccctg caactcgccg 10380
   gggccgatgt tctgttagtc gattccgatc cccagggcag tgcccgcgat tgggcggccg 10440
   tgcgggaaga tcaaccgcta accgttgtcg gcatcgaccg cccgacgatt gaccgcgacg 10500
   tgaaggccat cggccggcgc gacttcgtag tgatcgacgg agcgccccag gcggcggact 10560
   tggctgtgtc cgcgatcaag gcagccgact tcgtgctgat tccggtgcag ccaagccctt 10620
   acgacatatg ggccaccgcc gacctggtgg agctggttaa gcagcgcatt gaggtcacgg 10680
   atggaaggct acaagcggcc tttgtcgtgt cgcgggcgat caaaggcacg cgcatcggcg 10740
   gtgaggttgc cgaggcgctg gccgggtacg agctgcccat tcttga 10786
<210> 12
   <211> 10468
   <212> DNA
   <213> Artificial
<220>
   <223> Synthetic construct
<400> 12
   gtcccgtatc acgcagcgcg tgagctaccc aggcactgcc gccgccggca caaccgttct 60
   tgaatcagaa cccgagggcg acgctgcccg cgaggtccag gcgctggccg ctgaaattaa 120
   atcaaaactc atttgagtta atgaggtaaa gagaaaatga gcaaaagcac aaacacgcta 180
   agtgccggcc gtccgagcgc acgcagcagc aaggctgcaa cgttggccag cctggcagac 240
   acgccagcca tgaagcgggt caactttcag ttgccggcgg aggatcacac caagctgaag 300
   atgtacgcgg tacgccaagg caagaccatt accgagctgc tatctgaata catcgcgcag 360
   ctaccagagt aaatgagcaa atgaataaat gagtagatga attttagcgg ctaaaggagg 420
   cggcatggaa aatcaagaac aaccaggcac cgacgccgtg gaatgcccca tgtgtggagg 480
   aacgggcggt tggccaggcg taagcggctg ggttgtctgc cggccctgca atggcactgg 540
   aacccccaag cccgaggaat cggcgtgacg gtcgcaaacc atccggcccg gtacaaatcg 600
   gcgcggcgct gggtgatgac ctggtggaga agttgaaggc cgcgcaggcc gcccagcggc 660
   aacgcatcga ggcagaagca cgccccggtg aatcgtggca agcggccgct gatcgaatcc 720
   gcaaagaatc ccggcaaccg ccggcagccg gtgcgccgtc gattaggaag ccgcccaagg 780
   gcgacgagca accagatttt ttcgttccga tgctctatga cgtgggcacc cgcgatagtc 840
   gcagcatcat ggacgtggcc gttttccgtc tgtcgaagcg tgaccgacga gctggcgagg 900
   tgatccgcta cgagcttcca gacgggcacg tagaggtttc cgcagggccg gccggcatgg 960
   ccagtgtgtg ggattacgac ctggtactga tggcggtttc ccatctaacc gaatccatga 1020
   accgataccg ggaagggaag ggagacaagc ccggccgcgt gttccgtcca cacgttgcgg 1080
   acgtactcaa gttctgccgg cgagccgatg gcggaaagca gaaagacgac ctggtagaaa 1140
   cctgcattcg gttaaacacc acgcacgttg ccatgcagcg tacgaagaag gccaagaacg 1200
   gccgcctggt gacggtatcc gagggtgaag ccttgattag ccgctacaag atcgtaaaga 1260
   gcgaaaccgg gcggccggag tacatcgaga tcgagctagc tgattggatg taccgcgaga 1320
   tcacagaagg caagaacccg gacgtgctga cggttcaccc cgattacttt ttgatcgatc 1380
   ccggcatcgg ccgttttctc taccgcctgg cacgccgcgc cgcaggcaag gcagaagcca 1440
   gatggttgtt caagacgatc tacgaacgca gtggcagcgc cggagagttc aagaagttct 1500
   gtttcaccgt gcgcaagctg atcgggtcaa atgacctgcc ggagtacgat ttgaaggagg 1560
   aggcggggca ggctggcccg atcctagtca tgcgctaccg caacctgatc gagggcgaag 1620
   catccgccgg ttcctaatgt acggagcaga tgctagggca aattgcccta gcaggggaaa 1680
   aaggtcgaaa aggtctcttt cctgtggata gcacgtacat tgggaaccca aagccgtaca 1740
   ttgggaaccg gaacccgtac attgggaacc caaagccgta cattgggaac cggtcacaca 1800
   tgtaagtgac tgatataaaa gagaaaaaag gcgatttttc cgcctaaaac tctttaaaac 1860
   ttattaaaac tcttaaaacc cgcctggcct gtgcataact gtctggccag cgcacagccg 1920
   aagagctgca aaaagcgcct acccttcggt cgctgcgctc cctacgcccc gccgcttcgc 1980
   gtcggcctat cgcggccgct ggccgctcaa aaatggctgg cctacggcca ggcaatctac 2040
   cagggcgcgg acaagccgcg ccgtcgccac tcgaccgccg gcgcccacat caaggcaccc 2100
   tgcctcgcgc gtttcggtga tgacggtgaa aacctctgac acatgcagct cccggagacg 2160
   gtcacagctt gtctgtaagc ggatgccggg agcagacaag cccgtcaggg cgcgtcagcg 2220
   ggtgttggcg ggtgtcgggg cgcagccatg acccagtcac gtagcgatag cggagtgtat 2280
   actggcttaa ctatgcggca tcagagcaga ttgtactgag agtgcaccat atgcggtgtg 2340
   aaataccgca cagatgcgta aggagaaaat accgcatcag gcgctcttcc gcttcctcgc 2400
   tcactgactc gctgcgctcg gtcgttcggc tgcggcgagc ggtatcagct cactcaaagg 2460
   cggtaatacg gttatccaca gaatcagggg ataacgcagg aaagaacatg tgagcaaaag 2520
   gccagcaaaa ggccaggaac cgtaaaaagg ccgcgttgct ggcgtttttc cataggctcc 2580
   gcccccctga cgagcatcac aaaaatcgac gctcaagtca gaggtggcga aacccgacag 2640
   gactataaag ataccaggcg tttccccctg gaagctccct cgtgcgctct cctgttccga 2700
   ccctgccgct taccggatac ctgtccgcct ttctcccttc gggaagcgtg gcgctttctc 2760
   atagctcacg ctgtaggtat ctcagttcgg tgtaggtcgt tcgctccaag ctgggctgtg 2820
   tgcacgaacc ccccgttcag cccgaccgct gcgccttatc cggtaactat cgtcttgagt 2880
   ccaacccggt aagacacgac ttatcgccac tggcagcagc cactggtaac aggattagca 2940
   gagcgaggta tgtaggcggt gctacagagt tcttgaagtg gtggcctaac tacggctaca 3000
   ctagaaggac agtatttggt atctgcgctc tgctgaagcc agttaccttc ggaaaaagag 3060
   ttggtagctc ttgatccggc aaacaaacca ccgctggtag cggtggtttt tttgtttgca 3120
   agcagcagat tacgcgcaga aaaaaaggat ctcaagaaga tcctttgatc ttttctacgg 3180
   ggtctgacgc tcagtggaac gaaaactcac gttaagggat tttggtcatg catgatatat 3240
   ctcccaattt gtgtagggct tattatgcac gcttaaaaat aataaaagca gacttgacct 3300
   gatagtttgg ctgtgagcaa ttatgtgctt agtgcatcta atcgcttgag ttaacgccgg 3360
   cgaagcggcg tcggcttgaa cgaatttcta gctagacatt atttgccgac taccttggtg 3420
   atctcgcctt tcacgtagtg gacaaattct tccaactgat ctgcgcgcga ggccaagcga 3480
   tcttcttctt gtccaagata agcctgtcta gcttcaagta tgacgggctg atactgggcc 3540
   ggcaggcgct ccattgccca gtcggcagcg acatccttcg gcgcgatttt gccggttact 3600
   gcgctgtacc aaatgcggga caacgtaagc actacatttc gctcatcgcc agcccagtcg 3660
   ggcggcgagt tccatagcgt taaggtttca tttagcgcct caaatagatc ctgttcagga 3720
   accggatcaa agagttcctc cgccgctgga cctaccaagg caacgctatg ttctcttgct 3780
   tttgtcagca agatagccag atcaatgtcg atcgtggctg gctcgaagat acctgcaaga 3840
   atgtcattgc gctgccattc tccaaattgc agttcgcgct tagctggata acgccacgga 3900
   atgatgtcgt cgtgcacaac aatggtgact tctacagcgc ggagaatctc gctctctcca 3960
   ggggaagccg aagtttccaa aaggtcgttg atcaaagctc gccgcgttgt ttcatcaagc 4020
   cttacggtca ccgtaaccag caaatcaata tcactgtgtg gcttcaggcc gccatccact 4080
   gcggagccgt acaaatgtac ggccagcaac gtcggttcga gatggcgctc gatgacgcca 4140
   actacctctg atagttgagt cgatacttcg gcgatcaccg cttcccccat gatgtttaac 4200
   tttgttttag ggcgactgcc ctgctgcgta acatcgttgc tgctccataa catcaaacat 4260
   cgacccacgg cgtaacgcgc ttgctgcttg gatgcccgag gcatagactg taccccaaaa 4320
   aaacatgtca taacaagaag ccatgaaaac cgccactgcg ccgttaccac cgctgcgttc 4380
   ggtcaaggtt ctggaccagt tgcgtgacgg cagttacgct acttgcatta cagcttacga 4440
   accgaacgag gcttatgtcc actgggttcg tgcccgaatt gatcacaggc agcaacgctc 4500
   tgtcatcgtt acaatcaaca tgctaccctc cgcgagatca tccgtgtttc aaacccggca 4560
   gcttagttgc cgttcttccg aatagcatcg gtaacatgag caaagtctgc cgccttacaa 4620
   cggctctccc gctgacgccg tcccggactg atgggctgcc tgtatcgagt ggtgattttg 4680
   tgccgagctg ccggtcgggg agctgttggc tggctggtgg caggatatat tgtggtgtaa 4740
   acaaattgac gcttagacaa cttaataaca cattgcggac gtttttaatg tactgaatta 4800
   acgccgaatt gaattcctcg agtacgtagg atccatttaa attctagagg cgcgccgata 4860
   tcctctctta aggtagcgag ctcttaatta atagggataa cagggtaatg cggccgcaag 4920
   ctaaaacgac ggccagtgaa ttatcaactt tgtatagaaa agttgctctg ccgacagtgg 4980
   tcccaaagat ggacccccac ccacgaggag catcgtggaa aaagaagacg ttccaaccac 5040
   gtcttcaaag caagtggatt gatgtgataa catggtggag cacgacactc tcgtctactc 5100
   caagaatatc aaagatacag tctcagaaga ccaaagggct attgagactt ttcaacaaag 5160
   ggtaatatcg ggaaacctcc tcggattcca ttgcccagct atctgtcact tcatcaaaag 5220
   gacagtagaa aaggaaggtg gcacctacaa atgccatcat tgcgataaag gaaaggctat 5280
   cgttcaagat gcctctgccg acagtggtcc caaagatgga cccccaccca cgaggagcat 5340
   cgtggaaaaa gaagacgttc caaccacgtc ttcaaagcaa gtggattgat gtgatatctc 5400
   cactgacgta agggatgacg cacaatccca ctatccttcg caagaccctt cctctatata 5460
   aggaagttca tttcatttgg agaggacacg ctgcaagttt gtacaaaaaa gcaggctatg 5520
   gcaattacct tatccgcaac ttctttacct atttccgccc ggatccgggc aggttctccg 5580
   gccgcttggg tggagaggct attcggctat gactgggcac aacagacaat cggctgctct 5640
   gatgccgccg tgttccggct gtcagcgcag gggcgcccgg ttctttttgt caagaccgac 5700
   ctgtccggtg ccctgaatga actgcaggac gaggcagcgc ggctatcgtg gctggccacg 5760
   acgggcgttc cttgcgcagc tgtgctcgac gttgtcactg aagcgggaag ggactggctg 5820
   ctattgggcg aagtgccggg gcaggatctc ctgtcatctc accttgctcc tgccgagaaa 5880
   gtatccatca tggctgatgc aatgcggcgg ctgcatacgc ttgatccggc tacctgccca 5940
   ttcgaccacc aagcgaaaca tcgcatcgag cgagcacgta ctcggatgga agccggtctt 6000
   gtcgatcagg atgatctgga cgaagagcat caggggctcg cgccagccga actgttcgcc 6060
   aggctcaagg cgcgcatgcc cgacggcgag gatctcgtcg tgacccatgg cgatgcctgc 6120
   ttgccgaata tcatggtgga aaatggccgc ttttctggat tcatcgactg tggccggctg 6180
   ggtgtggcgg accgctatca ggacatagcg ttggctaccc gtgatattgc tgaagagctt 6240
   ggcggcgaat gggctgaccg cttcctcgtg ctttacggta tcgccgctcc cgattcgcag 6300
   cgcatcgcct tctatcgcct tcttgacgag ttcttctgaa cccagctttc ttgtacaaag 6360
   tggagtccgc aaaaatcacc agtctctctc tacaaatcta tctctctcta tttttctcca 6420
   gaataatgtg tgagtagttc ccagataagg gaattagggt tcttataggg tttcgctcat 6480
   gtgttgagca tataagaaac ccttagtatg tatttgtatt tgtaaaatac ttctatcaat 6540
   aaaatttcta attcctaaaa ccaaaatcca gtgacctcaa ctttattata catagttgat 6600
   aattcactgg ccgtgcttat tccatggctg caggtcgacg aattcaccgg ttagggataa 6660
   cagggtaata actataacgg tcctaaggta gcgagcggcc gcaagctaaa acgacggcca 6720
   gtgaattatc aactttgtat agaaaagttg gtttgtgtct tctagattaa tcctccaaac 6780
   ttttgattaa ccaaaaaaat tatcaaacta acatgttctc cttttttctt tagaaattct 6840
   aacgaattta tctttatact gatttgaata tacttaattt ggtcatttgg atgcccttta 6900
   caacctcctt accaaaatat tgatcacagt ttctattgct aaaatcacca acaaaacgca 6960
   tgtcgccatt cataattatg gtttcacacc tacaactagg ctaataagta aataagtaga 7020
   caactagact caggtttgaa aaaaccataa aagccatata gcgttttctc attgaaactg 7080
   cgaacacgat cgtgtgaatg ttgcagtttc tagttttgat acaaacaaac aaaaacacaa 7140
   tttaatctta gattaaaaag aaaaaagaga acggagccca ctagccactc cttcaaacgt 7200
   gtcttaccaa ctctcttcta gaaacaaatt aggcttcacc ttcctcttcc aacctctctc 7260
   tctctctctc tctctctttc tcaaaccatc tctccataaa gccctaattt cttcatcaca 7320
   agaatcagaa gaagaaacaa gtttgtacaa aaaagcaggc ttactgcaaa aaacttatgg 7380
   acctgcatct aattttcggt ccaacttgca caggaaagac gacgaccgcg atagctcttg 7440
   cccagcagac agggcttcca gtcctttcgc ttgatcgggt ccaatgctgt cctcaactat 7500
   caaccggaag cggacgacca acagtggaag aactgaaagg aacgacgcgt ctctaccttg 7560
   atgatcggcc tctggtggag ggtatcatcg cagccaagca agctcatcat aggctgatcg 7620
   aggaggtgta taatcatgag gccaacggcg ggcttattct tgagggagga tccacctcgt 7680
   tgctcaactg catggcgcga aacagctatt ggagtgcaga ttttcgttgg catattattc 7740
   gccacaagtt acccgaccaa gagaccttca tgaaagcggc caaggccaga gttaagcaga 7800
   tgttgcaccc cgctgcaggc cattctatta ttcaagagtt ggtttatctt tggaatgaac 7860
   ctcggctgag gcccattctg aaagagatcg atggatatcg atatgccatg ttgtttgcta 7920
   gccagaacca gatcacggca gatatgctat tgcagcttga cgcaaatatg gaaggtaagt 7980
   tgattaatgg gatcgctcag gagtatttca tccatgcgcg ccaacaggaa cagaaattcc 8040
   cccaagttaa cgcagccgct ttcgacggat tcgaaggtca tccgttcgga atgtattagg 8100
   tacccagctt tcttgtacaa agtgggatcg ttcaaacatt tggcaataaa gtttcttaag 8160
   attgaatcct gttgccggtc ttgcgatgat tatcatataa tttctgttga attacgttaa 8220
   gcatgtaata attaacatgt aatgcatgac gttatttatg agatgggttt ttatgattag 8280
   agtcccgcaa ttatacattt aatacgcgat agaaaacaaa atatagcgcg caaactagga 8340
   taaattatcg cgcgcggtgt catctatgtt actagatcca actttattat acatagttga 8400
   taattcactg gccgtcgctt attccatggc tgcaggtcga cgaattcacc ggttaactat 8460
   aacggtccta aggtagcgat ggcaaacagc tattatgggt attatgggtg gttctttatg 8520
   cggacactga cggctttatg cctgcaggtc gcgagcgatc gcggtaccgc ccgggcgtcg 8580
   acaggcctaa gcttagcttg agcttggatc agattgtcgt ttcccgcctt cagtttaaac 8640
   tatcagtgtt tgacaggata tattggcggg taaacctaag agaaaagagc gtttattaga 8700
   ataacggata tttaaaaggg cgtgaaaagg tttatccgtt cgtccatttg tatgtgcatg 8760
   ccaaccacag ggttcccctc gggatcaaag tactttgatc caacccctcc gctgctatag 8820
   tgcagtcggc ttctgacgtt cagtgcagcc gtcttctgaa aacgacatgt cgcacaagtc 8880
   ctaagttacg cgacaggctg ccgccctgcc cttttcctgg cgttttcttg tcgcgtgttt 8940
   tagtcgcata aagtagaata cttgcgacta gaaccggaga cattacgcca tgaacaagag 9000
   cgccgccgct ggcctgctgg gctatgcccg cgtcagcacc gacgaccagg acttgaccaa 9060
   ccaacgggcc gaactgcacg cggccggctg caccaagctg ttttccgaga agatcaccgg 9120
   caccaggcgc gaccgcccgg agctggccag gatgcttgac cacctacgcc ctggcgacgt 9180
   tgtgacagtg accaggctag accgcctggc ccgcagcacc cgcgacctac tggacattgc 9240
   cgagcgcatc caggaggccg gcgcgggcct gcgtagcctg gcagagccgt gggccgacac 9300
   caccacgccg gccggccgca tggtgttgac cgtgttcgcc ggcattgccg agttcgagcg 9360
   ttccctaatc atcgaccgca cccggagcgg gcgcgaggcc gccaaggccc gaggcgtgaa 9420
   gtttggcccc cgccctaccc tcaccccggc acagatcgcg cacgcccgcg agctgatcga 9480
   ccaggaaggc cgcaccgtga aagaggcggc tgcactgctt ggcgtgcatc gctcgaccct 9540
   gtaccgcgca cttgagcgca gcgaggaagt gacgcccacc gaggccaggc ggcgcggtgc 9600
   cttccgtgag gacgcattga ccgaggccga cgccctggcg gccgccgaga atgaacgcca 9660
   agaggaacaa gcatgaaacc gcaccaggac ggccaggacg aaccgttttt cattaccgaa 9720
   gagatcgagg cggagatgat cgcggccggg tacgtgttcg agccgcccgc gcacgtctca 9780
   accgtgcggc tgcatgaaat cctggccggt ttgtctgatg ccaagctggc ggcctggccg 9840
   gccagcttgg ccgctgaaga aaccgagcgc cgccgtctaa aaaggtgatg tgtatttgag 9900
   taaaacagct tgcgtcatgc ggtcgctgcg tatatgatgc gatgagtaaa taaacaaata 9960
   cgcaagggga acgcatgaag gttatcgctg tacttaacca gaaaggcggg tcaggcaaga 10020
   cgaccatcgc aacccatcta gcccgcgccc tgcaactcgc cggggccgat gttctgttag 10080
   tcgattccga tccccagggc agtgcccgcg attgggcggc cgtgcgggaa gatcaaccgc 10140
   taaccgttgt cggcatcgac cgcccgacga ttgaccgcga cgtgaaggcc atcggccggc 10200
   gcgacttcgt agtgatcgac ggagcgcccc aggcggcgga cttggctgtg tccgcgatca 10260
   aggcagccga cttcgtgctg attccggtgc agccaagccc ttacgacata tgggccaccg 10320
   ccgacctggt ggagctggtt aagcagcgca ttgaggtcac ggatggaagg ctacaagcgg 10380
   cctttgtcgt gtcgcgggcg atcaaaggca cgcgcatcgg cggtgaggtt gccgaggcgc 10440
   tggccgggta cgagctgccc attcttga 10468
<210> 13
   <211> 11000
   <212> DNA
   <213> Artificial
<220>
   <223> Synthetic construct
<400> 13
   gtcccgtatc acgcagcgcg tgagctaccc aggcactgcc gccgccggca caaccgttct    60
   tgaatcagaa cccgagggcg acgctgcccg cgaggtccag gcgctggccg ctgaaattaa   120
   atcaaaactc atttgagtta atgaggtaaa gagaaaatga gcaaaagcac aaacacgcta   180
   agtgccggcc gtccgagcgc acgcagcagc aaggctgcaa cgttggccag cctggcagac   240
   acgccagcca tgaagcgggt caactttcag ttgccggcgg aggatcacac caagctgaag   300
   atgtacgcgg tacgccaagg caagaccatt accgagctgc tatctgaata catcgcgcag   360
   ctaccagagt aaatgagcaa atgaataaat gagtagatga attttagcgg ctaaaggagg   420
   cggcatggaa aatcaagaac aaccaggcac cgacgccgtg gaatgcccca tgtgtggagg 480
   aacgggcggt tggccaggcg taagcggctg ggttgtctgc cggccctgca atggcactgg 540
   aacccccaag cccgaggaat cggcgtgacg gtcgcaaacc atccggcccg gtacaaatcg 600
   gcgcggcgct gggtgatgac ctggtggaga agttgaaggc cgcgcaggcc gcccagcggc 660
   aacgcatcga ggcagaagca cgccccggtg aatcgtggca agcggccgct gatcgaatcc 720
   gcaaagaatc ccggcaaccg ccggcagccg gtgcgccgtc gattaggaag ccgcccaagg 780
   gcgacgagca accagatttt ttcgttccga tgctctatga cgtgggcacc cgcgatagtc 840
   gcagcatcat ggacgtggcc gttttccgtc tgtcgaagcg tgaccgacga gctggcgagg 900
   tgatccgcta cgagcttcca gacgggcacg tagaggtttc cgcagggccg gccggcatgg 960
   ccagtgtgtg ggattacgac ctggtactga tggcggtttc ccatctaacc gaatccatga 1020
   accgataccg ggaagggaag ggagacaagc ccggccgcgt gttccgtcca cacgttgcgg 1080
   acgtactcaa gttctgccgg cgagccgatg gcggaaagca gaaagacgac ctggtagaaa 1140
   cctgcattcg gttaaacacc acgcacgttg ccatgcagcg tacgaagaag gccaagaacg 1200
   gccgcctggt gacggtatcc gagggtgaag ccttgattag ccgctacaag atcgtaaaga 1260
   gcgaaaccgg gcggccggag tacatcgaga tcgagctagc tgattggatg taccgcgaga 1320
   tcacagaagg caagaacccg gacgtgctga cggttcaccc cgattacttt ttgatcgatc 1380
   ccggcatcgg ccgttttctc taccgcctgg cacgccgcgc cgcaggcaag gcagaagcca 1440
   gatggttgtt caagacgatc tacgaacgca gtggcagcgc cggagagttc aagaagttct 1500
   gtttcaccgt gcgcaagctg atcgggtcaa atgacctgcc ggagtacgat ttgaaggagg 1560
   aggcggggca ggctggcccg atcctagtca tgcgctaccg caacctgatc gagggcgaag 1620
   catccgccgg ttcctaatgt acggagcaga tgctagggca aattgcccta gcaggggaaa 1680
   aaggtcgaaa aggtctcttt cctgtggata gcacgtacat tgggaaccca aagccgtaca 1740
   ttgggaaccg gaacccgtac attgggaacc caaagccgta cattgggaac cggtcacaca 1800
   tgtaagtgac tgatataaaa gagaaaaaag gcgatttttc cgcctaaaac tctttaaaac 1860
   ttattaaaac tcttaaaacc cgcctggcct gtgcataact gtctggccag cgcacagccg 1920
   aagagctgca aaaagcgcct acccttcggt cgctgcgctc cctacgcccc gccgcttcgc 1980
   gtcggcctat cgcggccgct ggccgctcaa aaatggctgg cctacggcca ggcaatctac 2040
   cagggcgcgg acaagccgcg ccgtcgccac tcgaccgccg gcgcccacat caaggcaccc 2100
   tgcctcgcgc gtttcggtga tgacggtgaa aacctctgac acatgcagct cccggagacg 2160
   gtcacagctt gtctgtaagc ggatgccggg agcagacaag cccgtcaggg cgcgtcagcg 2220
   ggtgttggcg ggtgtcgggg cgcagccatg acccagtcac gtagcgatag cggagtgtat 2280
   actggcttaa ctatgcggca tcagagcaga ttgtactgag agtgcaccat atgcggtgtg 2340
   aaataccgca cagatgcgta aggagaaaat accgcatcag gcgctcttcc gcttcctcgc 2400
   tcactgactc gctgcgctcg gtcgttcggc tgcggcgagc ggtatcagct cactcaaagg 2460
   cggtaatacg gttatccaca gaatcagggg ataacgcagg aaagaacatg tgagcaaaag 2520
   gccagcaaaa ggccaggaac cgtaaaaagg ccgcgttgct ggcgtttttc cataggctcc 2580
   gcccccctga cgagcatcac aaaaatcgac gctcaagtca gaggtggcga aacccgacag 2640
   gactataaag ataccaggcg tttccccctg gaagctccct cgtgcgctct cctgttccga 2700
   ccctgccgct taccggatac ctgtccgcct ttctcccttc gggaagcgtg gcgctttctc 2760
   atagctcacg ctgtaggtat ctcagttcgg tgtaggtcgt tcgctccaag ctgggctgtg 2820
   tgcacgaacc ccccgttcag cccgaccgct gcgccttatc cggtaactat cgtcttgagt 2880
   ccaacccggt aagacacgac ttatcgccac tggcagcagc cactggtaac aggattagca 2940
   gagcgaggta tgtaggcggt gctacagagt tcttgaagtg gtggcctaac tacggctaca 3000
   ctagaaggac agtatttggt atctgcgctc tgctgaagcc agttaccttc ggaaaaagag 3060
   ttggtagctc ttgatccggc aaacaaacca ccgctggtag cggtggtttt tttgtttgca 3120
   agcagcagat tacgcgcaga aaaaaaggat ctcaagaaga tcctttgatc ttttctacgg 3180
   ggtctgacgc tcagtggaac gaaaactcac gttaagggat tttggtcatg catgatatat 3240
   ctcccaattt gtgtagggct tattatgcac gcttaaaaat aataaaagca gacttgacct 3300
   gatagtttgg ctgtgagcaa ttatgtgctt agtgcatcta atcgcttgag ttaacgccgg 3360
   cgaagcggcg tcggcttgaa cgaatttcta gctagacatt atttgccgac taccttggtg 3420
   atctcgcctt tcacgtagtg gacaaattct tccaactgat ctgcgcgcga ggccaagcga 3480
   tcttcttctt gtccaagata agcctgtcta gcttcaagta tgacgggctg atactgggcc 3540
   ggcaggcgct ccattgccca gtcggcagcg acatccttcg gcgcgatttt gccggttact 3600
   gcgctgtacc aaatgcggga caacgtaagc actacatttc gctcatcgcc agcccagtcg 3660
   ggcggcgagt tccatagcgt taaggtttca tttagcgcct caaatagatc ctgttcagga 3720
   accggatcaa agagttcctc cgccgctgga cctaccaagg caacgctatg ttctcttgct 3780
   tttgtcagca agatagccag atcaatgtcg atcgtggctg gctcgaagat acctgcaaga 3840
   atgtcattgc gctgccattc tccaaattgc agttcgcgct tagctggata acgccacgga 3900
   atgatgtcgt cgtgcacaac aatggtgact tctacagcgc ggagaatctc gctctctcca 3960
   ggggaagccg aagtttccaa aaggtcgttg atcaaagctc gccgcgttgt ttcatcaagc 4020
   cttacggtca ccgtaaccag caaatcaata tcactgtgtg gcttcaggcc gccatccact 4080
   gcggagccgt acaaatgtac ggccagcaac gtcggttcga gatggcgctc gatgacgcca 4140
   actacctctg atagttgagt cgatacttcg gcgatcaccg cttcccccat gatgtttaac 4200
   tttgttttag ggcgactgcc ctgctgcgta acatcgttgc tgctccataa catcaaacat      4260
   cgacccacgg cgtaacgcgc ttgctgcttg gatgcccgag gcatagactg taccccaaaa      4320
   aaacatgtca taacaagaag ccatgaaaac cgccactgcg ccgttaccac cgctgcgttc      4380
   ggtcaaggtt ctggaccagt tgcgtgacgg cagttacgct acttgcatta cagcttacga      4440
   accgaacgag gcttatgtcc actgggttcg tgcccgaatt gatcacaggc agcaacgctc      4500
   tgtcatcgtt acaatcaaca tgctaccctc cgcgagatca tccgtgtttc aaacccggca      4560
   gcttagttgc cgttcttccg aatagcatcg gtaacatgag caaagtctgc cgccttacaa      4620
   cggctctccc gctgacgccg tcccggactg atgggctgcc tgtatcgagt ggtgattttg      4680
   tgccgagctg ccggtcgggg agctgttggc tggctggtgg caggatatat tgtggtgtaa      4740
   acaaattgac gcttagacaa cttaataaca cattgcggac gtttttaatg tactgaatta      4800
   acgccgaatt gaattcctcg agtacgtagg atccatttaa attctagagg cgcgccgata      4860
   tcctctctta aggtagcgag ctcttaatta atagggataa cagggtaatg cggccgcaag      4920
   ctaaaacgac ggccagtgaa ttatcaactt tgtatagaaa agttgctctg ccgacagtgg      4980
   tcccaaagat ggacccccac ccacgaggag catcgtggaa aaagaagacg ttccaaccac      5040
   gtcttcaaag caagtggatt gatgtgataa catggtggag cacgacactc tcgtctactc      5100
   caagaatatc aaagatacag tctcagaaga ccaaagggct attgagactt ttcaacaaag      5160
   ggtaatatcg ggaaacctcc tcggattcca ttgcccagct atctgtcact tcatcaaaag      5220
   gacagtagaa aaggaaggtg gcacctacaa atgccatcat tgcgataaag gaaaggctat      5280
   cgttcaagat gcctctgccg acagtggtcc caaagatgga cccccaccca cgaggagcat      5340
   cgtggaaaaa gaagacgttc caaccacgtc ttcaaagcaa gtggattgat gtgatatctc      5400
   cactgacgta agggatgacg cacaatccca ctatccttcg caagaccctt cctctatata      5460
   aggaagttca tttcatttgg agaggacacg ctgcaagttt gtacaaaaaa gcaggctaga      5520
   ccgggggcca atgagatatg aaaaagcctg aactcaccgc gacgtctgtc gagaagtttc      5580
   tgatcgaaaa gttcgacagc gtctccgacc tgatgcagct ctcggagggc gaagaatctc      5640
   gtgctttcag cttcgatgta ggagggcgtg gatatgtcct gcgggtaaat agctgcgccg      5700
   atggtttcta caaagatcgt tatgtttatc ggcactttgc atcggccgcg ctcccgattc      5760
   cggaagtgct tgacattggg gaattcagcg agagcctgac ctattgcatc tcccgccgtg      5820
   cacagggtgt cacgttgcaa gacctgcctg aaaccgaact gcccgctgtt ctgcagccgg      5880
   tcgcggaggc catggatgcg atcgctgcgg ccgatcttag ccagacgagc gggttcggcc      5940
   cattcggacc gcaaggaatc ggtcaataca ctacatggcg tgatttcata tgcgcgattg      6000
   ctgatcccca tgtgtatcac tggcaaactg tgatggacga caccgtcagt gcgtccgtcg      6060
   cgcaggctct cgatgagctg atgctttggg ccgaggactg ccccgaagtc cggcacctcg 6120
   tgcacgcgga tttcggctcc aacaatgtcc tgacggacaa tggccgcata acagcggtca 6180
   ttgactggag cgaggcgatg ttcggggatt cccaatacga ggtcgccaac atcttcttct 6240
   ggaggccgtg gttggcttgt atggagcagc agacgcgcta cttcgagcgg aggcatccgg 6300
   agcttgcagg atcgccgcgg ctccgggcgt atatgctccg cattggtctt gaccaactct 6360
   atcagagctt ggttgacggc aatttcgatg atgcagcttg ggcgcagggt cgatgcgacg 6420
   caatcgtccg atccggagcc gggactgtcg ggcgtacaca aatcgcccgc agaagcgcgg 6480
   ccggccgtct ggaccgatgg ctgtgtagaa gtactcgccg atagtggaaa ccgacgcccc 6540
   agcactcgtc cggacccagc tttcttgtac aaagtggagt ccgcaaaaat caccagtctc 6600
   tctctacaaa tctatctctc tctatttttc tccagaataa tgtgtgagta gttcccagat 6660
   aagggaatta gggttcttat agggtttcgc tcatgtgttg agcatataag aaacccttag 6720
   tatgtatttg tatttgtaaa atacttctat caataaaatt tctaattcct aaaaccaaaa 6780
   tccagtgacc tcaactttat tatacatagt tgataattca ctggccgtgc ttattccatg 6840
   gctgcaggtc gacgaattca ccggttaggg ataacagggt aatcgctacc ttaggaccgt 6900
   tatagttacg gccagtgcca ttaccctgtt atccctaacc ggtgacaact ttgtatagaa 6960
   aagttggttt gtgtcttcta gattaatcct ccaaactttt gattaaccaa aaaaattatc 7020
   aaactaacat gttctccttt tttctttaga aattctaacg aatttatctt tatactgatt 7080
   tgaatatact taatttggtc atttggatgc cctttacaac ctccttacca aactcactat 7140
   ggcaaatata tactattttc cattgtaaca taaatgtcca taatttgaat taaattcgtt 7200
   gcagtacgaa accatccaac tttgtccaaa aacaaaatcc ttataactat ttactttaat 7260
   gtaaatatat cctctacttt tgtttttaca accctagctc aaacaaattt attatttgcg 7320
   ataaaaaatc atatcgaaca aactcgatga tttttttttt cttacgttat taatgaaact 7380
   aaaatataga aaaaaacaag atgaaccaaa ttttcaccta tctaactact taaatataat 7440
   atgattaaat ttggtaaagt ttgaaaagtt tctttagaaa tgtgaaatat tgatcacagt 7500
   ttctattgct aaaatcacca acaaaacgca tgtcgccatt cataattatg gtttcacacc 7560
   tacaactagg ctaataagta aataagtaga caactagact caggtttgaa aaaaccataa 7620
   aagccatata gcgttttctc attgaaactg cgaacacgat cgtgtgaatg ttgcagtttc 7680
   tagttttgat acaaacaaac aaaaacacaa tttaatctta gattaaaaag aaaaaagaga 7740
   acggagccca ctagccactc cttcaaacgt gtcttaccaa ctctcttcta gaaacaaatt 7800
   aggcttcacc ttcctcttcc aacctctctc tctctctctc tctctttttc tcaaaccatc 7860
   tctccataaa gccctaattt cttcatcaca agaatcagaa gaagaaacaa gtttgtacaa 7920
   aaaagcaggc ttactgcaaa aaacttatgg acctgcatct aattttcggt ccaacttgca 7980
   caggaaagac gacgaccgcg atagctcttg cccagcagac agggcttcca gtcctttcgc 8040
   ttgatcgggt ccaatgctgt cctcaactat caaccggaag cggacgacca acagtggaag 8100
   aactgaaagg aacgacgcgt ctctaccttg atgatcggcc tctggtggag ggtatcatcg 8160
   cagccaagca agctcatcat aggctgatcg aggaggtgta taatcatgag gccaacggcg 8220
   ggcttattct tgagggagga tccacctcgt tgctcaactg catggcgcga aacagctatt 8280
   ggagtgcaga ttttcgttgg catattattc gccacaagtt acccgaccaa gagaccttca 8340
   tgaaagcggc caaggccaga gttaagcaga tgttgcaccc cgctgcaggc cattctatta 8400
   ttcaagagtt ggtttatctt tggaatgaac ctcggctgag gcccattctg aaagagatcg 8460
   atggatatcg atatgccatg ttgtttgcta gccagaacca gatcacggca gatatgctat 8520
   tgcagcttga cgcaaatatg gaaggtaagt tgattaatgg gatcgctcag gagtatttca 8580
   tccatgcgcg ccaacaggaa cagaaattcc cccaagttaa cgcagccgct ttcgacggat 8640
   tcgaaggtca tccgttcgga atgtattagg tacccagctt tcttgtacaa agtgggatcg 8700
   ttcaaacatt tggcaataaa gtttcttaag attgaatcct gttgccggtc ttgcgatgat 8760
   tatcatataa tttctgttga attacgttaa gcatgtaata attaacatgt aatgcatgac 8820
   gttatttatg agatgggttt ttatgattag agtcccgcaa ttatacattt aatacgcgat 8880
   agaaaacaaa atatagcgcg caaactagga taaattatcg cgcgcggtgt catctatgtt 8940
   actagatcca actttattat acatagttga ttcgtcgacc tgcagtcgct accttaggac 9000
   cgttatagtt atggcaaaca gctattatgg gtattatggg tggttcttta tgcggacact 9060
   gacggcttta tgcctgcagg tcgcgagcga tcgcggtacc gcccgggcgt cgacaggcct 9120
   aagcttagct tgagcttgga tcagattgtc gtttcccgcc ttcagtttaa actatcagtg 9180
   tttgacagga tatattggcg ggtaaaccta agagaaaaga gcgtttatta gaataacgga 9240
   tatttaaaag ggcgtgaaaa ggtttatccg ttcgtccatt tgtatgtgca tgccaaccac 9300
   agggttcccc tcgggatcaa agtactttga tccaacccct ccgctgctat agtgcagtcg 9360
   gcttctgacg ttcagtgcag ccgtcttctg aaaacgacat gtcgcacaag tcctaagtta 9420
   cgcgacaggc tgccgccctg cccttttcct ggcgttttct tgtcgcgtgt tttagtcgca 9480
   taaagtagaa tacttgcgac tagaaccgga gacattacgc catgaacaag agcgccgccg 9540
   ctggcctgct gggctatgcc cgcgtcagca ccgacgacca ggacttgacc aaccaacggg 9600
   ccgaactgca cgcggccggc tgcaccaagc tgttttccga gaagatcacc ggcaccaggc 9660
   gcgaccgccc ggagctggcc aggatgcttg accacctacg ccctggcgac gttgtgacag 9720
   tgaccaggct agaccgcctg gcccgcagca cccgcgacct actggacatt gccgagcgca 9780
   tccaggaggc cggcgcgggc ctgcgtagcc tggcagagcc gtgggccgac accaccacgc 9840
   cggccggccg catggtgttg accgtgttcg ccggcattgc cgagttcgag cgttccctaa 9900
   tcatcgaccg cacccggagc gggcgcgagg ccgccaaggc ccgaggcgtg aagtttggcc 9960
   cccgccctac cctcaccccg gcacagatcg cgcacgcccg cgagctgatc gaccaggaag 10020
   gccgcaccgt gaaagaggcg gctgcactgc ttggcgtgca tcgctcgacc ctgtaccgcg 10080
   cacttgagcg cagcgaggaa gtgacgccca ccgaggccag gcggcgcggt gccttccgtg 10140
   aggacgcatt gaccgaggcc gacgccctgg cggccgccga gaatgaacgc caagaggaac 10200
   aagcatgaaa ccgcaccagg acggccagga cgaaccgttt ttcattaccg aagagatcga 10260
   ggcggagatg atcgcggccg ggtacgtgtt cgagccgccc gcgcacgtct caaccgtgcg 10320
   gctgcatgaa atcctggccg gtttgtctga tgccaagctg gcggcctggc cggccagctt 10380
   ggccgctgaa gaaaccgagc gccgccgtct aaaaaggtga tgtgtatttg agtaaaacag 10440
   cttgcgtcat gcggtcgctg cgtatatgat gcgatgagta aataaacaaa tacgcaaggg 10500
   gaacgcatga aggttatcgc tgtacttaac cagaaaggcg ggtcaggcaa gacgaccatc 10560
   gcaacccatc tagcccgcgc cctgcaactc gccggggccg atgttctgtt agtcgattcc 10620
   gatccccagg gcagtgcccg cgattgggcg gccgtgcggg aagatcaacc gctaaccgtt 10680
   gtcggcatcg accgcccgac gattgaccgc gacgtgaagg ccatcggccg gcgcgacttc 10740
   gtagtgatcg acggagcgcc ccaggcggcg gacttggctg tgtccgcgat caaggcagcc 10800
   gacttcgtgc tgattccggt gcagccaagc ccttacgaca tatgggccac cgccgacctg 10860
   gtggagctgg ttaagcagcg cattgaggtc acggatggaa ggctacaagc ggcctttgtc 10920
   gtgtcgcggg cgatcaaagg cacgcgcatc ggcggtgagg ttgccgaggc gctggccggg 10980
   tacgagctgc ccattcttga 11000
<210> 14
   <211> 10682
   <212> DNA
   <213> Artificial
<220>
   <223> Synthetic construct
<400> 14
   gtcccgtatc acgcagcgcg tgagctaccc aggcactgcc gccgccggca caaccgttct      60
   tgaatcagaa cccgagggcg acgctgcccg cgaggtccag gcgctggccg ctgaaattaa     120
   atcaaaactc atttgagtta atgaggtaaa gagaaaatga gcaaaagcac aaacacgcta     180
   agtgccggcc gtccgagcgc acgcagcagc aaggctgcaa cgttggccag cctggcagac     240
   acgccagcca tgaagcgggt caactttcag ttgccggcgg aggatcacac caagctgaag     300
   atgtacgcgg tacgccaagg caagaccatt accgagctgc tatctgaata catcgcgcag     360
   ctaccagagt aaatgagcaa atgaataaat gagtagatga attttagcgg ctaaaggagg     420
   cggcatggaa aatcaagaac aaccaggcac cgacgccgtg gaatgcccca tgtgtggagg     480
   aacgggcggt tggccaggcg taagcggctg ggttgtctgc cggccctgca atggcactgg     540
   aacccccaag cccgaggaat cggcgtgacg gtcgcaaacc atccggcccg gtacaaatcg      600
   gcgcggcgct gggtgatgac ctggtggaga agttgaaggc cgcgcaggcc gcccagcggc     660
   aacgcatcga ggcagaagca cgccccggtg aatcgtggca agcggccgct gatcgaatcc     720
   gcaaagaatc ccggcaaccg ccggcagccg gtgcgccgtc gattaggaag ccgcccaagg     780
   gcgacgagca accagatttt ttcgttccga tgctctatga cgtgggcacc cgcgatagtc     840
   gcagcatcat ggacgtggcc gttttccgtc tgtcgaagcg tgaccgacga gctggcgagg     900
   tgatccgcta cgagcttcca gacgggcacg tagaggtttc cgcagggccg gccggcatgg     960
   ccagtgtgtg ggattacgac ctggtactga tggcggtttc ccatctaacc gaatccatga    1020
   accgataccg ggaagggaag ggagacaagc ccggccgcgt gttccgtcca cacgttgcgg    1080
   acgtactcaa gttctgccgg cgagccgatg gcggaaagca gaaagacgac ctggtagaaa    1140
   cctgcattcg gttaaacacc acgcacgttg ccatgcagcg tacgaagaag gccaagaacg    1200
   gccgcctggt gacggtatcc gagggtgaag ccttgattag ccgctacaag atcgtaaaga    1260
   gcgaaaccgg gcggccggag tacatcgaga tcgagctagc tgattggatg taccgcgaga    1320
   tcacagaagg caagaacccg gacgtgctga cggttcaccc cgattacttt ttgatcgatc    1380
   ccggcatcgg ccgttttctc taccgcctgg cacgccgcgc cgcaggcaag gcagaagcca    1440
   gatggttgtt caagacgatc tacgaacgca gtggcagcgc cggagagttc aagaagttct    1500
   gtttcaccgt gcgcaagctg atcgggtcaa atgacctgcc ggagtacgat ttgaaggagg    1560
   aggcggggca ggctggcccg atcctagtca tgcgctaccg caacctgatc gagggcgaag    1620
   catccgccgg ttcctaatgt acggagcaga tgctagggca aattgcccta gcaggggaaa    1680
   aaggtcgaaa aggtctcttt cctgtggata gcacgtacat tgggaaccca aagccgtaca    1740
   ttgggaaccg gaacccgtac attgggaacc caaagccgta cattgggaac cggtcacaca    1800
   tgtaagtgac tgatataaaa gagaaaaaag gcgatttttc cgcctaaaac tctttaaaac    1860
   ttattaaaac tcttaaaacc cgcctggcct gtgcataact gtctggccag cgcacagccg    1920
   aagagctgca aaaagcgcct acccttcggt cgctgcgctc cctacgcccc gccgcttcgc    1980
   gtcggcctat cgcggccgct ggccgctcaa aaatggctgg cctacggcca ggcaatctac    2040
   cagggcgcgg acaagccgcg ccgtcgccac tcgaccgccg gcgcccacat caaggcaccc    2100
   tgcctcgcgc gtttcggtga tgacggtgaa aacctctgac acatgcagct cccggagacg    2160
   gtcacagctt gtctgtaagc ggatgccggg agcagacaag cccgtcaggg cgcgtcagcg    2220
   ggtgttggcg ggtgtcgggg cgcagccatg acccagtcac gtagcgatag cggagtgtat    2280
   actggcttaa ctatgcggca tcagagcaga ttgtactgag agtgcaccat atgcggtgtg 2340
   aaataccgca cagatgcgta aggagaaaat accgcatcag gcgctcttcc gcttcctcgc 2400
   tcactgactc gctgcgctcg gtcgttcggc tgcggcgagc ggtatcagct cactcaaagg 2460
   cggtaatacg gttatccaca gaatcagggg ataacgcagg aaagaacatg tgagcaaaag 2520
   gccagcaaaa ggccaggaac cgtaaaaagg ccgcgttgct ggcgtttttc cataggctcc 2580
   gcccccctga cgagcatcac aaaaatcgac gctcaagtca gaggtggcga aacccgacag 2640
   gactataaag ataccaggcg tttccccctg gaagctccct cgtgcgctct cctgttccga 2700
   ccctgccgct taccggatac ctgtccgcct ttctcccttc gggaagcgtg gcgctttctc 2760
   atagctcacg ctgtaggtat ctcagttcgg tgtaggtcgt tcgctccaag ctgggctgtg 2820
   tgcacgaacc ccccgttcag cccgaccgct gcgccttatc cggtaactat cgtcttgagt 2880
   ccaacccggt aagacacgac ttatcgccac tggcagcagc cactggtaac aggattagca 2940
   gagcgaggta tgtaggcggt gctacagagt tcttgaagtg gtggcctaac tacggctaca 3000
   ctagaaggac agtatttggt atctgcgctc tgctgaagcc agttaccttc ggaaaaagag 3060
   ttggtagctc ttgatccggc aaacaaacca ccgctggtag cggtggtttt tttgtttgca 3120
   agcagcagat tacgcgcaga aaaaaaggat ctcaagaaga tcctttgatc ttttctacgg 3180
   ggtctgacgc tcagtggaac gaaaactcac gttaagggat tttggtcatg catgatatat 3240
   ctcccaattt gtgtagggct tattatgcac gcttaaaaat aataaaagca gacttgacct 3300
   gatagtttgg ctgtgagcaa ttatgtgctt agtgcatcta atcgcttgag ttaacgccgg 3360
   cgaagcggcg tcggcttgaa cgaatttcta gctagacatt atttgccgac taccttggtg 3420
   atctcgcctt tcacgtagtg gacaaattct tccaactgat ctgcgcgcga ggccaagcga 3480
   tcttcttctt gtccaagata agcctgtcta gcttcaagta tgacgggctg atactgggcc 3540
   ggcaggcgct ccattgccca gtcggcagcg acatccttcg gcgcgatttt gccggttact 3600
   gcgctgtacc aaatgcggga caacgtaagc actacatttc gctcatcgcc agcccagtcg 3660
   ggcggcgagt tccatagcgt taaggtttca tttagcgcct caaatagatc ctgttcagga 3720
   accggatcaa agagttcctc cgccgctgga cctaccaagg caacgctatg ttctcttgct 3780
   tttgtcagca agatagccag atcaatgtcg atcgtggctg gctcgaagat acctgcaaga 3840
   atgtcattgc gctgccattc tccaaattgc agttcgcgct tagctggata acgccacgga 3900
   atgatgtcgt cgtgcacaac aatggtgact tctacagcgc ggagaatctc gctctctcca 3960
   ggggaagccg aagtttccaa aaggtcgttg atcaaagctc gccgcgttgt ttcatcaagc 4020
   cttacggtca ccgtaaccag caaatcaata tcactgtgtg gcttcaggcc gccatccact 4080
   gcggagccgt acaaatgtac ggccagcaac gtcggttcga gatggcgctc gatgacgcca 4140
   actacctctg atagttgagt cgatacttcg gcgatcaccg cttcccccat gatgtttaac 4200
   tttgttttag ggcgactgcc ctgctgcgta acatcgttgc tgctccataa catcaaacat 4260
   cgacccacgg cgtaacgcgc ttgctgcttg gatgcccgag gcatagactg taccccaaaa 4320
   aaacatgtca taacaagaag ccatgaaaac cgccactgcg ccgttaccac cgctgcgttc 4380
   ggtcaaggtt ctggaccagt tgcgtgacgg cagttacgct acttgcatta cagcttacga 4440
   accgaacgag gcttatgtcc actgggttcg tgcccgaatt gatcacaggc agcaacgctc 4500
   tgtcatcgtt acaatcaaca tgctaccctc cgcgagatca tccgtgtttc aaacccggca 4560
   gcttagttgc cgttcttccg aatagcatcg gtaacatgag caaagtctgc cgccttacaa 4620
   cggctctccc gctgacgccg tcccggactg atgggctgcc tgtatcgagt ggtgattttg 4680
   tgccgagctg ccggtcgggg agctgttggc tggctggtgg caggatatat tgtggtgtaa 4740
   acaaattgac gcttagacaa cttaataaca cattgcggac gtttttaatg tactgaatta 4800
   acgccgaatt gaattcctcg agtacgtagg atccatttaa attctagagg cgcgccgata 4860
   tcctctctta aggtagcgag ctcttaatta atagggataa cagggtaatg cggccgcaag 4920
   ctaaaacgac ggccagtgaa ttatcaactt tgtatagaaa agttgctctg ccgacagtgg 4980
   tcccaaagat ggacccccac ccacgaggag catcgtggaa aaagaagacg ttccaaccac 5040
   gtcttcaaag caagtggatt gatgtgataa catggtggag cacgacactc tcgtctactc 5100
   caagaatatc aaagatacag tctcagaaga ccaaagggct attgagactt ttcaacaaag 5160
   ggtaatatcg ggaaacctcc tcggattcca ttgcccagct atctgtcact tcatcaaaag 5220
   gacagtagaa aaggaaggtg gcacctacaa atgccatcat tgcgataaag gaaaggctat 5280
   cgttcaagat gcctctgccg acagtggtcc caaagatgga cccccaccca cgaggagcat 5340
   cgtggaaaaa gaagacgttc caaccacgtc ttcaaagcaa gtggattgat gtgatatctc 5400
   cactgacgta agggatgacg cacaatccca ctatccttcg caagaccctt cctctatata 5460
   aggaagttca tttcatttgg agaggacacg ctgcaagttt gtacaaaaaa gcaggctaga 5520
   ccggggggca atgagatatg aaaaagcctg aactcaccgc gacgtctgtc gagaagtttc 5580
   tgatcgaaaa gttcgacagc gtctccgacc tgatgcagct ctcggagggc gaagaatctc 5640
   gtgctttcag cttcgatgta ggagggcgtg gatatgtcct gcgggtaaat agctgcgccg 5700
   atggtttcta caaagatcgt tatgtttatc ggcactttgc atcggccgcg ctcccgattc 5760
   cggaagtgct tgacattggg gaattcagcg agagcctgac ctattgcatc tcccgccgtg 5820
   cacagggtgt cacgttgcaa gacctgcctg aaaccgaact gcccgctgtt ctgcagccgg 5880
   tcgcggaggc catggatgcg atcgctgcgg ccgatcttag ccagacgagc gggttcggcc 5940
   cattcggacc gcaaggaatc ggtcaataca ctacatggcg tgatttcata tgcgcgattg 6000
   ctgatcccca tgtgtatcac tggcaaactg tgatggacga caccgtcagt gcgtccgtcg 6060
   cgcaggctct cgatgagctg atgctttggg ccgaggactg ccccgaagtc cggcacctcg 6120
   tgcacgcgga tttcggctcc aacaatgtcc tgacggacaa tggccgcata acagcggtca 6180
   ttgactggag cgaggcgatg ttcggggatt cccaatacga ggtcgccaac atcttcttct 6240
   ggaggccgtg gttggcttgt atggagcagc agacgcgcta cttcgagcgg aggcatccgg 6300
   agcttgcagg atcgccgcgg ctccgggcgt atatgctccg cattggtctt gaccaactct 6360
   atcagagctt ggttgacggc aatttcgatg atgcagcttg ggcgcagggt cgatgcgacg 6420
   caatcgtccg atccggagcc gggactgtcg ggcgtacaca aatcgcccgc agaagcgcgg 6480
   ccggccgtct ggaccgatgg ctgtgtagaa gtactcgccg atagtggaaa ccgacgcccc 6540
   agcactcgtc cggacccagc tttcttgtac aaagtggagt ccgcaaaaat caccagtctc 6600
   tctctacaaa tctatctctc tctatttttc tccagaataa tgtgtgagta gttcccagat 6660
   aagggaatta gggttcttat agggtttcgc tcatgtgttg agcatataag aaacccttag 6720
   tatgtatttg tatttgtaaa atacttctat caataaaatt tctaattcct aaaaccaaaa 6780
   tccagtgacc tcaactttat tatacatagt tgataattca ctggccgtgc ttattccatg 6840
   gctgcaggtc gacgaattca ccggttaggg ataacagggt aataactata acggtcctaa 6900
   ggtagcgagc ggccgcaagc taaaacgacg gccagtgaat tatcaacttt gtatagaaaa 6960
   gttggtttgt gtcttctaga ttaatcctcc aaacttttga ttaaccaaaa aaattatcaa 7020
   actaacatgt tctccttttt tctttagaaa ttctaacgaa tttatcttta tactgatttg 7080
   aatatactta atttggtcat ttggatgccc tttacaacct ccttaccaaa atattgatca 7140
   cagtttctat tgctaaaatc accaacaaaa cgcatgtcgc cattcataat tatggtttca 7200
   cacctacaac taggctaata agtaaataag tagacaacta gactcaggtt tgaaaaaacc 7260
   ataaaagcca tatagcgttt tctcattgaa actgcgaaca cgatcgtgtg aatgttgcag 7320
   tttctagttt tgatacaaac aaacaaaaac acaatttaat cttagattaa aaagaaaaaa 7380
   gagaacggag cccactagcc actccttcaa acgtgtctta ccaactctct tctagaaaca 7440
   aattaggctt caccttcctc ttccaacctc tctctctctc tctctctctt tttctcaaac 7500
   catctctcca taaagcccta atttcttcat cacaagaatc agaagaagaa acaagtttgt 7560
   acaaaaaagc aggcttactg caaaaaactt atggacctgc atctaatttt cggtccaact 7620
   tgcacaggaa agacgacgac cgcgatagct cttgcccagc agacagggct tccagtcctt 7680
   tcgcttgatc gggtccaatg ctgtcctcaa ctatcaaccg gaagcggacg accaacagtg 7740
   gaagaactga aaggaacgac gcgtctctac cttgatgatc ggcctctggt ggagggtatc 7800
   atcgcagcca agcaagctca tcataggctg atcgaggagg tgtataatca tgaggccaac 7860
   ggcgggctta ttcttgaggg aggatccacc tcgttgctca actgcatggc gcgaaacagc 7920
   tattggagtg cagattttcg ttggcatatt attcgccaca agttacccga ccaagagacc 7980
   ttcatgaaag cggccaaggc cagagttaag cagatgttgc accccgctgc aggccattct 8040
   attattcaag agttggttta tctttggaat gaacctcggc tgaggcccat tctgaaagag 8100
   atcgatggat atcgatatgc catgttgttt gctagccaga accagatcac ggcagatatg 8160
   ctattgcagc ttgacgcaaa tatggaaggt aagttgatta atgggatcgc tcaggagtat 8220
   ttcatccatg cgcgccaaca ggaacagaaa ttcccccaag ttaacgcagc cgctttcgac 8280
   ggattcgaag gtcatccgtt cggaatgtat taggtaccca gctttcttgt acaaagtggg 8340
   atcgttcaaa catttggcaa taaagtttct taagattgaa tcctgttgcc ggtcttgcga 8400
   tgattatcat ataatttctg ttgaattacg ttaagcatgt aataattaac atgtaatgca 8460
   tgacgttatt tatgagatgg gtttttatga ttagagtccc gcaattatac atttaatacg 8520
   cgatagaaaa caaaatatag cgcgcaaact aggataaatt atcgcgcgcg gtgtcatcta 8580
   tgttactaga tccaacttta ttatacatag ttgataattc actggccgtc gcttattcca 8640
   tggctgcagg tcgacgaatt caccggttaa ctataacggt cctaaggtag cgatggcaaa 8700
   cagctattat gggtattatg ggtggttctt tatgcggaca ctgacggctt tatgcctgca 8760
   ggtcgcgagc gatcgcggta ccgcccgggc gtcgacaggc ctaagcttag cttgagcttg 8820
   gatcagattg tcgtttcccg ccttcagttt aaactatcag tgtttgacag gatatattgg 8880
   cgggtaaacc taagagaaaa gagcgtttat tagaataacg gatatttaaa agggcgtgaa 8940
   aaggtttatc cgttcgtcca tttgtatgtg catgccaacc acagggttcc cctcgggatc 9000
   aaagtacttt gatccaaccc ctccgctgct atagtgcagt cggcttctga cgttcagtgc 9060
   agccgtcttc tgaaaacgac atgtcgcaca agtcctaagt tacgcgacag gctgccgccc 9120
   tgcccttttc ctggcgtttt cttgtcgcgt gttttagtcg cataaagtag aatacttgcg 9180
   actagaaccg gagacattac gccatgaaca agagcgccgc cgctggcctg ctgggctatg 9240
   cccgcgtcag caccgacgac caggacttga ccaaccaacg ggccgaactg cacgcggccg 9300
   gctgcaccaa gctgttttcc gagaagatca ccggcaccag gcgcgaccgc ccggagctgg 9360
   ccaggatgct tgaccaccta cgccctggcg acgttgtgac agtgaccagg ctagaccgcc 9420
   tggcccgcag cacccgcgac ctactggaca ttgccgagcg catccaggag gccggcgcgg 9480
   gcctgcgtag cctggcagag ccgtgggccg acaccaccac gccggccggc cgcatggtgt 9540
   tgaccgtgtt cgccggcatt gccgagttcg agcgttccct aatcatcgac cgcacccgga 9600
   gcgggcgcga ggccgccaag gcccgaggcg tgaagtttgg cccccgccct accctcaccc 9660
   cggcacagat cgcgcacgcc cgcgagctga tcgaccagga aggccgcacc gtgaaagagg 9720
   cggctgcact gcttggcgtg catcgctcga ccctgtaccg cgcacttgag cgcagcgagg 9780
   aagtgacgcc caccgaggcc aggcggcgcg gtgccttccg tgaggacgca ttgaccgagg 9840
   ccgacgccct ggcggccgcc gagaatgaac gccaagagga acaagcatga aaccgcacca 9900
   ggacggccag gacgaaccgt ttttcattac cgaagagatc gaggcggaga tgatcgcggc 9960
   cgggtacgtg ttcgagccgc ccgcgcacgt ctcaaccgtg cggctgcatg aaatcctggc 10020
   cggtttgtct gatgccaagc tggcggcctg gccggccagc ttggccgctg aagaaaccga 10080
   gcgccgccgt ctaaaaaggt gatgtgtatt tgagtaaaac agcttgcgtc atgcggtcgc 10140
   tgcgtatatg atgcgatgag taaataaaca aatacgcaag gggaacgcat gaaggttatc 10200
   gctgtactta accagaaagg cgggtcaggc aagacgacca tcgcaaccca tctagcccgc 10260
   gccctgcaac tcgccggggc cgatgttctg ttagtcgatt ccgatcccca gggcagtgcc 10320
   cgcgattggg cggccgtgcg ggaagatcaa ccgctaaccg ttgtcggcat cgaccgcccg 10380
   acgattgacc gcgacgtgaa ggccatcggc cggcgcgact tcgtagtgat cgacggagcg 10440
   ccccaggcgg cggacttggc tgtgtccgcg atcaaggcag ccgacttcgt gctgattccg 10500
   gtgcagccaa gcccttacga catatgggcc accgccgacc tggtggagct ggttaagcag 10560
   cgcattgagg tcacggatgg aaggctacaa gcggcctttg tcgtgtcgcg ggcgatcaaa 10620
   ggcacgcgca tcggcggtga ggttgccgag gcgctggccg ggtacgagct gcccattctt 10680
   ga 10682
<210> 15
   <211> 7862
   <212> DNA
   <213> Artificial
<220>
   <223> Synthetic construct
<400> 15
   ttatacatag ttgataattc actggccgtc gtgggggatc cactagttct agagcggccg     60
   ccaccgcggt ggagctccag cttttgttcc ctttagtgag ggttaatttc gagcttggcg    120
   taatcatggt catagctgtt tcctgtgtga aattgttatc cgctcacaat tccacacaac    180
   atacgagccg gaagcataaa gtgtaaagcc tggggtgcct aatgagtgag ctaactcaca    240
   ttaattgcgt tgcgctcact gcccgctttc cagtcgggaa acctgtcgtg ccagctgcat    300
   taatgaatcg gccaacgcgc ggggagaggc ggtttgcgta ttgggcgctc ttccgcttcc    360
   tcgctcactg actcgctgcg ctcggtcgtt cggctgcggc gagcggtatc agctcactca    420
   aaggcggtaa tacggttatc cacagaatca ggggataacg caggaaagaa catgtgagca    480
   aaaggccagc aaaaggccag gaaccgtaaa aaggccgcgt tgctggcgtt tttccatagg    540
   ctccgccccc ctgacgagca tcacaaaaat cgacgctcaa gtcagaggtg gcgaaacccg    600
   acaggactat aaagatacca ggcgtttccc cctggaagct ccctcgtgcg ctctcctgtt    660
   ccgaccctgc cgcttaccgg atacctgtcc gcctttctcc cttcgggaag cgtggcgctt    720
   tctcatagct cacgctgtag gtatctcagt tcggtgtagg tcgttcgctc caagctgggc 780
   tgtgtgcacg aaccccccgt tcagcccgac cgctgcgcct tatccggtaa ctatcgtctt 840
   gagtccaacc cggtaagaca cgacttatcg ccactggcag cagccactgg taacaggatt 900
   agcagagcga ggtatgtagg cggtgctaca gagttcttga agtggtggcc taactacggc 960
   tacactagaa ggacagtatt tggtatctgc gctctgctga agccagttac cttcggaaaa 1020
   agagttggta gctcttgatc cggcaaacaa accaccgctg gtagcggtgg tttttttgtt 1080
   tgcaagcagc agattacgcg cagaaaaaaa ggatctcaag aagatccttt gatcttttct 1140
   acggggtctg acgctcagtg gaacgaaaac tcacgttaag ggattttggt catgagatta 1200
   tcaaaaagga tcttcaccta gatcctttta aattaaaaat gaagttttaa atcaatctaa 1260
   agtatatatg agtaaacttg gtctgacagt taccaatgct taatcagtga ggcacctatc 1320
   tcagcgatct gtctatttcg ttcatccata gttgcctgac tccccgtcgt gtagataact 1380
   acgatacggg agggcttacc atctggcccc agtgctgcaa tgataccgcg agacccacgc 1440
   tcaccggctc cagatttatc agcaataaac cagccagccg gaagggccga gcgcagaagt 1500
   ggtcctgcaa ctttatccgc ctccatccag tctattaatt gttgccggga agctagagta 1560
   agtagttcgc cagttaatag tttgcgcaac gttgttgcca ttgctacagg catcgtggtg 1620
   tcacgctcgt cgtttggtat ggcttcattc agctccggtt cccaacgatc aaggcgagtt 1680
   acatgatccc ccatgttgtg caaaaaagcg gttagctcct tcggtcctcc gatcgttgtc 1740
   agaagtaagt tggccgcagt gttatcactc atggttatgg cagcactgca taattctctt 1800
   actgtcatgc catccgtaag atgcttttct gtgactggtg agtactcaac caagtcattc 1860
   tgagaatagt gtatgcggcg accgagttgc tcttgcccgg cgtcaatacg ggataatacc 1920
   gcgccacata gcagaacttt aaaagtgctc atcattggaa aacgttcttc ggggcgaaaa 1980
   ctctcaagga tcttaccgct gttgagatcc agttcgatgt aacccactcg tgcacccaac 2040
   tgatcttcag catcttttac tttcaccagc gtttctgggt gagcaaaaac aggaaggcaa 2100
   aatgccgcaa aaaagggaat aagggcgaca cggaaatgtt gaatactcat actcttcctt 2160
   tttcaatatt attgaagcat ttatcagggt tattgtctca tgagcggata catatttgaa 2220
   tgtatttaga aaaataaaca aataggggtt ccgcgcacat ttccccgaaa agtgccacct 2280
   gacgcgccct gtagcggcgc attaagcgcg gcgggtgtgg tggttacgcg cagcgtgacc 2340
   gctacacttg ccagcgccct agcgcccgct cctttcgctt tcttcccttc ctttctcgcc 2400
   acgttcgccg gctttccccg tcaagctcta aatcgggggc tccctttagg gttccgattt 2460
   agtgctttac ggcacctcga ccccaaaaaa cttgattagg gtgatggttc acgtagtggg 2520
   ccatcgccct gatagacggt ttttcgccct ttgacgttgg agtccacgtt ctttaatagt 2580
   ggactcttgt tccaaactgg aacaacactc aaccctatct cggtctattc ttttgattta     2640
   taagggattt tgccgatttc ggcctattgg ttaaaaaatg agctgattta acaaaaattt     2700
   aacgcgaatt ttaacaaaat attaacgctt acaatttcca ttcgccattc aggctgcgca     2760
   actgttggga agggcgatcg gtgcgggcct cttcgctatt acgccagctg gcgaaagggg     2820
   gatgtgctgc aaggcgatta agttgggtaa cgccagggtt ttcccagtca cgacgttgta     2880
   aaacgacggc cagtgaattg taatacgact cactataggg cgaattgggt accgggcccc     2940
   ccctcgaggt cgacggtatc gataagcttg atatcgaatt ctcatgtttg acagcttatc     3000
   atcggatcta gtaacataga tgacaccgcg cgcgataatt tatcctagtt tgcgcgctat     3060
   attttgtttt ctatcgcgta ttaaatgtat aattgcggga ctctaatcat aaaaacccat     3120
   ctcataaata acgtcatgca ttacatgtta attattacat gcttaacgta attcaacaga     3180
   aattatatga taatcatcgc aagaccggca acaggattca atcttaagaa actttattgc     3240
   caaatgtttg aacgatctgc aggtcgacgg atcagatctc ggtgacgggc aggaccggac     3300
   ggggcggtac cggcaggctg aagtccagct gccagaaacc cacgtcatgc cagttcccgt     3360
   gcttgaagcc ggccgcccgc agcatgccgc ggggggcata tccgagcgcc tcgtgcatgc     3420
   gcacgctcgg gtcgttgggc agcccgatga cagcgaccac gctcttgaag ccctgtgcct     3480
   ccagggactt cagcaggtgg gtgtagagcg tggagcccag tcccgtccgc tggtggcggg     3540
   gggagacgta cacggtcgac tcggccgtcc agtcgtaggc gttgcgtgcc ttccaggggc     3600
   ccgcgtaggc gatgccggcg acctcgccgt ccacctcggc gacgagccag ggatagcgct     3660
   cccgcagacg gacgaggtcg tccgtccact cctgcggttc ctgcggctcg gtacggaagt     3720
   tgaccgtgct tgtctcgatg tagtggttga cgatggtgca gaccgccggc atgtccgcct     3780
   cggtggcacg gcggatgtcg gccgggcgtc gttctgggct catggttact tcctaatcga     3840
   tggatcctct agagtcgacc tgcagaagta acaccaaaca acagggtgag catcgacaaa     3900
   agaaacagta ccaagcaaat aaatagcgta tgaaggcagg gctaaaaaaa tccacatata     3960
   gctgctgcat atgccatcat ccaagtatat caagatcaaa ataattataa aacatacttg     4020
   tttattataa tagataggta ctcaaggtta gagcatatga atagatgctg catatgccat     4080
   catgtatatg catcagtaaa acccacatca acatgtatac ctatcctaga tcgatatttc     4140
   catccatctt aaactcgtaa ctatgaagat gtatgacaca cacatacagt tccaaaatta     4200
   ataaatacac caggtagttt gaaacagtat tctactccga tctagaacga atgaacgacc     4260
   gcccaaccac accacatcat cacaaccaag cgaacaaaaa gcatctctgt atatgcatca     4320
   gtaaaacccg catcaacatg tatacctatc ctagatcgat atttccatcc atcattttca     4380
   attcgtaact atgaatatgt atggcacaca catacagatc caaaattaat aaatccacca     4440
   ggtagtttga aacagaattc tactccgatc tagaacgacc gcccaaccag accacatcat     4500
   cacaaccaag acaaaaaaaa gcatgaaaag atgacccgac aaacaagtgc acggcatata 4560
   ttgaaataaa ggaaaagggc aaaccaaacc ctatgcaacg aaacaaaaaa aatcatgaaa 4620
   tcgatcccgt ctgcggaacg gctagagcca tcccaggatt ccccaaagag aaacactggc 4680
   aagttagcaa tcagaacgtg tctgacgtac aggtcgcatc cgtgtacgaa cgctagcagc 4740
   acggatctaa cacaaacacg gatctaacac aaacatgaac agaagtagaa ctaccgggcc 4800
   ctaaccatgg accggaacgc cgatctagag aaggtagaga gggggggggg gggaggacga 4860
   gcggcgtacc ttgaagcgga ggtgccgacg ggtggatttg ggggagatct ggttgtgtgt 4920
   gtgtgcgctc cgaacaacac gaggttgggg aaagagggtg tggagggggt gtctatttat 4980
   tacggcgggc gaggaaggga aagcgaagga gcggtgggaa aggaatcccc cgtagctgcc 5040
   ggtgccgtga gaggaggagg aggccgcctg ccgtgccggc tcacgtctgc cgctccgcca 5100
   cgcaatttct ggatgccgac agcggagcaa gtccaacggt ggagcggaac tctcgagagg 5160
   ggtccagagg cagcgacaga gatgccgtgc cgtctgcttc gcttggcccg acgcgacgct 5220
   gctggttcgc tggttggtgt ccgttagact cgtcgacggc gtttaacagg ctggcattat 5280
   ctactcgaaa caagaaaaat gtttccttag tttttttaat ttcttaaagg gtatttgttt 5340
   aatttttagt cactttattt tattctattt tatatctaaa ttattaaata aaaaaactaa 5400
   aatagagttt tagttttctt aatttagagg ctaaaataga ataaaataga tgtactaaaa 5460
   aaattagtct ataaaaacca ttaaccctaa accctaaatg gatgtactaa taaaatggat 5520
   gaagtattat ataggtgaag ctatttgcaa aaaaaaagga gaacacatgc acactaaaaa 5580
   gataaaactg tagagtcctg ttgtcaaaat actcaattgt cctttagacc atgtctaact 5640
   gttcatttat atgattctct aaaacactga tattattgta gtactataga ttatattatt 5700
   cgtagagtaa agtttaaata tatgtataaa gatagataaa ctgcacttca aacaagtgtg 5760
   acaaaaaaaa tatgtggtaa ttttttataa cttagacatg caatgctcat tatctctaga 5820
   gaggggcacg accgggtcac gctgcactgc aggcatgcaa gcttgaattc ctgcagcccc 5880
   gccaagctat caactttgta tagaaaagtt ggtttgtgtc ttctagatta atcctccaaa 5940
   cttttgatta accaaaaaaa ttatcaaact aacatgttct ccttttttct ttagaaattc 6000
   taacgaattt atctttatac tgatttgaat atacttaatt tggtcatttg gatgcccttt 6060
   acaacctcct taccaaactc actatggcaa atatatacta ttttccattg taacataaat 6120
   gtccataatt tgaattaaat tcgttgcagt acgaaaccat ccaactttgt ccaaaaacaa 6180
   aatccttata actatttact ttaatgtaaa tatatcctct acttttgttt ttacaaccct 6240
   agctcaaaca aatttattat ttgcgataaa aaatcatatc gaacaaactc gatgattttt 6300
   tttttcttac gttattaatg aaactaaaat atagaaaaaa acaagatgaa ccaaattttc 6360
   acctatctaa ctacttaaat ataatatgat taaatttggt aaagtttgaa aagtttcttt     6420
   agaaatgtga aatattgatc acagtttcta ttgctaaaat caccaacaaa acgcatgtcg     6480
   ccattcataa ttatggtttc acacctacaa ctaggctaat aagtaaataa gtagacaact     6540
   agactcaggt ttgaaaaaac cataaaagcc atatagcgtt ttctcattga aactgcgaac     6600
   acgatcgtgt gaatgttgca gtttctagtt ttgatacaaa caaacaaaaa cacaatttaa     6660
   tcttagatta aaaagaaaaa agagaacgga gcccactagc cactccttca aacgtgtctt     6720
   accaactctc ttctagaaac aaattaggct tcaccttcct cttccaacct ctctctctct     6780
   ctctctctct ttttctcaaa ccatctctcc ataaagccct aatttcttca tcacaagaat     6840
   cagaagaaga aacaagtttg tacaaaaaag caggcttact gcaaaaaact tatggacctg     6900
   catctaattt tcggtccaac ttgcacagga aagacgacga ccgcgatagc tcttgcccag     6960
   cagacagggc ttccagtcct ttcgcttgat cgggtccaat gctgtcctca actatcaacc     7020
   ggaagcggac gaccaacagt ggaagaactg aaaggaacga cgcgtctcta ccttgatgat     7080
   cggcctctgg tggagggtat catcgcagcc aagcaagctc atcataggct gatcgaggag     7140
   gtgtataatc atgaggccaa cggcgggctt attcttgagg gaggatccac ctcgttgctc     7200
   aactgcatgg cgcgaaacag ctattggagt gcagattttc gttggcatat tattcgccac     7260
   aagttacccg accaagagac cttcatgaaa gcggccaagg ccagagttaa gcagatgttg     7320
   caccccgctg caggccattc tattattcaa gagttggttt atctttggaa tgaacctcgg     7380
   ctgaggccca ttctgaaaga gatcgatgga tatcgatatg ccatgttgtt tgctagccag     7440
   aaccagatca cggcagatat gctattgcag cttgacgcaa atatggaagg taagttgatt     7500
   aatgggatcg ctcaggagta tttcatccat gcgcgccaac aggaacagaa attcccccaa     7560
   gttaacgcag ccgctttcga cggattcgaa ggtcatccgt tcggaatgta ttaggtaccc     7620
   agctttcttg tacaaagtgg agtccgcaaa aatcaccagt ctctctctac aaatctatct     7680
   ctctctattt ttctccagaa taatgtgtga gtagttccca gataagggaa ttagggttct     7740
   tatagggttt cgctcatgtg ttgagcatat aagaaaccct tagtatgtat ttgtatttgt     7800
   aaaatacttc tatcaataaa atttctaatt cctaaaacca aaatccagtg acctcaactt     7860
   ta                                                                    7862